# EUROPÄISCHE PATENTANMELDUNG

(11) **EP 1 544 301 A1**
(43) Veröffentlichungstag der Anmeldung: **22.06.2005**
(21) Anmeldenummer: 05005648.0
(22) Anmeldetag: 09.11.1995
(51) Int. Cl.: C12N 15/52, C12N 15/82, A61K 35/78, C07K 14/415

(54) **DNA-Moleküle codierend Enzyme, die an der Stärkesynthese beteiligt sind, Vektoren, Bakterien, transgene Pflanzenzellen und Pflanzen enthaltend diese Moleküle**

(30) Priorität: 10.11.1994 DE 4441408
(62) Teilanmeldung aus: 95937059.4
(71) Anmelder: Bayer CropScience GmbH, 65929 Frankfurt/Main (DE)
(72) Erfinder: Kossmann, Jens, 7130 Somerset West (ZA); Springer, Franziska, 12159 Berlin (DE); Abel, Gernot, 2100 Kopenhagen (DK)
(74) Vertreter: VOSSIUS & PARTNER

(57) **Zusammenfassung**

Die vorliegende Erfindung betrifft DNA-Moleküle die für Enzyme codieren, die an der Stärkesynthese in Pflanzen beteiligt sind. Bei diesen Enzymen handelt es sich um zwei verschiedene Isoförmen der löslichen Stärkesynthase sowie um eine Stärkekorn-gebundene Stärkesynthase.

Weiterhin betrifft diese Erfindung Vektoren, Bakterien, sowie mit den beschriebenen DNA-Molekülen transformierte Pflanzenzellen und aus diesen regenerierbare Pflanzen.

Ferner betrifft die Erfindung Stärke, die aus Pflanzen mit gesteigerter oder verringerter Aktivität der beschriebenen Proteine isoliert werden kann.

## Beschreibung

Die vorliegende Erfindung betrifft DNA-Moleküle, die Enzyme codieren, die an der Stärkesynthese in Pflanzen beteiligt sind. Bei diesen Enzymen handelt es sich um zwei verschiedene Isoformen der löslichen Stärkesynthase sowie um eine Stärkekorn-gebundene Stärkesynthase.
Weiterhin betrifft diese Erfindung Vektoren, Bakterien, sowie mit den beschriebenen DNA-Molekülen transformierte Pflanzenzellen und aus diesen regenerierbare Pflanzen.
Ferner werden Verfahren zur Herstellung transgener Pflanzen beschrieben, die aufgrund der Einführung von DNA-Molekülen, die lösliche bzw. Stärkekorn-gebundene Stärkesynthasen codieren, eine in ihren Eigenschaften veränderte Stärke synthetisieren.

Im Hinblick auf die zunehmende Bedeutung, die pflanzlichen Inhaltsstoffen als erneuerbaren Rohstoffquellen in letzter Zeit beigemessen wird, ist es eine der Aufgaben der biotechnologischen Forschung, sich um eine Anpassung dieser pflanzlichen Rohstoffe an die Anforderungen der verarbeitenden Industrie zu bemühen. Um eine Anwendung von nachwachsenden Rohstoffen in möglichst vielen Einsatzgebieten zu ermöglichen, ist es darüber hinaus erforderlich, eine große Stoffvielfalt zu erreichen.
Neben Ölen, Fetten und Proteinen stellen Polysaccharide die wesentlichen nachwachsenden Rohstoffe aus Pflanzen dar. Eine zentrale Stellung bei den Polysacchariden nimmt neben Cellulose die Stärke ein, die einer der wichtigsten Speicherstoffe in höheren Pflanzen ist. Neben Mais, Reis und Weizen spielt die Kartoffel bei der Stärkeproduktion eine wichtige Rolle.

Das Polysaccharid Stärke ist ein Polymer aus chemisch einheitlichen Grundbausteinen, den Glucosemolekülen. Es handelt sich dabei jedoch um ein sehr komplexes Gemisch aus unterschiedlichen Molekülformen, die sich hinsichtlich ihres Polymerisationsgrades und des Auftretens von Verzweigungen der Glucoseketten unterscheiden. Daher stellt Stärke keinen einheitlichen Rohstoff dar. Man unterscheidet insbesondere die Amylose-Stärke, ein im wesentlichen unverzweigtes Polymer aus α-1,4-glycosidisch verknüpften Glucosemolekülen, von der Amylopektin-Stärke, die ihrerseits ein komplexes Gemisch aus unterschiedlich verzweigten Glucoseketten darstellt. Die Verzweigungen kommen dabei durch das Auftreten von zusätzlichen α-1,6-glycosidischen Verknüpfungen zustande. In typischen für die Stärkeproduktion verwendeten Pflanzen, wie z.B. Mais oder Kartoffel, besteht die synthetisierte Stärke zu ca. 25 % aus Amylosestärke und zu ca. 75 % aus Amylopektin-Stärke.

Um eine möglichst breite Anwendung von Stärke zu ermöglichen, erscheint es wünschenswert, Pflanzen zur Verfügung zu stellen, die in der Lage sind, modifizierte Stärke zu synthetisieren, die sich für verschiedene Verwendungszwecke besonders eignet. Eine Möglichkeit, derartige Pflanzen bereitzustellen, besteht - neben züchterischen Maßnahmen - in der gezielten genetischen Veränderung des Stärkemetabolismus stärkeproduzierender Pflanzen durch gentechnologische Methoden. Voraussetzung hierfür ist jedoch die Identifizierung und Charakterisierung der an der Stärkesynthese und/oder
-modifikation beteiligten Enzyme sowie die Isolierung der entsprechenden, diese Enzyme codierende DNA-Moleküle.

Die biochemischen Synthesewege, die zum Aufbau von Stärke führen, sind im wesentlichen bekannt. Die Stärkesynthese in pflanzlichen Zellen findet in den Plastiden statt. In photosynthetisch aktiven Geweben sind dies die Chloroplasten, in photosynthetisch inaktiven, stärkespeichernden Geweben die Amyloplasten.

Die wichtigsten an der Stärkesynthese beteiligten Enzyme sind die Stärkesynthasen sowie Verzweigungsenzyme. Bei den Stärkesynthasen sind verschiedene Isoformen beschrieben, die alle eine Polymerisierungsreaktion durch Übertragung eines Glucosylrestes von ADP-Glucose auf α-1,4-Glucane katalysieren. Verzweigungsenzyme katalysieren die Einführung von α-1,6-Verzweigungen in lineare α-1,4-Glucane.
Darüber hinaus wird die Beteiligung weiterer Enzymaktivitäten, beispielsweise hydrolytischer oder phosphorolytischer, an der Stärkesynthese diskutiert (Preiss in Oxford Surveys of Plant Molecular and Cell Biology, Oxford University Press, Vol. 7 (1991), 59-114). Im Fall des "R-Enzyms", des sogenannten Disproportionierungsenzyms, und der Stärkephosphorylasen kann ebenfalls eine Beteiligung an der Stärkesynthese nicht ausgeschlossen werden, obwohl diese Enzyme bisher meist mit dem Stärkeabbau in Verbindung gebracht werden. Stärkesynthasen können in zwei Klassen eingeteilt werden: die Stärkekorn-gebundenen Stärkesynthasen ("granule-bound starch synthases"; GBSS), die überwiegend an Stärkekörner gebunden, aber auch in löslicher Form vorliegen, und die löslichen Stärkesynthasen ("soluble starch synthases"; SSS). Für verschiedene Pflanzenspezies werden innerhalb dieser Klassen wiederum verschiedene Isoformen beschrieben, die sich hinsichtlich ihrer Abhängigkeit von Startermolekülen unterscheiden (sogenannte "primer dependent" (Typ II) und "primer independent" (Typ I) starch synthases).
Lediglich für die Isoform GBSS I gelang es bisher, die genaue Funktion bei der Stärkesynthese zu ermitteln. Pflanzen, in denen diese Enzymaktivität stark oder vollkommen reduziert ist, synthetisieren eine amylosefreie (sogenannte "waxy") Stärke (Shure et al., Cell 35 (1983), 225-233; Visser et al., Mol. Gen. Genet. 225 (1991), 289-296; WO 92/11376), so daß diesem Enzym eine entscheidende Rolle bei der Synthese der Amylosestärke zugesprochen wird. Dieses Phänomen wird ebenfalls in Zellen der Grünalge *Chlamydomonas* reinhardtii beobachtet (Delrue et al., J. Bacteriol. 174 (1992), 3612-3620). Bei *Chlamydomonas* konnte darüber hinaus gezeigt werden, daß GBSS I nicht nur an der Synthese der Amylose beteiligt ist, sondern auch einen Einfluß auf die Amylopektinsynthese besitzt. In Mutanten, die keine GBSS I-Aktivität aufweisen, fehlt eine bestimmte Fraktion des normalerweise synthetisierten Amylopektins, die längerkettige Glucane aufweist.

Die Funktionen der anderen Isoformen der Stärkekorn-gebundenen Stärkesynthasen, insbesondere der GBSS II, und der löslichen Stärkesynthasen sind bisher unklar. Es wird angenommen, daß die löslichen Stärkesynthasen zusammen mit Verzweigungsenzymen an der Synthese des Amylopektins beteiligt sind (siehe z.B. Ponstein et al., Plant Physiol. 92 (1990), 234-241) und daß sie eine wichtige Funktion bei der Regulation der Stärkesyntheserate spielen.
Bei Kartoffel wurden die Isoformen GBSS I, GBSS II, sowie zwei bzw. drei Isoformen der löslichen Stärkesynthasen, die bisher nicht näher bezeichnet wurden, identifiziert (Ponstein et al., Plant Physiol. 92 (1990), 234-241; Smith et al., Planta 182 (1990), 599-604; Hawker et al., Phytochemistry 11 (1972), 1287-1293). Für Erbse wurde ebenfalls eine GBSS II nachgewiesen (Dry et al., The Plant Journal 2,2 (1992), 193-202).
Eine GBSS I aus Kartoffel codierende cDNA sowie eine genomische DNA sind bereits beschrieben (Visser et al., Plant Sci. 64 (1989), 185-192; van der Leij et al., Mol. Gen. Genet. 228 (1991), 240-248). Nucleinsäuresequenzen, die weitere Stärkekorn-gebundene Stärkesynthasen oder eine der löslichen Stärkesynthase-Isoformen aus Kartoffel codieren, lagen jedoch bisher noch nicht vor.
Außer bei der Kartoffel wurden lösliche Stärkesynthasen auch in einer Reihe weiterer Pflanzenarten identifiziert. Lösliche Stärkesynthasen sind beispielsweise bis zur Homogenität aus Erbse (Denyer und Smith, Planta 186 (1992), 609-617) und Mais (WO 94/09144) isoliert worden. Im Fall der Erbse stellte sich heraus, daß die als SSS II identifizierte Isoform der löslichen Stärkesynthase identisch ist mit der Stärkekorn-gebundenen Stärkesynthase GBSS II (Denyer et al., Plant J. 4 (1993), 191-198). Für einige weitere Pflanzenspezies wurde das Vorhandensein mehrerer SSS-Isoformen mit Hilfe chromatographischer Methoden beschrieben, beispielsweise bei Gerste (Tyynelä und Schulman, Physiologia Plantarum 89 (1993) 835-841; Kreis, Planta 148 (1980), 412-416), Mais (Pollock und Preiss, Arch. Biochem. Biophys. 204 (1980), 578-588) und Weizen (Rijven, Plant Physiol. 81 (1986), 448-453). DNA-Sequenzen, die diese Proteine codieren, wurden jedoch bisher nicht beschrieben.
Eine cDNA-Sequenz, die eine lösliche Stärkesynthase codiert, wurde bisher lediglich für Reis beschrieben (Baba et al., Plant Physiol. 103 (1993), 565-573).
Um Möglichkeiten bereitzustellen, beliebige stärkespeicherde Pflanzen dahingehend zu verändern, daß sie eine modifizierte Stärke synthetisieren, ist es erforderlich, jeweils DNA-Sequenzen zu identifizieren, die die verschiedenen Isoformen der Stärkekorn-gebundenen bzw. löslichen Stärkesynthasen codieren.

Der vorliegenden Erfindung liegt somit die Aufgabe zugrunde, DNA-Moleküle, insbesondere aus Kartoffel, zur Verfügung zu stellen, die an der Stärkebiosynthese beteiligte Enzyme codieren und mit deren Hilfe es möglich ist, gentechnisch veränderte Pflanzen herzustellen, die eine erhöhte oder erniedrigte Aktivität dieser Enzyme aufweisen, wodurch es zu einer Veränderung der chemischen und/oder physikalischen Eigenschaften der in diesen Pflanzen synthetisierten Stärke kommt.
Diese Aufgabe wird durch die Bereitstellung der in den Patentansprüchen bezeichneten Ausführungsformen gelöst.

Die Erfindung betrifft daher DNA-Moleküle, die Stärkesynthasen codieren, insbesondere solche DNA-Moleküle, die Stärkekorn-gebundene Stärkesynthasen der Isoform II codieren, als auch DNA-Moleküle, die lösliche Stärkesynthasen codieren.

Insbesondere betrifft die vorliegende Erfindung DNA-Moleküle, die Proteine mit der biologischen Aktivität einer Stärkekorn-gebundenen Stärkesynthase der Isoform II (GBSSII) codieren oder ein biologisch aktives Fragment eines solchen Proteins, wobei derartige Moleküle vorzugsweise Proteine mit der unter Seq ID No. 8 angegebenen Aminosäuresequenz codieren. Insbesondere betrifft die Erfindung DNA-Moleküle mit der unter Seq D No. 7 angegebenen Nucleotidsequenz, bevorzugt Moleküle, die die in Seq ID No. 7 angegebene codierende Region umfassen.
Gegenstand der Erfindung sind ebenfalls DNA-Moleküle, die eine GBSSII codieren und deren Sequenz aufgrund der Degeneration des genetischen Codes von den Nucleotidsequenzen der oben beschriebenen DNA-Moleküle abweicht.
Ferner betrifft die vorliegende Erfindung DNA-Moleküle, die GBSSII codieren und die mit einem der oben beschriebenen DNA-Moleküle hybridisieren. Derartige DNA-Moleküle stammen vorzugsweise aus stärkespeichernden Pflanzen, insbesondere dicotylen Pflanzen, und besonders bevorzugt aus Kartoffel.
Die durch die erfindungsgemäßen DNA-Moleküle codierten GBSSII-Proteine haben vorzugsweise ein Molekulargewicht von 85±5 kD. GBSSII-Proteine liegen vorwiegend an Stärkekörner gebunden vor, können jedoch auch in löslicher Form vorliegen.

Ferner betrifft die vorliegende Erfindung DNA-Moleküle, die Proteine mit der biologischen Aktivität einer löslichen Stärkesynthase der Isoform B (SSSB) codieren oder ein biologisch aktives Fragment eines solchen Proteins, wobei derartige Moleküle vorzugsweise Proteine mit der unter Seq ID No. 10 angegebenen Aminosäuresequenz codieren. Insbesondere betrifft die Erfindung DNA-Moleküle mit der unter Seq ID No. 9 angegebenen Nucleotidsequenz, bevorzugt Moleküle, die die in Seq ID No. 9 angegebene codierende Region umfassen.
Gegenstand der Erfindung sind ebenfalls DNA-Moleküle, die eine SSSB codieren und deren Sequenz aufgrund der Degeneration des genetischen Codes von den Nucleotidsequenzen der oben beschriebenen DNA-Moleküle abweicht.
Ferner betrifft die vorliegende Erfindung DNA-Moleküle, die SSSB codieren und die mit einem der oben beschriebenen DNA-Moleküle hybridisieren. Ausgenommen sind dabei DNA-Moleküle aus Reis. Die durch die erfindungsgemäßen DNA-Moleküle codierten SSSB-Proteine haben vorzugsweise ein Molekulargewicht von 78±5 kD.
Die enzymatischen Eigenschaften der SSSB-Proteine sind in den Beispielen beschrieben.

Die Erfindung betrifft weiterhin DNA-Moleküle, die Proteine mit der biologischen Aktivität einer löslichen Stärkesynthase der Isoform A (SSSA) codieren. Derartige Proteine können beispielsweise dadurch charakterisiert werden, daß sie von einem Antikörper, der gegen das Peptid mit der Aminosäuresequenz NH2-GTGGLRDTVENC-COOH (Seq ID No. 13) gerichtet ist, erkannt werden. Die enzymatischen Eigenschaften der SSSA-Proteine sind in den Beispielen beschrieben.
Ein Beispiel für ein DNA-Molekül, das ein derartiges Protein codiert, ist ein DNA-Molekül mit der in Seq ID No. 11 dargestellten codierenden Region. Dieses DNA-Molekül kann verwendet werden, um aus anderen Organismen, insbesondere Pflanzen DNA-Moleküle zu isolieren, die SSSA-Proteine codieren. Somit betrifft die vorliegende Erfindung auch DNA-Moleküle, die Proteine mit der biologischen Aktivität einer löslichen Stärkesynthase der Isoform A (SSSA) codieren oder ein biologisch aktives Fragment eines solchen Proteins, wobei derartige Moleküle vorzugsweise Proteine mit der unter Seq ID No. 12 angegebenen Aminosäuresequenz codieren. Insbesondere betrifft die Erfindung DNA-Moleküle mit der unter SeqID No. 11 angegebenen Nucleotidsequenz, bevorzugt Moleküle, die die in Seq ID No. 11 angegebene codierende Region umfassen.
Gegenstand der Erfindung sind ebenfalls DNA-Moleküle, die eine SSSA codieren und deren Sequenz aufgrund der Degeneration des genetischen Codes von den Nucleotidsequenzen der oben beschriebenen DNA-Moleküle abweicht.
Ferner betrifft die vorliegende Erfindung DNA-Moleküle, die SSSA codieren und die mit einem der oben beschriebenen DNA-Moleküle hybridisieren.
Das SSSA-Protein hat dabei vorzugsweise in einer SDS-Gelelektrophorese ein apparentes Molekulargewicht von ca. 120 bis 140 kD, insbesondere von ca. 135 kD.

Der Begriff "Hybridisierung" bedeutet im Rahmen dieser Erfindung eine Hybridisierung unter konventionellen Hybridisierungsbedingungen, vorzugsweise unter stringenten Bedingungen, wie sie beispielsweise in Sambrock et al., Molecular Cloning, A Laboratory Manual, 2. Aufl. (1989) Cold Spring Harbor Laboratory Press, Cold Spring Harbor, NY) beschrieben sind. DNA-Moleküle, die mit den erfindungsgemäßen DNA-Molekülen hybridisieren, können prinzipiell aus jedem beliebigen Organismus (d.h. Proparyonten oder Eukaryonten, insbesondere aus Bakterien, Pilzen, Algen, Pflanzen oder tierischen Oprganismen) stammen, der derartige DNA-Moleküle besitzt. Sie stammen vorzugsweise aus monokotylen oder dikotylen Pflanzen, insbesondere aus Nutzpflanzen, und besonders bevorzugt aus Stärke-speichernden Pflanzen.
DNA-Moleküle, die mit den erfindungsgemäßen DNA-Molekülen hybridisieren, können z.B. aus genomischen oder aus cDNA-Bibliotheken verschiedener Organismen isoliert werden.
Die Identifizierung und Isolierung derartiger DNA-Moleküle aus Pflanzen oder anderen Organismen kann dabei unter Verwendung der erfindungsgemäßen DNA-Moleküle oder Teile dieser DNA-Moleküle bzw. der reversen Komplemente dieser Moleküle erfolgen, z.B. mittels Hybridisierung nach Standardverfahren (siehe z.B. Sambrook et al., 1989, Molecular Cloning, A Laboratory Manual, 2. Aufl. Cold Spring Harbor Laboratory Press, Cold Spring Harbor, NY).

Als Hybridisierungsprobe können z.B. DNA-Moleküle verwendet werden, die exakt die oder im wesentlichen die unter Seq ID No. 7, 9 oder 11 angegebene DNA-Sequenz oder Teile dieser Sequenz aufweisen. Bei den als Hybridisierungsprobe verwendeten DNA-Fragmenten kann es sich auch um synthetische DNA-Fragmente handeln, die mit Hilfe der gängigen DNA-Synthesetechniken hergestellt wurden und deren Sequenz im wesentlichen mit der eines erfindungsgemäßen DNA-Moleküls übereinstimmt. Hat man Gene identifiziert und isoliert, die mit den erfindungsgemäßen DNA-Sequenzen hybridisieren, ist eine Bestimmung der Sequenz und eine Analyse der Eigenschaften der von dieser Sequenz codierten Proteine erforderlich.

Die mit den erfindungsgemäßen DNA-Molekülen hybridisierenden Moleküle umfassen auch Fragmente, Derivate und allelische Varianten der oben beschriebenen DNA-Moleküle, die eines der oben beschriebenen Proteine codieren. Unter Fragmenten werden dabei Teile der DNA-Moleküle verstanden, die lang genug sind, um eines der beschriebenen Proteine zu codieren. Der Ausdruck Derivat bedeutet in diesem Zusammenhang, daß die DNA-Sequenzen dieser Moleküle sich von den Sequenzen der oben beschriebenen DNA-Moleküle an einer oder mehreren Positionen unterscheiden und einen hohen Grad an Homologie zu diesen DNA-Sequenzen aufweisen. Homologie bedeutet dabei eine Sequenzidentität von mindestens 40 %, insbesondere eine Identität von mindestens 60 %, vorzugsweise über 80 % und besonders bevorzugt über 90 %. Die Abweichungen zu den oben beschriebenen DNA-Molekülen können dabei durch Deletion, Substitution, Insertion oder Rekombination entstanden sein.
Homologie bedeutet ferner, daß funktionelle und/oder strukturelle Äquivalenz zwischen den betreffenden DNA-Molekülen oder den durch sie codierten Proteinen, besteht. Bei den DNA-Molekülen, die homolog zu den oben beschriebenen DNA-Molekülen sind und Derivate dieser DNA-Moleküle darstellen, handelt es sich in der Regel um Variationen dieser DNA-Moleküle, die Modifikationen darstellen, die dieselbe biologische Funktion ausüben. Es kann sich dabei sowohl um natürlicherweise auftretende Variationen handeln, beispielsweise um Sequenzen aus anderen Organismen, oder um Mutationen, wobei diese Mutationen auf natürliche Weise aufgetreten sein können oder durch gezielte Mutagenese eingeführt wurden. Ferner kann es sich bei den Variationen um synthetisch hergestellte Sequenzen handeln. Bei den allelischen Varianten kann es sich sowohl um natürlich auftretende Varianten handeln, als auch um synthetisch hergestellte oder durch rekombinante DNA-Techniken erzeugte Varianten.
Die von den verschiedenen Varianten der erfindungsgemäßen DNA-Moleküle codierten Proteine weisen bestimmte gemeinsame Charakteristika auf. Dazu können z.B. Enzymaktivität, Molekulargewicht, immunologische Reaktivität, Konformation etc. gehören, sowie physikalische Eigenschaften wie z.B. das Laufverhalten in Gelelektrophoresen, chromatographisces Verhalten, Sedimentationskoeffizienten, Löslichkeit, spektroskopische Eigenschaften, Stabilität; pH-Optimum, TemperaturOptimum etc.

Wichtige Charakteristika einer Stärkesynthase sind: i) ihre Lokalisation im Stroma der Plastiden pflanzlicher Zellen; ii) ihre Fähigkeit zur Synthese linearer α-1,4-verknüpfter Polyglucane unter Verwendung von ADP-Glucose als Substrat. Diese Aktivität kann wie in Denyer und Smith (Planta 186 (1992), 606-617) und in den Beispielen beschrieben bestimmt werden.

Die erfindungsgemäßen DNA-Moleküle können prinzipiell aus jedem Organismus stammen, der die beschriebenen Proteine exprimiert, vorzugsweise aus Pflanzen, insbesondere aus stärkesynthetisierenden bzw. stärkespeichernden Pflanzen. Diese können sowohl monokotyle oder auch dikotyle Pflanzen sein. Besonders bevorzugt sind dabei z.B. Getreidearten (wie Gerste, Roggen, Hafer, Weizen etc.), Mais, Reis, Erbse, Maniok, Kartoffel usw.

Ferner betrifft die Erfindung Vektoren, insbesondere Plasmide, Cosmide, Viren, Bacteriophagen und andere in der Gentechnik gängige Vektoren, die die oben beschriebenen erfindungsgemäßen DNA-Moleküle enthalten.

In einer bevorzugten Ausführungsform sind die in den Vektoren enthaltenen DNA-Moleküle verknüpft mit DNA-Elementen, die die Transkription und Synthese einer translatierbaren RNA in prokaryontischen oder eukaryontischen Zellen gewährleisten.

Die Expression der erfindungsgemäßen DNA-Moleküle in prokaryontischen Zellen, beispielsweise in *Escherichia coli,* ist insofern interessant, als daß auf diese Weise eine genauere Charakterisierung der enzymatischen Aktivitäten dieser Enzyme, für die diese Moleküle codieren, ermöglicht wird. Es ist insbesondere möglich, das Produkt, das von den entsprechenden Enzymen in Abwesenheit anderer, in der pflanzlichen Zelle an der Stärkesynthese beteiligter Enzyme synthetisiert wird, zu charakterisieren. Dies läßt Rückschlüsse zu auf die Funktion, die das entsprechende Protein bei der Stärkesynthese in der Pflanzenzelle ausübt.
Darüber hinaus ist es möglich, mittels gängiger molekularbiologischer Techniken (siehe z.B. Sambrook et al., 1989, Molecular Cloning, A Laboratory Manual, 2. Aufl. Cold Spring Harbor Laboratory Press, Cold Spring Harbor, NY) verschiedenartige Mutationen in die erfindungsgemäßen DNA-Moleküle einzuführen, wodurch es zur Synthese von Proteinen mit eventuell veränderten biologischen Eigenschaften kommt. Hierbei ist zum einen die Erzeugung von Deletionsmutanten möglich, bei denen durch fortschreitende Deletionen vom 5'- oder vom 3'- Ende der codierenden DNA-Sequenz DNA-Moleküle erzeugt werden, die zur Synthese entsprechend verkürzter Proteine führen. Durch derartige Deletionen am 5'-Ende der DNA-Sequenz ist es beispielsweise möglich, Aminosäuresequenzen zu identifizieren, die für die Translokation des Enzyms in die Plastiden verantwortlich sind (Transitpeptide). Dies erlaubt es, gezielt Enzyme herzustellen, die durch Entfernen der entsprechenden Sequenzen nicht mehr in den Plastiden, sondern im Cytosol lokalisiert sind, oder aufgrund der Addition von andereren Signalsequenzen in anderen Kompartimenten lokalisiert sind.
Andererseits ist auch die Einführung von Punktmutationen denkbar an Positionen, bei denen eine Veränderung der Aminosäuresequenz einen Einfluß beispielweise auf die Enzymaktivität oder die Regulierung des Enzyms hat. Auf diese Weise können z.B. Mutanten hergestellt werden, die einen veränderten Kₘ-Wert besitzen oder nicht mehr den normalerweise in der Zelle vorliegenden Regulationsmechanismen über allosterische Regulation oder kovalente Modifizierung unterliegen.
Des weiteren können Mutanten hergestellt werden, die eine veränderte Substrat- oder Produktspezifität aufweisen, wie z.B. Mutanten, die als Substrat ADP-Glucose-6-Phosphat anstatt ADP-Glucose verwenden. Weiterhin können Mutanten hergestellt werden, die ein verändertes Aktivitäts-TemperaturProfil aufweisen.
Für die gentechnische Manipulation in prokaryontischen Zellen können die erfindungsgemäßen DNA-Moleküle oder Teile dieser Moleküle in Plasmide eingebracht werden, die eine Mutagenese oder eine Sequenzveränderung durch Rekombination von DNA-Sequenzen erlauben. Mit Hilfe von Standardverfahren (vgl. Sambrook et al., 1989, Molecular Cloning: A laboratory manual, 2. Aufl., Cold Spring Harbor Laboratory Press, NY, USA) können Basenaustausche vorgenommen oder natürliche oder synthetische Sequenzen hinzugefügt werden. Für die Verbindung der DNA-Fragmente untereinander können an die Fragmente Adaptoren oder *linker* angesetzt werden. Ferner können Manipulationen, die passende Restriktionsschnittstellen zur Verfügung stellen oder die überflüssige DNA oder Restriktionsschnittstellen entfernen, eingesetzt werden. Wo Insertionen, Deletionen oder Substitutionen in Frage kommen, können in vitro-Mutagenese, *"primer repair*", Restriktion oder Ligation verwendet werden. Als Analysemethode werden im allgemeinen eine Sequenzanalyse, eine Restriktionsanalyse und weitere biochemisch-molekularbiologische Methoden durchgeführt.

In einer weiteren Ausführungsform betrifft die Erfindung Wirtszellen, insbesondere prokaryontische oder eukaryontische Zellen, die ein oben beschriebenes erfindungsgemäßes DNA-Molekül oder einen erfindungsgemäßen Vektor enthalten. Dabei handelt es sich vorzugsweise um bakterielle Zellen oder pflanzliche Zellen.

Gegenstand der Erfindung sind ferner die Proteine, die durch die erfindungsgemäßen DNA-Moleküle codiert werden, sowie Verfahren zu deren Herstellung, wobei eine erfindungsgemäße Wirtszelle unter Bedingungen kultiviert wird, die die Synthese des Proteins erlauben, und das Protein aus den kultivierten Zellen und/oder dem Kulturmedium isoliert wird.

Es wurde nun gefunden, daß es durch die Bereitstellung der erfindungsgemäßen DNA-Moleküle möglich ist, mit Hilfe gentechnischer Methoden in den Stärkemetabolismus von Pflanzen einzugreifen, wie es bisher nicht möglich war, und ihn dahingehend zu verändern, daß es zur Synthese einer Stärke kommt, die in ihren physikalisch-chemischen Eigenschaften, insbesondere dem Amylose/Amylopektin-Verhältnis, dem Verzweigungsgrad, der durchschnittlichen Kettenlänge, dem Phosphatgehalt, der Verkleisterung, der Stärkekorngröße und/oder der Stärkekornform im Vergleich zu in Wildtyp-Pflanzen synthetisierter Stärke verändert ist. Lösliche Stärkesynthasen spielen beispielsweise eine zentrale Rolle bei der Regulation der Syntheserate der Stärke. Daher ist durch eine Erhöhung der Aktivität dieser Enzyme oder durch die Bereitstellung von Mutanten, die nicht mehr den zelleigenen Regulationsmechanismen unterliegen und/oder unterschiedliche Temperaturabhängigkeiten in bezug auf ihre Aktivität besitzen, eine Ertragssteigerung in entsprechend gentechnisch veränderten Pflanzen möglich. Die wirtschaftliche Bedeutung der Möglichkeit des Eingriffs in die Stärkesynthese allein bei Kartoffelpflanzen ist offensichtlich: Die Kartoffel ist beispielsweise in Europa neben Mais und Weizen eine der wichtigsten Pflanzen zur Stärkegewinnung. Ca. 20 % der in Europa jährlich produzierten Stärke wird aus Kartoffeln gewonnen. Ferner weist Kartoffelstärke im Vergleich zu Stärke aus Mais und Weizen einige vorteilhafte Eigenschaften auf, beispielsweise einen niedrigen Protein- und Lipidgehalt sowie verhältnismäßig große Stärkekörner, Phosphatgehalt, weshalb sie, falls dies möglich ist, vorzugsweise verwendet wird.
Möglich ist somit die Expression der erfindungsgemäßen DNA-Moleküle in pflanzlichen Zellen, um die Aktivität einer oder mehrerer Stärkesynthasen zu erhöhen. Ferner ist es möglich, die erfindungsgemäßen DNA-Moleküle nach dem Fachmann bekannten Methoden zu modifizieren, um Stärkesynthasen zu erhalten, die nicht mehr den zelleigenen Regulationsmechanismen unterliegen, bzw. veränderte Temperaturabhängigkeiten oder Substrat- bzw. Produktspezifitäten aufweisen.

Es besteht grundsätzlich die Möglichkeit, daß das synthetisierte Protein in jedem beliebigen Kompartiment der pflanzlichen Zelle lokalisiert sein kann. Um die Lokalisation in einem bestimmten Kompartiment zu erreichen, muß die die Lokalisation in Plastiden gewährleistende Sequenz deletiert werden und die verbleibende codierende Region gegebenenfalls mit DNA-Sequenzen verknüpft werden, die die Lokalisierung in dem jeweiligen Kompartiment gewährleisten. Derartige Sequenzen sind bekannt (Siehe beispielsweise Braun et al., 1992, EMBO J. 11:3219-3227; Wolter et al., 1988, Proc. Natl. Acad. Sci. USA 85:846-850; Sonnewald et al., 1991, Plant J. 1:95-106).

Die vorliegende Erfindung betrifft somit auch transgene Pflanzenzellen, die ein erfindungsgemäßes DNA-Molekül enthalten, wobei dieses mit regulatorischen DNA-Elementen verknüpft ist, die die Transkription in pflanzlichen Zellen gewährleisten, insbesondere mit einem Promotor, der in bezug auf das DNA-Molekül heterolog ist.
Die transgenen Pflanzenzellen können nach dem Fachmann bekannten Techniken zu ganzen Pflanzen regeneriert werden. Die durch Regeneration der erfindungsgemäßen transgenen Pflanzenzellen erhältlichen Pflanzen sind ebenfalls Gegenstand der vorliegenden Erfindung. Ferner sind Gegenstand der Erfindung Pflanzen, die die obenbeschriebenen transgenen Pflanzenzellen enthalten. Bei den transgenen Pflanzen kann es sich prinzipiell um Pflanzen jeder beliebigen Pflanzenspezies handeln, d.h. sowohl monokotyle als auch dikotyle Pflanzen. Bevorzugt handelt es sich um Nutzpflanzen, wie z.B. Getreidearten (Roggen, Gerste Hafer, Weizen etc.), Reis, Mais, Erbse, Maniok oder Kartoffel.
Die Erfindung betrifft ebenfalls Vermehrungsmaterial der erfindungsgemäßen Pflanzen, beispielsweise Früchte, Samen, Knollen, Stecklinge etc.

Die erfindungsgemäßen transgenen Pflanzenzellen und Pflanzen synthetisieren aufgrund der Expression bzw. zusätzlichen Expression eines erfindungsgemäßen DNA-Moleküls eine Stärke, die im Vergleich zu Stärke aus Wildtyp-Pflanzen, d.h. nicht-transformierten Pflanzen, modifiziert ist, insbesondere im Hinblick auf die Viskosität wäßriger Lösungen dieser Stärke und/oder den Phosphatgehalt.
Gegenstand der vorliegenden Erfindung ist somit auch die aus den erfindungsgemäßen transgenen Pflanzenzellen und Pflanzen erhältliche Stärke.

Ein weiterer Gegenstand der Erfindung sind transgene Pflanzenzellen, in denen die Aktivität eines erfindungsgemäßen Proteins verringert ist im Vergleich zu nicht-transformierten Pflanzen. Es wurde gefunden, daß es in Pflanzenzellen mit einer verringerten Aktivität eines erfindungsgemäßen Proteins zur Synthese einer Stärke mit veränderten chemischen und/oder physikalischen Eigenschaften kommt verglichen mit Stärke aus Wildtyp-Pflanzenzellen.

Die Herstellung von Pflanzenzellen mit einer verringerten Aktivität eines erfindungsgemäßen Proteins kann beispielsweise unter Verwendung der erfindungsgemäßen DNA-Moleküle erreicht werden. Möglich sind hierbei die Expression einer entsprechenden antisense-RNA, einer sense-RNA zur Erzielung eines Cosuppressionseffektes oder die Expression eines entsprechend konstruierten Ribozyms, das spezifisch Transkripte spaltet, die eines der erfindungsgemäßen Proteine codieren.
Vorzugsweise wird zur Reduzierung der Aktivität eines erfindungsgemäßen Proteins in pflanzlichen Zellen eine antisense-RNA exprimiert.
Hierzu kann zum einen ein DNA-Molekül verwendet werden, das die gesamte für ein erfindungsgemäßes Protein codierende Sequenz einschließlich eventuell vorhandener flankierender Sequenzen umfaßt, als auch DNA-Moleküle, die nur Teile der codierenden Sequenz umfassen, wobei diese Teile lang genug sein müssen, um in den Zellen einen antisense-Effekt zu bewirken. Es können im allgemeinen Sequenzen bis zu einer Mindestlänge von 15 bp, vorzugsweise einer Länge von 100-500 bp, für eine effiziente antisense-Inhibition insbesondere Sequenzen mit einer Länge über 500 bp verwendet werden. In der Regel werden DNA-Moleküle verwendet, die kürzer als 5000 bp, vorzugsweise Sequenzen, die kürzer als 2500 bp sind. Bevorzugt werden DNA-Moleküle verwendet, die homolog in bezug auf die zu transformierende Pflanzenspezies sind.
Möglich ist auch die Verwendung von DNA-Sequenzen, die einen hohen Grad an Homologie zu den Sequenzen der erfindungsgemäßen DNA-Moleküle aufweisen, aber nicht vollkommen identisch sind. Die minimale Homologie sollte größer als ca. 65 % sein. Die Verwendung von Sequenzen mit Homologien zwischen 95 und 100 % ist zu bevorzugen.

Die erfindungsgemäßen transgenen Pflanzenzellen können nach dem Fachmann bekannten Techniken zu ganzen Pflanzen regeneriert werden. Gegenstand der Erfindung sind somit auch Pflanzen, die die erfindungsgemäßen transgenen Pflanzenzellen enthalten. Bei diesen Pflanzen kann es sich prinzipiell um Pflanzen jeder beliebigen Pflanzenspezies handeln, d.h. sowohl monokotyle als auch dikotyle Pflanzen. Vorzugsweise handelt es sich um Nutzpflanzen, insbesondere stärkespeichernde Pflanzen, wie z.B. Getreidearten (Roggen, Gerste, Hafer, Weizen, etc.), Reis, Mais, Erbse, Maniok oder Kartoffel. Die Erfindung betrifft ebenfalls Vermehrungsmaterial der erfindungsgemäßen Pflanzen, wie z.B. Früchte, Samen, Knollen, Stecklinge etc.

Die erfindungsgemäßen transgenen Pflanzenzellen und Pflanzen synthetisieren aufgrund der Verringerung der Aktivität eines der erfindungsgemäßen Proteine eine Stärke, die im Vergleich zu Stärke aus nicht-transformierten Pflanzenzellen bzw. Pflanzen veränderte chemische und/oder physikalische Eigenschaften aufweisen. Diese Stärke zeigt beispielsweise eine veränderte Viskosität ihrer wäßrigen Lösungen und/oder einen veränderten Phosphatgehalt.

Gegenstand der Erfindung ist somit auch die aus den vorgehend beschriebenen transgenen Pflanzenzellen und Pflanzen erhältliche Stärke.

Die erfindungsgemäßen Stärken können nach dem Fachmann bekannten Verfahren modifiziert werden und eignen sich in unmodifizierter oder modifizierter Form für verschiedene Verwendungen im Nahrungsmittel- oder Nicht-Nahrungsmittelbereich.

Die Einsatzmöglichkeit der Stärke läßt sich grundsätzlich in zwei große Bereiche unterteilen. Der eine Bereich umfaßt die Hydrolyseprodukte der Stärke, hauptsächlich Glucose und Glucanbausteine, die über enzymatische oder chemische Verfahren erhalten werden. Sie dienen als Ausgangsstoff für weitere chemische Modifikationen und Prozesse, wie Fermentation. Von Bedeutung kann hier die Einfachheit und kostengünstige Ausführung eines Hydrolyseverfahrens sein, wie es gegenwärtig im wesentlichen enzymatisch unter Verwendung von Amyloglucosidase verläuft. Vorstellbar wäre eine Kosteneinsparung durch einen geringeren Einsatz von Enzymen. Eine Strukturveränderung der Stärke, z.B. Oberflächenvergrößerung des Korns, leichtere Verdaulichkeit durch geringeren Verzweigungsgrad oder eine sterische Struktur, die die Zugänglichkeit für die eingesetzten Enzyme begrenzt, könnte dies bewirken.
Der andere Bereich, in dem die Stärke wegen ihrer polymeren Struktur als sogenannte native Stärke verwendet wird, gliedert sich in zwei weitere Einsatzgebiete:

### 1. Nahrungsmittelindustrie

Stärke ist ein klassischer Zusatzstoff für viele Nahrungsmittel, bei denen sie im wesentlichen die Funktion des Bindens von wäßrigen Zusatzstoffen übernimmt bzw. eine Erhöhung der Viskosität oder aber eine erhöhte Gelbildung hervorruft. Wichtige Eigenschaftsmerkmale sind das Fließ- und Sorptionsverhalten, die Quell- und Verkleisterungstemperatur, die Viskosität und Dickungsleistung, die Löslichkeit der Stärke, die Transparenz und Kleisterstruktur, die Hitze-, Scher- und Säurestabilität, die Neigung zur Retrogradation, die Fähigkeit zur Filmbildung, die Gefrier/Taustabilität, die Verdaulichkeit sowie die Fähigkeit zur Komplexbildung mit z.B. anorganischen oder organischen Ionen.

### 2. Nicht-Nahrungmittelindustrie

In diesem großen Bereich wird Stärke als Hilfsstoff für unterschiedliche Herstellungsprozesse bzw. als Zusatzstoff in technischen Produkten eingesetzt. Bei der Verwendung von Stärke als Hilfsstoff ist hier insbesondere die Papier- und Pappeindustrie zu nennen. Stärke dient dabei in erster Linie zur Retardation (Zurückhaltung von Feststoffen), der Abbindung von Füllstoff- und Feinstoffteilchen, als Festigungsstoff und zur Entwässerung. Darüber hinaus werden die günstigen Eigenschaften der Stärke in bezug auf die Steifigkeit, die Härte, den Klang, den Griff, den Glanz, die Glätte, die Spaltfestigkeit sowie die Oberflächen ausgenutzt.

### 2.1 Papier- und Pappeindustrie

Innerhalb des Papierherstellungsprozesses sind vier Anwendungsbereiche, nämlich Oberfläche, Strich, Masse und Sprühen, zu unterscheiden.
Die Anforderungen an die Stärke in bezug auf die Oberflächenbehandlung sind im wesentlichen ein hoher Weißegrad, eine angepaßte Viskosität, eine hohe Viskositätsstabilität, eine gute Filmbildung sowie eine geringe Staubbildung. Bei der Verwendung im Strich spielt der Feststoffgehalt, eine angepaßte Viskosität, ein hohes Bindevermögen sowie eine hohe Pigmentaffinität eine wichtige Rolle. Als Zusatz zur Masse ist eine rasche, gleichmäßige, verlustfreie Verteilung, eine hohe mechanische Stabilität und eine vollständige Zurückhaltung im Papierfließ von Bedeutung. Beim Einsatz der Stärke im Sprühbereich sind ebenfalls ein angepaßter Feststoffgehalt, hohe Viskosität sowie ein hohes Bindevermögen von Bedeutung.

### 2.2 Klebstoffindustrie

Ein großer Einsatzbereich der Stärken besteht in der Klebstoffindustrie, wo man die Einsatzmöglichkeiten in vier Teilbereiche gliedert: die Verwendung als reinem Stärkeleim, die Verwendung bei mit speziellen Chemikalien aufbereiteten Stärkeleimen, die Verwendung von Stärke als Zusatz zu synthetischen Harzen und Polymerdispersionen sowie die Verwendung von Stärken als Streckmittel für synthetische Klebstoffe. 90 % der Klebstoffe auf Stärkebasis werden in den Bereichen Wellpappenherstellung, Herstellung von Papiersäcken, Beuteln und Tüten, Herstellung von Verbundmaterialien für Papier und Aluminium, Herstellung von Kartonagen und Wiederbefeuchtungsleim für Briefumschläge, Briefmarken usw. eingesetzt.

### 2.3 Textil- und Textilpflegemittelindustrie

Ein großes Einsatzfeld für Stärken als Hilfmittel und Zusatzstoff ist der Bereich Herstellung von Textilien und Textilpflegemitteln. Innerhalb der Textilindustrie sind die folgenden vier Einsatzbereiche zu unterscheiden: Der Einsatz der Stärke als Schlichtmittel, d.h. als Hilfstoff zur Glättung und Stärkung des Klettverhaltens zum Schutz gegen die beim Weben angreifenden Zugkräfte sowie zur Erhöhung der Abriebfestigkeit beim Weben, Stärke als Mittel zur Textilaufrüstung vor allem nach qualitätsverschlechternden Vorbehandlungen, wie Bleichen, Färben usw., Stärke als Verdickungsmittel bei der Herstellung von Farbpasten zur Verhinderung von Farbstoffdiffusionen sowie Stärke als Zusatz zu Kettungsmitteln für Nähgarne.

### 2.4 Baustoffindustrie

Der vierte Einsatzbereich ist die Verwendung der Stärken als Zusatz bei Baustoffen. Ein Beispiel ist die Herstellung von Gipskartonplatten, bei der die im Gipsbrei vermischte Stärke mit dem Wasser verkleistert, an die Oberfläche der Gipsplatte diffundiert und dort den Karton an die Platte bindet.
Weitere Einsatzbereiche sind die Beimischung zu Putz- und Mineralfasern. Bei Transportbeton werden Stärkeprodukte zur Verzögerung der Abbindung eingesetzt.

### 2.5 Bodenstabilisation

Ein weiterer Markt für Stärke bietet sich bei der Herstellung von Mitteln zur Bodenstabilisation an, die bei künstlichen Erdbewegungen zum temporären Schutz der Bodenpartikel gegenüber Wasser eingesetzt werden. Kombinationsprodukte aus Stärke und Polymeremulsionen sind nach heutiger Kenntnis in ihrer Erosions- und verkrustungsmindernden Wirkung den bisher eingesetzten Produkten gleichzusetzen, liegen preislich aber deutlich unter diesen.

### 2.6 Einsatz bei Pflanzenschutz- und Düngemitteln

Ein Einsatzbereich liegt bei der Verwendung der Stärke in Pflanzenschutzmitteln zur Veränderung der spezifischen Eigenschaften der Präparate. So werden Stärken zur Verbesserung der Benetzung von Pflanzenschutz- und Düngemitteln, zur dosierten Freigabe der Wirkstoffe, zur Umwandlung flüssiger, flüchtiger und/oder übelriechender Wirkstoffe in mikrokristalline, stabile, formbare Substanzen, zur Mischung inkompatibler Verbindungen und zur Verlängerung der Wirkdauer durch Verminderung der Zersetzung eingesetzt.

### 2.7 Pharmaka, Medizin und Kosmetikindustrie

Ein weiteres Einsatzgebiet besteht im Bereich der Pharmaka, Medizin und Kosmetikindustrie. In der pharmazeutischen Industrie werden Stärken als Bindemittel für Tabletten oder zur Bindemittelverdünnung in Kapseln eingesetzt. Weiterhin dienen Stärken als Tablettensprengmittel, da sie nach dem Schlucken Flüssigkeit absorbieren und nach kurzer Zeit soweit quellen, daß der Wirkstoff freigesetzt wird. Medizinische Gleit- und Wundpuder basieren aus qualitativen Gründen auf Stärke. Im Bereich der Kosmetik werden Stärken beispielsweise als Träger von Puderzusatzstoffen, wie Düften und Salicylsäure eingesetzt. Ein relativ großer Anwendungsbereich für Stärke liegt bei Zahnpasta.

### 2.8 Stärkezusatz zu Kohle und Brikett

Einen Einsatzbereich bietet die Stärke als Zusatzstoff zu Kohle und Brikett. Kohle kann mit einem Stärkezusatz quantitativ hochwertig agglomeriert bzw. brikettiert werden, wodurch ein frühzeitiges Zerfallen der Briketts verhindert wird. Der Stärkezusatz liegt bei Grillkohle zwischen 4 und 6 %, bei kalorierter Kohle zwischen 0,1 und 0,5 %. Des weiteren gewinnen Stärken als Bindemittel an Bedeutung, da durch ihren Zusatz zu Kohle und Brikett der Ausstoß schädlicher Stoffe deutlich vermindert werden kann.

### 2.9 Erz- und Kohleschlammaufbereitung

Die Stärke kann ferner bei der Erz- und Kohleschlammaufbereitung als Flockungsmittel eingesetzt werden.

### 2.10 Gießereihilfsstoff

Ein weiterer Einsatzbereich besteht als Zusatz zu Gießereihilfsstoffen. Bei verschiedenen Gußverfahren werden Kerne benötigt, die aus Bindemittel-versetzten Sänden hergestellt werden. Als Bindemittel wird heute überwiegend Bentonit eingesetzt, das mit modifizierten Stärken, meist Quellstärken, versetzt ist.
Zweck des Stärkezusatzes ist die Erhöhung der Fließfestigkeit sowie die Verbesserung der Bindefestigkeit. Darüber hinaus können die Quellstärken weitere produktionstechnische Anforderungen, wie im kalten Wasser dispergierbar, rehydratisierbar, gut in Sand mischbar und hohes Wasserbindungsvermögen, aufweisen.

### 2.11 Einsatz in der Kautschukindustrie

In der Kautschukindustrie kann die Stärke zur Verbesserung der technischen und optischen Qualität eingesetzt werden. Gründe sind dabei die Verbesserung des Oberflächenglanzes, die Verbesserung des Griffs und des Aussehens, dafür wird Stärke vor der Kaltvulkanisation auf die klebrigen gummierten Flächen von Kautschukstoffen gestreut, sowie die Verbesserung der Bedruckbarkeit des Kautschuks.

### 2.12 Herstellung von Lederersatzstoffen

Eine weitere Absatzmöglichkeit der modifizierten Stärken besteht bei der Herstellung von Lederersatzstoffen.

### 2.13 Stärke in synthetischen Polymeren

Auf dem Kunststoffsektor zeichnen sich folgende Einsatzgebiete ab: die Einbindung von Stärkefolgeprodukten in den Verarbeitungsprozess (Stärke ist nur Füllstoff, es besteht keine direkte Bindung zwischen synthetischem Polymer und Stärke) oder alternativ die Einbindung von Stärkefolgeprodukten in die Herstellung von Polymeren (Stärke und Polymer gehen eine feste Bindung ein).

Die Verwendung der Stärken als reinem Füllstoff ist verglichen mit den anderen Stoffen wie Talkum nicht wettbewerbsfähig. Anders sieht es aus, wenn die spezifischen Stärkeeigenschaften zum Tragen kommen und hierdurch das Eigenschaftsprofil der Endprodukte deutlich verändert wird. Ein Beispiel hierfür ist die Anwendung von Stärkeprodukten bei der Verarbeitung von Thermoplasten, wie Polyäthylen. Hierbei werden die Stärke und das synthetische Polymer durch Koexpression im Verhältnis von 1 : 1 zu einem 'master batch' kombiniert, aus dem mit granuliertem Polyäthylen unter Anwendung herkömmlicher Verfahrenstechniken diverse Produkte hergestellt werden. Durch die Einbindung von Stärke in Polyäthylenfolien kann eine erhöhte Stoffdurchlässigkeit bei Hohlkörpern, eine verbesserte Wasserdampfdurchlässigkeit, ein verbessertes Antistatikverhalten, ein verbessertes Antiblockverhalten sowie eine verbesserte Bedruckbarkeit mit wäßrigen Farben erreicht werden. Gegenwärtige Nachteile betreffen die ungenügende Transparenz, die verringerte Zugfestigkeit sowie eine verringerte Dehnbarkeit.
Eine andere Möglichkeit ist die Anwendung der Stärke in Polyurethanschäumen. Mit der Adaption der Stärkederivate sowie durch die verfahrenstechnische Optimierung ist es möglich, die Reaktion zwischen synthetischen Polymeren und den Hydroxygruppen der Stärken gezielt zu steuern. Das Ergebnis sind Polyurethanfolien, die durch die Anwendung von Stärke folgende Eigenschaftsprofile erhalten: eine Verringerung des Wärmeausdehnungskoeffizienten, Verringerung des Schrumpfverhaltens, Verbesserung des Druck/Spannungsverhaltens, Zunahme der Wasserdampfdurchlässigkeit ohne Veränderung der Wasseraufnahme, Verringerung der Entflammbarkeit und der Aufrißdichte, kein Abtropfen brennbarer Teile, Halogenfreiheit und verminderte Alterung. Nachteile, die gegenwärtig noch vorhanden sind, sind verringerte Druckfestigkeit sowie eine verringerte Schlagfestigkeit.

Die Produktentwicklung beschränkt sich inzwischen nicht mehr nur auf Folien. Auch feste Kunststoffprodukte, wie Töpfe, Platten und Schalen, sind mit einem Stärkegehalt von über 50 % herzustellen. Des weiteren sind Stärke/ Polymermischungen günstig zu beurteilen, da sie eine sehr viel höhere biologische Abbaubarkeit aufweisen.
Außerordentliche Bedeutung haben weiterhin auf Grund ihres extremen Wasserbindungsvermögen Stärkepfropfpolymerisate gewonnen. Dies sind Produkte mit einem Rückgrat aus Stärke und einer nach dem Prinzip des Radikalkettenmechanismus aufgepfropften Seitengitters eines synthetischen Monomers. Die heute verfügbaren Stärkepfropfpolymerisate zeichnen sich durch ein besseres Binde- und Rückhaltevermögen von bis zu 1000 g Wasser pro g Stärke bei hoher Viskosität aus. Die Anwendungsbereiche für diese Superabsorber haben sich in den letzten Jahren stark ausgeweitet und liegen im Hygienebereich mit Produkten Windeln und Unterlagen sowie im landwirtschaftlichen Sektor, z.B. bei Saatgutpillierungen.

Entscheidend für den Einsatz der neuen, gentechnisch veränderten Stärken sind zum einen die Struktur, Wassergehalt, Proteingehalt, Lipidgehalt, Fasergehalt, Asche/Phosphatgehalt, Amylose/Amylopektinverhältnis, Molmassenverteilung, Verzweigungsgrad, Korngröße und -form sowie Kristallisation, zum anderen auch die Eigenschaften, die in folgende Merkmale münden: Fließ- und Sorptionsverhalten, Verkleisterungstemperatur, Viskosität, Dickungsleistung, Löslichkeit, Kleisterstruktur, Transparenz, Hitze-, Scher- und Säurestabilität, Retrogradationsneigung, Gelbildung, Gefrier/Taustabilität, Komplexbildung, Jodbindung, Filmbildung, Klebekraft, Enzymstabilität, Verdaulichkeit und Reaktivität.
Die Erzeugung modifizierter Stärken mittels gentechnischer Eingriffe in einer transgenen Pflanze kann zum einen die Eigenschaften der aus der Pflanze gewonnenen Stärke dahingehend verändern, daß weitere Modifikationen mittels chemischer oder physikalischer Verfahren nicht mehr notwendig erscheinen. Zum anderen können die durch gentechnische Verfahren veränderte Stärken weiteren chemischen Modifikationen unterworfen werden, was zu weiteren Verbesserungen der Qualität für bestimmte der oben beschriebenen Einsatzgebiete führt. Diese chemischen Modifikationen sind grundsätzlich bekannt. Insbesondere handelt es sich dabei um Modifikationen durch
- Hitzebehandlung,
- Säurebehandlung,
- Oxidation und
- Veresterungen, welche zur Entstehung von Phosphat-, Nitrat-, Sulfat-, Xanthat-, Acetat- und Citratstärken führen. Weitere organische Säuren können ebenfalls zur Veresterung eingesetzt werden:

- Erzeugung von Stärkeethern Stärke-Alkylether, O-Allylether, Hydroxylalkylether, O-Carboxylmethylether, N-haltige Stärkeether, P-haltige Stärkeether, S-haltige Stärkeether
- Erzeugung von vernetzten Stärken
- Erzeugung von Stärke-Pfropf-Polymerisaten

Zur Expression der erfindungsgemäßen DNA-Moleküle in sense- oder antisense-Orientierung in pflanzlichen Zellen werden diese mit regulatorischen DNA-Elementen verknüpft, die die Transkription in pflanzlichen Zellen gewährleisten. Hierzu zählen insbesondere Promotoren.

Der Promotor kann dabei so gewählt sein, daß die Expression konstitutiv erfolgt oder nur in einem bestimmten Gewebe, zu einem bestimmten Zeitpunkt der Pflanzenentwicklung oder zu einem durch äußere Einflüsse determinierten Zeitpunkt. In Bezug auf die Pflanze kann der Promotor homolog oder heterolog sein. Sinnvolle Promotoren sind z.B. der Promotor der 35S RNA des Cauliflower Mosaic Virus für eine konstitutive Expression, der Patatingen-Promotor B33 (Rocha-Sosa et al., EMBO J. 8 (1989), 23-29) für eine knollenspezifische Expression in Kartoffeln oder ein Promotor, der eine Expression lediglich in photosynthetisch aktiven Geweben sicherstellt, z.B. der ST-LS1-Promotor (Stockhaus et al., Proc. Natl. Acad. Sci. USA 84 (1987), 7943-7947; Stockhaus et al., EMBO J. 8 (1989), 2445-2451) oder für eine endosperm-spezifische Expression der HMG-Promotor aus Weizen oder Promotoren von Zein-Genen aus Mais.

Ferner kann eine Terminationssequenz vorhanden sein, die der korrekten Beendigung der Transkription dient sowie der Addition eines Poly-A-Schwanzes an das Transkript, dem eine Funktion bei der Stabilisierung der Transkripte beigemessen wird. Derartige Elemente sind in der Literatur beschrieben (vgl. Gielen et al., EMBO J. 8 (1989), 23-29) und sind beliebig austauschbar.

Prinzipiell ist es erfindungsgemäß möglich, Pflanzen herzustellen, bei denen nur die Aktivität einer Isoform der SSS bzw. der GBSS II verändert ist, als auch Pflanzen, bei denen gleichzeitig die Aktivitäten mehrerer Stärkesynthaseformen verändert sind. Dabei sind alle Kombinationen und Permutationen denkbar.
Durch die Veränderung der Aktivitäten einer oder mehrerer Isoformen der Stärkesynthasen in Pflanzen kommt es zur Synthese einer in ihrer Struktur veränderten Stärke.
Durch die Steigerung der Aktivität einer oder mehrerer Isoformen der Stärkesynthasen in den Zellen der stärkespeichernden Gewebe transformierter Pflanzen wie z.B. in der Knolle bei der Kartoffel oder in dem Endosperm von Mais oder Weizen kann es darüber hinaus zu einer Ertragssteigerung kommen.

Da die GBSS I aus Kartoffel codierende DNA-Sequenz bereits bekannt ist (Visser et al., Plant Sci. 64 (1989), 185-192), stehen somit für alle bisher in Kartoffel identifizierten Stärkesynthasen codierende DNA-Sequenzen zur Verfügung. Dies erlaubt nun sowohl die Identifizierung der Funktion der einzelnen Isoformen bei der Stärkebiosynthese, als auch die Herstellung gentechnisch veränderter Pflanzen, bei denen die Aktivitäten eines oder mehrerer dieser Enzyme verändert sind. Dies ermöglicht die Synthese einer Stärke mit veränderter Struktur und somit veränderten physikalisch-chemischen Eigenschaften in derartig manipulierten Pflanzen.
Die erfindungsgemäßen DNA-moleküle können daher auch dazu verwendet werden, Pflanzen herzustellen, bei denen die Aktivität der benannten Stärkesynthasen erhöht oder verringert ist und gleichzeitig die Aktivitäten anderer, an der Stärkebiosynthese beteiligter Enzyme verändert sind. Es sind dabei alle möglichen Kombinationen denkbar. Beispielsweise können gemäß dem beschriebenen Verfahren DNA-Sequenzen, die SSS-Proteine oder GBSS II codieren, in Pflanzenzellen eingebracht werden, bei denen bereits die Synthese endogener GBSS I-Proteine aufgrund eines antisense-Effektes inhibiert ist (wie beschrieben in Visser et al., Mol. Gen. Genet. 225 (1991), 289-296) oder die Synthese des Verzweigungsenzyms inhibiert ist (wie beschrieben in WO92/14827).
Soll die Inhibierung der Synthese mehrerer Stärke-Synthasen in transformierten Pflanzen erreicht werden, so können DNA-Moleküle zur Transformation verwendet werden, die gleichzeitig mehrere, die entsprechenden Stärkesynthasen codierenden Regionen in antisense-Orientierung unter der Kontrolle eines geeigneten Promotors enthalten. Hierbei kann alternativ jede Sequenz unter der Kontrolle eines eigenen Promotors stehen, oder die Sequenzen können als Fusion von einem gemeinsamen Promotor transkribiert werden. Letztere Alternative wird in der Regel vorzuziehen sein, da in diesem Fall die Synthese der entsprechenden Proteine in etwa gleichem Maße inhibiert werden sollte.

Weiterhin ist die Konstruktion von DNA-Molekülen möglich, bei denen neben DNA-Sequenzen, die Stärke-Synthasen codieren, weitere DNA-Sequenzen, die andere Proteine, die an der Stärkesynthese oder -modifikation beteiligt sind, in antisense-Orienierung an einen geeigneten Promotor gekoppelt sind. Die Sequenzen können hierbei wiederum hintereinandergeschaltet sein und von einem gemeinsamen Promotor transkribiert werden. Für die Länge der einzelnen codierenden Regionen, die in einem derartigen Konstrukt verwendet werden, gilt das, was oben bereits für die Herstellung von anti-sense-Konstrukten ausgeführt wurde. Eine obere Grenze für die Anzahl der in einem derartigen DNA-Molekül von einem Promotor aus transkribierten antisense-Fragmente gibt es nicht. Das entstehende Transkript sollte aber in der Regel eine Länge von 10 kb, vorzugsweise von 5 kb nicht überschreiten.
Codierende Regionen, die in derartigen DNA-Molekülen in Kombination mit anderen codierenden Regionen in antisense-Orientierung hinter einem geeigneten Promotor lokalisiert sind, können aus DNA-Sequenzen stammen, die für folgende Proteine codieren: Stärkekorn-gebundene (GBSS I und II) und lösliche Stärkesynthasen (SSS I und II), Verzweigungsenzyme (Koßmann et al., Mol. Gen. Genet. 230 (1991), 39-44), "Debranching"-Enzyme (R-Enzyme), Disproportionierungsenzyme (Takaha et al., J. Biol. Chem. 268 (1993), 1391-1396) und Stärkephosphorylasen. Dies ist nur eine beispielhafte Aufzählung. Auch die Verwendung anderer DNA-Sequenzen im Rahmen einer derartigen Kombination ist denkbar.
Mit Hilfe derartiger Konstrukte ist es möglich, in Pflanzenzellen, die mit diesen transformiert wurden, die Synthese mehrerer Enzyme gleichzeitig zu inhibieren.

Zur Vorbereitung der Einführung fremder Gene in höhere Pflanzen stehen eine große Anzahl von Clonierungsvektoren zur Verfügung, die ein Replikationssignal für E.coli und ein Markergen zur Selektion transformierter Bakterienzellen enthalten. Beispiele für derartige Vektoren sind pBR322, pUC-Serien, M13mp-Serien, pACYC184 usw. Die gewünschte Sequenz kann an einer passenden Restriktionsschnittstelle in den Vektor eingeführt werden. Das erhaltene Plasmid wird für die Transformation von *E.coli*-Zellen verwendet. Transformierte *E.coli*-Zellen werden in einem geeigneten Medium gezüchtet, anschließend geerntet und lysiert. Das Plasmid wird wiedergewonnen. Als Analysemethode zur Charakterisierung der gewonnenen Plasmid-DNA werden im allgemeinen Restriktionsanalysen, Gelelektrophoresen und weitere biochemisch-molekularbiologische Methoden eingesetzt. Nach jeder Manipulation kann die Plasmid DNA gespalten und gewonnene DNA-Fragmente mit anderen DNA-Sequenzen verknüpft werden. Jede Plasmid-DNA-Sequenz kann in den gleichen oder anderen Plasmiden cloniert werden.
Für die Einführung von DNA in eine pflanzliche Wirtszelle stehen eine Vielzahl von Techniken zur Verfügung. Diese Techniken umfassen die Transformation pflanzlicher Zellen mit T-DNA unter Verwendung von *Agrobacterium tumefaciens* oder *Agrobacterium rhizogenes* als Transformationsmittel, die Fusion von Protoplasten, die Injektion, die Elektroporation von DNA, die Einbringung von DNA mittels der biolistischen Methode sowie weitere Möglichkeiten.
Bei der Injektion und Elektroporation von DNA in Pflanzenzellen werden an sich keine speziellen Anforderungen an die verwendeten Plasmide gestellt. Es können einfache Plasmide wie z.B. pUC-Derivate verwendet werden. Sollen aber aus derartig transformierten Zellen ganze Pflanzen regeneriert werden, ist die Anwesenheit eines selektierbaren Markergens notwendig.
Je nach Einführungsmethode gewünschter Gene in die Pflanzenzelle können weitere DNA-Sequenzen erforderlich sein. Werden z.B. für die Transformation der Pflanzenzelle das Ti- oder Ri-Plasmid verwendet, so muß mindestens die rechte Begrenzung, häufig jedoch die rechte und linke Begrenzung der Ti- und Ri-Plasmid T-DNA als Flankenbereich mit den einzuführenden Genen verbunden werden.

Werden für die Transformation Agrobakterien verwendet, muß die einzuführende DNA in spezielle Plasmide cloniert werden, und zwar entweder in einen intermediären Vektor oder in einen binären Vektor. Die intermediären Vektoren können aufgrund von Sequenzen, die homolog zu Sequenzen in der T-DNA sind, durch homologe Rekombination in das Ti- oder Ri-Plasmid der Agrobakterien integriert werden. Dieses enthält außerdem die für den Transfer der T-DNA notwendige vir-Region. Intermediäre Vektoren können nicht in Agrobakterien replizieren. Mittels eines Helferplasmids kann der intermediäre Vektor auf *Agrobacterium tumefaciens* übertragen werden (Konjugation). Binäre Vektoren können sowohl in E.coli als auch in Agrobakterien replizieren. Sie enthalten ein Selektionsmarker-Gen und einen Linker oder *Polylinker*, welche von der rechten und linken T-DNA Grenzregion eingerahmt werden. Sie können direkt in die Agrobakterien transformiert werden (Holsters et al. Mol. Gen. Genet. 163 (1978), 181-187). Das als Wirtszelle dienende Agrobakterium soll ein Plasmid, das eine vir-Region trägt, enthalten. Die vir-Region ist für den Transfer der T-DNA in die Pflanzenzelle notwendig. Zusätzliche T-DNA kann vorhanden sein. Das derartig transformierte Agrobakterium wird zur Transformation von Pflanzenzellen verwendet.
Die Verwendung von T-DNA für die Transformation von Pflanzenzellen ist intensiv untersucht und ausreichend in EP 120516; Hoekema, In: The Binary Plant Vector System Offsetdrukkerij Kanters B.V., Alblasserdam (1985), Chapter V; Fraley et al., Crit. Rev. Plant. Sci., 4: 1-46 und An et al. EMBO J. 4 (1985), 277-287 beschrieben worden.
Für den Transfer der DNA in die Pflanzenzelle können Pflanzen-Explantate zweckmäßigerweise mit *Agrobacterium tumefaciens* oder *Agrobacterium rhizogenes* kokultiviert werden. Aus dem infizierten Pflanzenmaterial (z.B. Blattstücke, Stengelsegmente, Wurzeln, aber auch Protoplasten oder Suspensions-kultivierte Pflanzenzellen) können dann in einem geeigneten Medium, welches Antibiotika oder Biozide zur Selektion transformierter Zellen enthalten kann, wieder ganze Pflanzen regeneriert werden. Die so erhaltenen Pflanzen können dann auf Anwesenheit der eingeführten DNA untersucht werden. Andere Möglichkeiten der Einführung fremder DNA unter Verwendung des biolistsischen Verfahrens oder durch Protoplastentransformation sind bekannt (vergl. z.B. Willmitzer, L., 1993 Transgenic plants. In: Biotechnology, A Multi-Volume Comprehensive Treatise (H.J. Rehm, G. Reed, A. Pühler, P. Stadler, eds.), Vol. 2, 627-659, VCH Weinheim-New York-Basel-Cambridge).
Ist die eingeführte DNA einmal im Genom der Pflanzenzelle integriert, so ist sie dort in der Regel stabil und bleibt auch in den Nachkommen der ursprünglich transformierten Zelle erhalten. Sie enthält normalerweise einen Selektionsmarker, der den transformierten Pflanzenzellen Resistenz gegenüber einem Biozid oder einem Antibiotikum wie Kanamycin, G 418, Bleomycin, Hygromycin oder Phosphinotricin u.a. vermittelt. Der individuelle gewählte Marker sollte daher die Selektion transformierter Zellen gegenüber Zellen, denen die eingeführte DNA fehlt, gestatten.
Die transformierten Zellen wachsen innerhalb der Pflanze in der üblichen Weise (siehe auch McCormick et al. (1986) Plant Cell Reports 5:81-84). Die resultierenden Pflanzen können normal angezogen werden und mit Pflanzen, die die gleiche transformierte Erbanlage oder andere Erbanlagen besitzen, gekreuzt werden. Die daraus entstehenden hybriden Individuen haben die entsprechenden phänotypischen Eigenschaften.
Es sollten zwei oder mehrere Generationen angezogen werden, um sicherzustellen, daß das phänotypische Merkmal stabil beibehalten und vererbt wird. Auch sollten Samen geerntet werden, um sicherzustellen, daß der entsprechende Phänotyp oder andere Eigenarten erhalten geblieben sind.

Das im Rahmen der vorliegenden Erfindung verwendete Plasmid pBinARHyg wurde bei der als internationale Hinterlegungsstelle anerkannten Deutschen Sammlung von Mikroorganismen (DSM) in Braunschweig, Bundesrepublik Deutschland, entsprechend den Anforderungen des Budapester Vertrages für die internationale Anerkennung der Hinterlegung von Mikroorganismen zum Zwecke der Patentierung am 20.10.1994 unter der Nummer DSM 9505 hinterlegt.

### Verwendete Abkürzungen

- bp: Basenpaar
- GBSS: granule bound starch synthase (Stärkekorngebundene Stärkesynthase)
- IPTG: Isopropyl β-D-Thiogalacto-Pyranosid
- SSS: soluble starch synthase (lösliche Stärkesynthase)
- PMSF: Phenylmethylsulfonylfluorid
- VK: Vollängeclon

In den Beispielen verwendete Medien und Lösungen:
- 20 x SSC: 175,3 g NaCl
88,2 g Natrium-Citrat
ad 1000 ml mit ddH₂O
pH 7,0 mit 10 N NaOH
- Puffer A: 50 mM Tris-HCl pH 8,0
2,5 mM DTT
2 mM EDTA
0,4 mM PMSF
10 % Glycerin
0,1 % Natriumdithionit
- Puffer B: 50 mM Tris-HCl pH 7,6
2,5 mM DTT
2 mM EDTA
- Puffer C: 0,5 M Natriumcitrat pH 7,6
50 mM Tris-HCl pH 7,6
2,5 mM DTT
2 mM EDTA
- 10 x TBS: 0,2 M Tris-HCl pH 7,5
5,0 M NaCl
- 10 x TBST: 10 x TBS
0,1 % (Vol/Vol) Tween 20
- Elutionspuffer: 25 mM Tris pH 8,3
250 mM Glycin
- Dialysepuffer: 50 mM Tris-HCl pH 7,0
50 mM NaCl
2 mM EDTA
14,7 mM β-Mercaptoethanol
0,5 mM PMSF
- Proteinpuffer: 50 mM Natriumphosphatpuffer pH 7,2
10 mM EDTA
0,5 mM PMSF
14,7 mM β-Mercaptoethanol

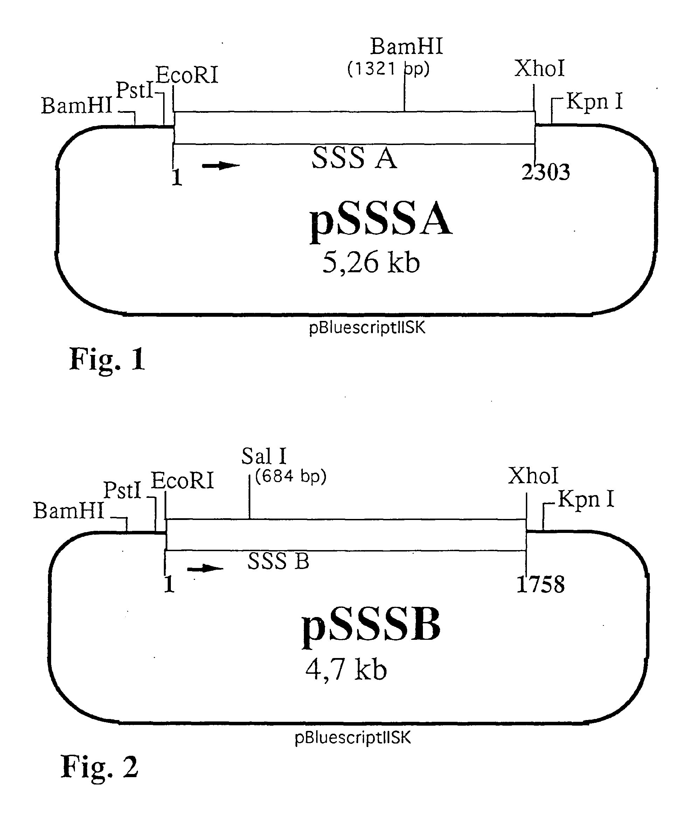

**Fig. 1** zeigt das Plasmid pSSSA
   Die fein gezogene Linie entspricht der Sequenz von pBluescript II SK(-). Die starke Linie repräsentiert die cDNA, die die Isoform SSS A aus *Solanum tuberosum* codiert. Restriktionsschnittstellen der Insertion sind angegeben. Die cDNA-Insertion ist zwischen die *EcoR I*- und *Xho I*-Schnittstellen des Polylinkers des Plasmids ligiert. Die DNA-Sequenz der cDNA-Insertion ist unter Seq ID No. 1 angegeben.
**Fig. 2** zeigt das Plasmid pSSSB
   Die fein gezogene Linie entspricht der Sequenz von pBluescript II SK(-). Die starke Linie repräsentiert die cDNA, die die Isoform SSS B aus *Solanum tuberosum* codiert. Restriktionsschnittstellen der Insertion sind angegeben. Die cDNA-Insertion ist zwischen die EcoR *I-* und *Xho I*-Schnittstellen des Polylinkers des Plasmids ligiert. Die DNA-Sequenz der cDNA-Insertion ist unter Seq ID No. 2 angegeben.

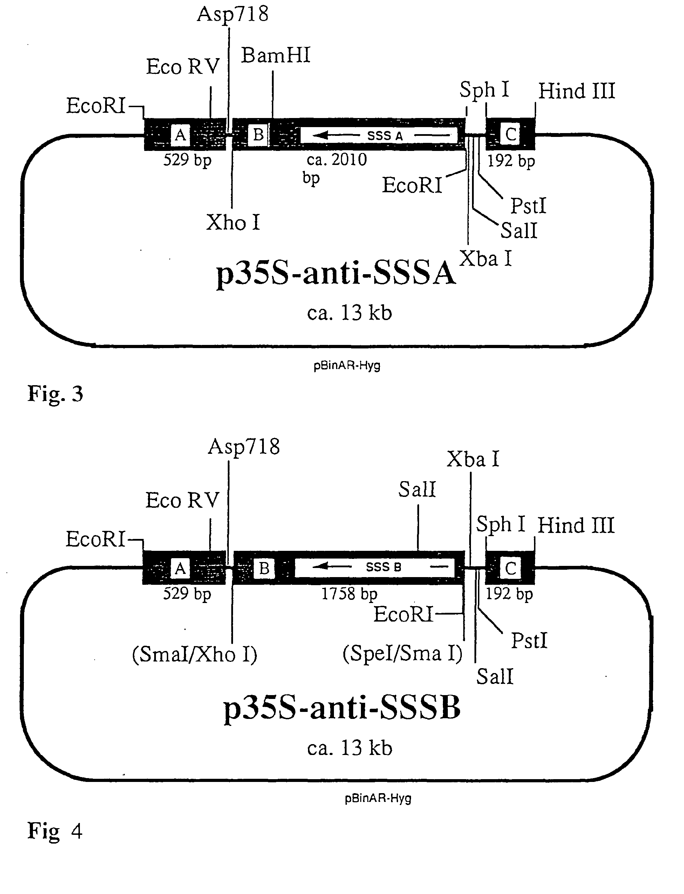

**Fig 3** zeigt das Plasmid p35S-anti-SSSA
   Aufbau des Plasmids:
   - A =: Fragment A: CaMV 35S-Promotor, nt 6909-7437 (Franck et al., Cell 21 (1980), 285-294)
   - B =: Fragment B: cDNA aus *Solanum tuberosum* codierend für lösliche Stärkesynthase; Isoform SSSA; *Xba I*/*Asp718*-Fragment aus pSSSA, ca. 2,1 kb Orientierung zum Promotor: antisense
   - C =: Fragment C: nt 11748-11939 der T-DNA des Ti-Plasmids pTiACH5 (Gielen et al., EMBO J. 3 (1984), 835-846)
Fig. 4 zeigt das Plasmid p35S-anti-SSSB
   Aufbau des Plasmids:
   - A =: Fragment A: CaMV 35S-Promotor, nt 6909-7437 (Franck et al., Cell 21 (1980), 285-294)
   - B =: Fragment B: cDNA aus *Solanum tuberosum* codierend für lösliche Stärkesynthase; Isoform SSSB; *Xho I*/*Spe I*-Fragment aus pSSSB, ca. 1,8 kb Orientierung zum Promotor: antisense
   - C =: Fragment C: nt 11748-11939 der T-DNA des Ti-Plasmids pTiACH5 (Gielen et al.; EMBO J. 3 (1984), 835-846)

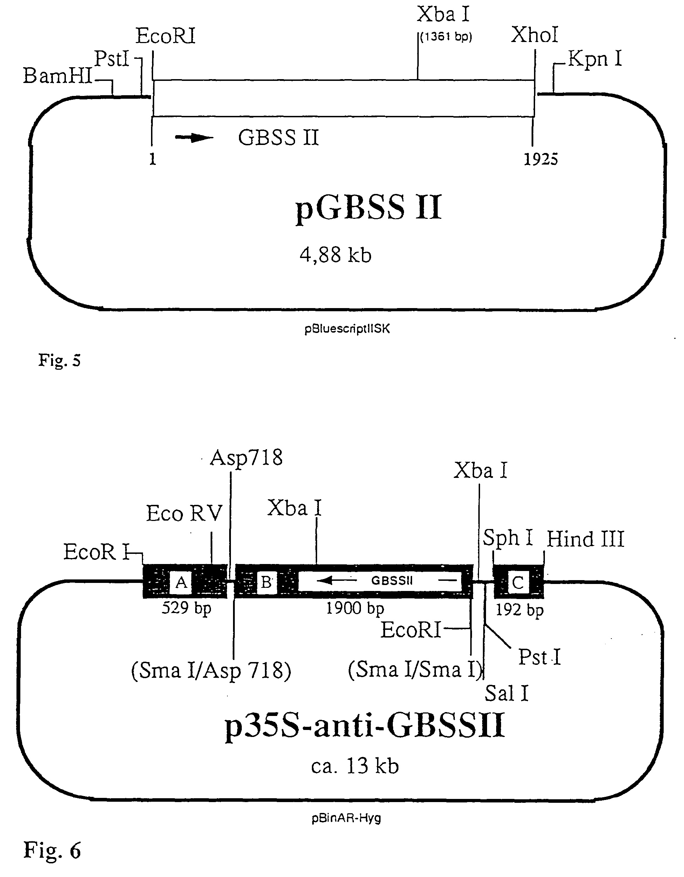

Fig. 5 zeigt das Plasmid pGBSSII
   Die fein gezogene Linie entspricht der Sequenz von pBluescript II SK(-). Die starke Linie repräsentiert die cDNA, die die Isoform GBSS II aus *Solanum tuberosum* codiert. Restriktionsschnittstellen der Insertion sind angegeben. Die cDNA-Insertion ist zwischen die EcoR *I*- und *Xho I*-Schnittstellen des Polylinkers des Plasmids ligiert. Die DNA-Sequenz der cDNA-Insertion ist unter Seq ID No. 3 angegeben.
**Fig. 6** zeigt das Plasmid p35S-anti-GBSSII
   Aufbau des Plasmids:
   - A =: Fragment A: CaMV 35S-Promotor, nt 6909-7437 (Franck et al., Cell 21 (1980), 285-294)
   - B =: Fragment B: cDNA aus *Solanum tuberosum* codierend für Stärkekorn-gebundene Stärkesynthase; Isoform GBSS II;
   *Sma I*/*Asp 718*-Fragment aus pGBSS II, ca. 1,9 kb Orientierung zum Promotor: antisense
   - C =: Fragment C: nt 11748-11939 der T-DNA des Ti-Plasmids pTiACH5 (Gielen et al., EMBO J. 3 (1984) 835-846)

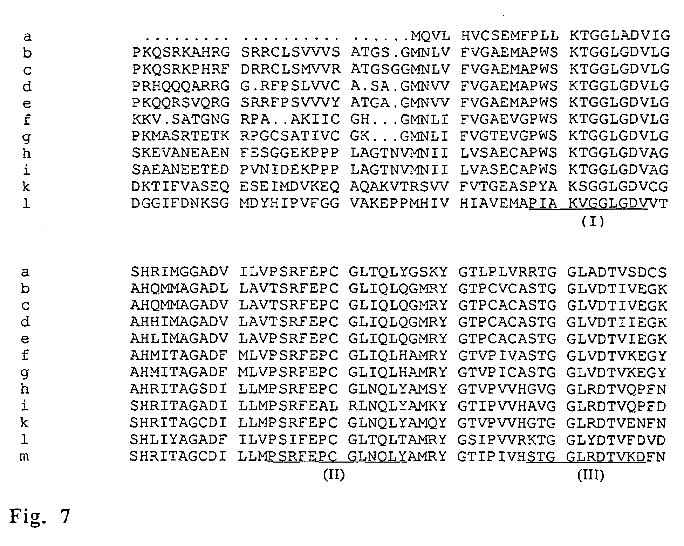

**Fig. 7** zeigt einen partiellen Vergleich der Aminosäuresequenzen von prokaryontischen Glycogensynthasen, Stärkekorn-gebundenen Stärkesynthasen und löslichen Stärkesynthasen aus verschiedenen Organismen.
   - a:: Glycogensynthase aus *E. coli*
   - b:: GBSS I aus Gerste
   - c:: GBSS I aus Weizen
   - d:: GBSS I aus Mais
   - e:: GBSS I aus Reis
   - f:: GBSS I aus Maniok
   - g:: GBSS I aus Kartoffel
   - h:: GBSS II aus Erbse
   - i:: GBSS II aus Kartoffel
   - k:: SSS aus Reis
   - l:: SSS A aus Kartoffel
   - m:: SSS B aus Kartoffel

   Die markierten Bereiche (I), (II) und (III) geben drei Peptidsequenzen an, die zwischen den verschiedenen Stärkesynthasen bzw. Glycogensynthasen stark konserviert sind.

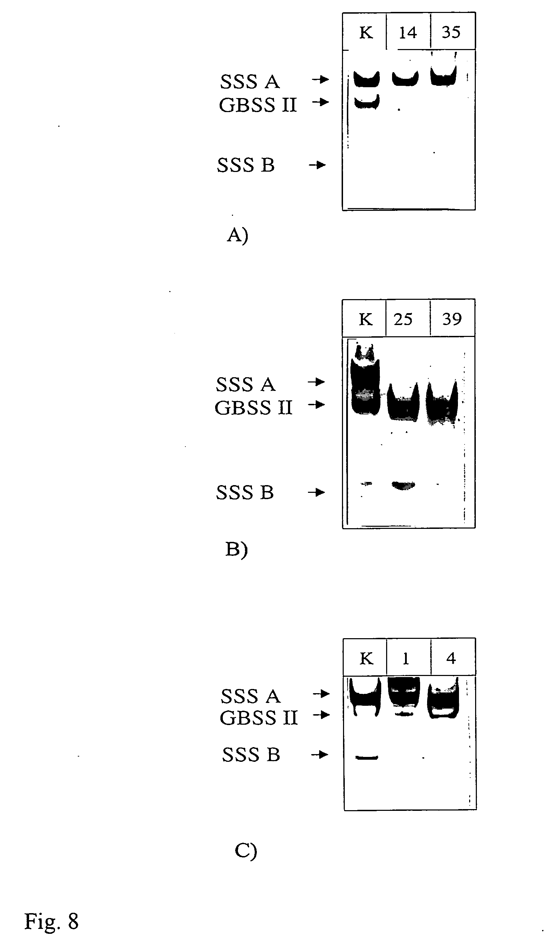

**Fig. 8** zeigt Aktivitäts-Gele der löslichen Stärkesynthase-Isoformen aus Knollenextrakten von Wildtyp- und Stärkesynthase-"Antisense"-Kartoffelpflanzen.
   A) GBSS II-"Antisense"-Pflanze, Linie 14 und 35, K = Wildtyp-Pflanze
   B) SSS A-"Antisense"-Pflanze, Linie 25 und 39, K = Wildtyp-Pflanze
   C) SSS B-"Antisense"-Pflanze, Linie 1 und 4, K = Wildtyp-Pflanze

Je 50 µg des Proteinextraktes wurden auf einem 7,5%igen nativen Gel getrennt und die Aktivitäten der Synthase-Isoformen im Citrat-stimulierten Ansatz mit 0,1 % Amylopektin als "Primer" bestimmt. Die synthetisierten Glucane wurden mit Lugolscher Lösung angefärbt.

Die Beispiele erläutern die Erfindung.

In den Beispielen werden die folgenden Methoden verwendet:

### 1. Clonierungsverfahren

Zur Clonierung in *E.coli* wurde der Vektor pBluescript II SK (Stratagene) verwendet.
Für die Pflanzentransformation wurden die Genkonstruktionen in den binären Vektor pBinAR Hyg (DSM 9505) cloniert.

### 2. Bakterienstämme

Für den Bluescript-Vektor und für die pBinAR Hyg-Konstrukte wurde der *E.coli-*Stamm DH5α (Bethesda Research Laboratories, Gaithersburgh, USA) verwendet. Für die in *vivo excision* wurde der E.coli-Stamm XL1-Blue verwendet.
Die Transformation der Plasmide in die Kartoffelpflanzen wurde mit Hilfe des *Agrobacterium tumefaciens*-Stammes C58C1 pGV2260 durchgeführt (Deblaere et al., Nucl. Acids Res. 13 (1985), 4777-4788).

### 3. Transformation von Agrobacterium tumefaciens

Der Transfer der DNA erfolgte durch direkte Transformation nach der Methode von Höfgen&Willmitzer (Nucl. Acids Res. 16 (1988), 9877). Die Plasmid-DNA transformierter Agrobakterien wurde nach der Methode von Birnboim&Doly (Nucl. Acids Res. 7 (1979), 1513-1523) isoliert und nach geeigneter Restriktionsspaltung gelelektrophoretisch analysiert.

### 4. Transformation von Kartoffeln

Zehn kleine mit dem Skalpell verwundete Blätter einer Kartoffel-Sterilkultur (Solanum tuberosum L.cv. Desiree) wurden in 10 ml MS-Medium (Murashige&Skoog, Physiol. Plant. 15 (1962), 473) mit 2 % Saccharose gelegt, welches 50 µl einer unter Selektion gewachsenen *Agrobacterium* tumefaciens-Übernachtkultur enthielt. Nach 3-5 minütigem, leichtem Schütteln erfolgte eine weitere Inkubation für 2 Tage im Dunkeln. Daraufhin wurden die Blätter zur Kallusinduktion auf MS-Medium mit 1,6 % Glucose, 5 mg/l Naphthylessigsäure, 0,2 mg/l Benzylaminopurin, 250 mg/l Claforan, 50 mg/l Kanamycin, und 0,80 % Bacto Agar gelegt. Nach einwöchiger Inkubation bei 25°C und 3000 Lux wurden die Blätter zur Sproßinduktion auf MS-Medium mit 1,6 % Glucose, 1,4 mg/l Zeatinribose, 20 mg/l Naphthylessigsäure, 20 mg/l Giberellinsäure, 250 mg/l Claforan, 50 mg/l Kanamycin, und 0,80 % Bacto Agar gelegt.

### 5. Radioaktive Markierung von DNA-Fragmenten

Die radiokative Markierung von DNA-Fragmenten wurde mit Hilfe eines DNA-Random Primer Labelling Kits der Firma Boehringer (Deutschland) nach den Angaben des Herstellers durchgeführt.

### 6. Bestimmung der Stärkesynthase-Aktivität

Die Bestimmung der Stärkesynthaseaktivität erfolgte durch Bestimmung des Einbaus von ¹⁴C-Glucose aus ADP[¹⁴C-Glucose] in ein in Methanol/KCl unlösliches Produkt wie beschrieben in Denyer und Smith (Planta 186 (1992), 609-617).

### 7. Nachweis von löslichen Stärkesynthasen im nativen Gel

Zum Nachweis der Aktivität löslicher Stärkesynthasen durch nicht-denaturierende Gelelektrophorese wurden Gewebeproben von Kartoffelknollen in 50 mM Tris-HCl pH 7,6, 2 mM DTT, 2,5 mM EDTA, 10 % Glycerin und 0,4 mM PMSF aufgeschlossen. Die Elektrophorese wurde in einer MiniProtean II Kammer (BioRAD) durchgeführt. Die Monomerkonzentration der 1,5 mm dicken Gele war 7,5 % (Gew./Vol.), und als Gel- wie auch Laufpuffer diente 25 mM Tris-Glycin pH 8,4. Gleiche Mengen an Proteinextrakt wurden aufgetragen und für 2 h bei 10 mA je Gel aufgetrennt.
Anschließend erfolgte die Inkubation der Aktivitäts-Gele in 50 mM Tricine-NaOH pH 8,5, 25 mM Kaliumacetat, 2 mM EDTA, 2 mM DTT, 1 mM ADP-Glucose, 0,1 % (Gew./Vol.) Amylopektin und 0,5 M Natriumcitrat. Gebildete Glucane wurden mit Lugolscher Lösung angefärbt.

### 8. Stärkeanalytik

Die von den transgenen Kartoffelpflanzen gebildete Stärke wurde durch folgende Methoden charakterisiert:
a) Bestimmung des Phosphatgehaltes
   In der Kartoffelstärke können einige Glucoseeinheiten an den Kohlenstoffatomen der Position C3 und C6 phosphoryliert sein. Zur Bestimmung des Phosphorylierungsgrades an der C6-Position der Glucose wurden 100 mg Stärke in 1 ml 0,7 M HCl für 4 Stunden bei 95°C hydrolysiert (Nielsen et al., Plant Physiol. 105 (1994), 111-117). Nach Neutralisation mit 0,7 M KOH wurden zur Glucose-6-phosphat-Bestimmung 50 µl des Hydrolysats einem optisch-enzymatischen Test unterzogen. Die Änderung der Absorption des Testansatzes (100 mM Imidazol/HCl; 10 mM MgCl₂; 0,4 mM NAD; 2 Units Glucose-6-phosphat-Dehydrogenase aus *Leuconostoc mesenteroides;* 30°C) wurde bei 334 nm verfolgt.
b) Analyse der Seitenkettenlängenverteilung
   Zur Analyse der Seitenketten der Stärkemoleküle wurde 1 ml einer 0,1%igen Stärkelösung mit ca. 1 Unit Isoamylase über Nacht bei 37°C in 100 mM Na-Citrat-Puffer, pH 4,0 verdaut (Y.C. Lee, Analytical Biochemistry 189 (1990), 151-162). Die Trennung der einzelnen Glucanketten erfolgte mittels eines komplexen Gradienten über HPLC (Säule PA1; Laufmittel 150 mM NaOH mit Na-Acetat-Gradienten).
c) Korngrößenbestimmung
   Die Korngrößenbestimmung wurde mit einem Fotosedimentometer des Typs "Lumosed" der Firma Retsch GmbH, Deutschland, durchgeführt. Hierfür wurden 0,2 g Stärke in ca. 150 ml Wasser suspendiert und sofort vermessen. Das vom Hersteller mitgelieferte Programm berechnete den mittleren Durchmesser der Stärkekörner auf der Annahme einer durchschnittlichen Dichte der Stärke von 1,5 g/l.
d) Verkleisterungseigenschaften
   Die Verkleisterungskurven der Stärke wurden mit einem Viskograph E der Firma Brabender oHG, Deutschland, oder mit einem Rapid Visco Analyser, Newport Scientific Pty Ltd, Investment Support Group, Warriewood NSW 2102, Australien, aufgezeichnet. Bei Verwendung des Viskographen E wurde eine Suspension von 30 g Stärke in 450 ml Wasser folgendem Heizprogramm unterzogen: aufheizen von 50°C auf 96°C mit 3°/min, 30 Minuten konstant halten, abkühlen auf 30°C mit 3°/min und abermals 30 Minuten konstant halten . Das Temperaturprofil lieferte charakteristische Verkleisterungseigenschaften.
   Bei Messung mittels des Rapid Visco Analysers wurde eine Suspension von 2 g Stärke in 25 ml Wasser folgendem Heizprogramm unterzogen: 50 s bei 50°C suspendieren, aufheizen von 50°C auf 95°C mit 12°/min, 2,5 Minuten konstant halten, abkühlen auf 50°C mit 16,4°/min und abermals 2 Minuten konstant halten. Das Temperaturprofil lieferte die maximale und Endviskosität sowie die Verkleisterungstemperatur.

### Beispiel 1

### Identifizierung, Isolierung und Charakterisierung zweier cDNAs, die die Isoformen SSS B und GBSS II der Stärkesynthasen aus Solanum tuberosum codieren

Zwar wurden SSS-Proteine bereits in einer ganzen Reihe von Pflanzenspezies, u.a. in Kartoffel, nachgewiesen und cDNA-Sequenzen für SSS-Proteine aus Reis beschrieben (Baba et al., s.o.), jedoch ist bisher die Reinigung dieser Proteine aus Kartoffel oder anderen Pflanzen sowie die Identifizierung entsprechender DNA-Sequenzen nicht gelungen. Die Problematik bei der Isolierung derartiger DNA-Sequenzen besteht darin, daß die homogene Reinigung lösliche Stärkesynthasen aus technischen Gründen trotz zahlreicher Versuche bisher erfolglos blieb. Die löslichen Synthasen kopurifizieren in allen Reinigungsschritten mit dem Verzweigungsenzym und anderen Verunreinigungen. Für die Bestimmung partieller Aminosäuresequenzen sind diese Proteine daher bislang nicht zugänglich. Daher ist es sehr schwierig, cDNA-Sequenzen durch Hybridisierung mit aus der Aminosäuresequenz abgeleiteten degenerierten Oligonucleotiden zu identifizieren. Ebenso besteht aus denselben Gründen nicht die Möglichkeit, Antikörper zu entwickeln, die diese Enzyme spezifisch erkennen und somit für die Durchmusterung von Expressionsbanken eingesetzt werden könnten.
Die Isolierung von DNA-Sequenzen, die für SSS-Proteine aus Kartoffel codieren, mit Hilfe der Hybridisierung mit heterologen Proben, die lösliche Stärkesynthasen aus anderen Pflanzenspezies codieren, setzt voraus, daß eine ausreichend hohe Homologie besteht und gleichzeitig keine signifikanten Homologien zu anderen codierenden DNA-Sequenzen vorliegen. Im Fall der einzigen zur Verfügung stehenden heterologen DNA-Sequenz aus Reis (Baba et al., s.o.) war jedoch bekannt, daß diese hohen Homologien zu den Stärkekorn-gebundenen Stärkesynthasen aus Reis sowie zu GBSS I und daher vermutlich auch zu GBSS II, aus Kartoffel hat. Aufgrund dieser hohen Homologien zu GBSS I und II kommt es beim Durchmustern von cDNA-Banken zu Kreuzhybridisierung mit GBSS I- und IIcDNAs. Die Identifizierung von cDNAs, die für SSS-Proteine codieren kann daher nur durch ein differentielles Screening erreicht werden. Dies setzt jedoch voraus, daß cDNA-Sequenzen für GBSS I- und II-Proteine aus Kartoffel zur Verfügung stehen. cDNA-Sequenzen, die für GBSS II aus Kartoffel codieren, waren jedoch bisher nicht zugänglich.

Im folgenden wird die Isolierung einer für eine lösliche Stärkesynthase aus Kartoffel codierenden cDNA beschrieben.
Hierzu wurde zunächst ein DNA-Fragment aus einer cDNA aus Reis, die eine lösliche Stärkesynthase codiert (Baba et al., 1993, Plant Physiol. 103:565-573), mit Hilfe der "Polymerase chain reaction" amplifiziert. Als Primer wurden dabei folgende Oligonucleotide verwendet:

Oligonucleotid 1: 5'-ACAGGATCCTGTGCTATGCGGCGTGTGAAG-3'
(Seq ID No. 14)

Oligonucleotid 2: 5'-TTGGGATCCGCAATGCCCACAGCATTTTTTTC-3'
(Seq ID No. 15)

Das aus der PCR-resultierende Fragment war 1067 bp lang. Dieses DNA-Fragment wurde später als heterologe Probe für die Identifizierung für lösliche Stärkesynthasen codierender cDNA-Sequenzen aus Kartoffel verwendet.

Für die Herstellung einer cDNA-Bibliothek wurde aus Kartoffelknollen der Kartoffelvarietät "Berolina" poly(A⁺)-mRNA isoliert. Ausgehend von der poly(A⁺)-mRNA wurde nach der Methode von Gubler und Hoffmann (1983, Gene 25:263-269) unter Verwendung eines *Xho I*-Oligo d(t)₁₈-Primers cDNA hergestellt. Diese wurde nach *EcoR I*-Linkeraddition mit *Xho I* nachgeschnitten und orientiert in einen mit *EcoR I* und *Xho I* geschnittenen Lambda ZAP II-Vektor (Stratagene) ligiert.
500 000 Plaques einer derart konstruierten cDNA-Bibliothek wurden mit Hilfe der heterologen Probe aus Reis auf DNA-Sequenzen hin untersucht, die homolog zu dieser sind. Da die verwendete Probe aus Reis eine starke Kreuzhybridisierung mit verschiedenen Sequenzen aus Kartoffel aufweist, war eine direkte Identifizierung von cDNA-Molekülen, die lösliche Stärkesynthasen codieren, nicht möglich. Aus Homologievergleichen war bekannt, daß die das SSS-Protein aus Reis codierende cDNA eine hohe Homologie zu der bereits aus Kartoffel isolierten GBSS I-cDNA aufweist. Da GBSS I und GBSS II in anderen Organismen starke Homologien aufweisen, war zu vermuten, daß die Probe aus Reis auch eine hohe Homologie zu GBSS II-Sequenzen aus Kartoffel aufweist. Um eine Identifizierung von cDNA-Sequenzen zu ermöglichen, die eine lösliche Stärkesynthase aus Kartoffel codieren, war es daher notwendig, über Sequenzen zu verfügen, die GBSS I und II aus Kartoffel codieren. DNA-Sequenzen, die GBSS I aus Kartoffel codieren waren bereits beschrieben, jedoch keine, die GBSS II aus Kartoffel codieren. Es wurde daher zunächst eine cDNA isoliert, die GBSS II aus Kartoffel codiert.

Hierzu wurden Stärkekorn-gebundene Proteine aus Kartoffelstärke isoliert. Die Isolierung erfolgte durch Elektroelution in einer Elutionsvorrichtung, die analog zu dem "Model 422 Electro-Eluter" (BIORAD Laboratories Inc., USA) konstruiert war, aber ein wesentlich größeres Volumen aufwies (ca. 200 ml). Es wurden 25 g getrocknete Stärke in Elutionspuffer aufgenommen (Endvolumen 80 ml). Die Suspension wurde im Wasserbad auf 70-80°C erwärmt. Anschließend wurden 72,07 g Harnstoff zugegeben (Endkonzentration 8 M) und das Volumen mit Elutionspuffer auf 180 ml aufgefüllt. Die Stärke löste sich unter ständigem Rühren und bekam eine kleisterartige Konsistenz. Die Proteine wurden aus der Lösung mit Hilfe des Elutionsvorrichtung über Nacht elektroeluiert (100 V; 50-60 mA). Die eluierten Proteine wurden vorsichtig aus der Apparatur entnommen. Schwebstoffe wurden durch kurze Zentrifugation entfernt. Der Überstand wurde 2-3 mal je eine Stunde bei 4°C gegen Dialysepuffer dialysiert. Anschließend wurde das Volumen der Proteinlösung bestimmt. Die Proteine wurden durch Zugabe von Ammoniumsulfat (90 % Endkonzentration) gefällt. Die Zugabe erfolgte unter ständigem Rühren bei 0°C. Die gefällten Proteine wurden durch Zentrifugation sedimentiert und in Proteinpuffer aufgenommen.

Die isolierten Proteine wurden zur Herstellung von polyclonalen Antikörpern aus Kaninchen verwendet, die spezifisch Stärkekorn-gebundene Proteine erkennen. Mit Hilfe derartiger Antikörper wurde anschließend nach Standardmethoden eine cDNA-Expressionsbibliothek nach Sequenzen durchgemustert, die Stärkekorn-gebundene Proteine codieren. Die Expressionsbibliothek wurde wie bereits oben beschrieben hergestellt.
Positive Phagenclone wurden unter Anwendung von Standardverfahren weiter gereinigt. Mit Hilfe der *in-vivo-excision*-Methode wurden von positiven Phagenclonen *E. coli*-Clone gewonnen, die ein doppelsträngiges pBluescript-Plasmid mit der jeweiligen cDNA-Insertion enthalten. Nach Überprüfung der Größe und des Restriktionsmusters der Insertionen wurden geeignete Clone, weiter analysiert. Ein Clon cGBSSII, wurde dabei als ein Clon identifiziert, der das GBSSII-Protein codiert.
Aus diesem Clon wurde das Plasmid pGBSSII (Fig. 5) isoliert und seine cDNA-Insertion durch Standardverfahren mittels der Didesoxymethode (Sanger et al., Proc. Natl. Acad. Sci. USA 74 (1977), 5463-5467) bestimmt. Die Insertion ist 1925 bp lang und stellt lediglich eine partielle cDNA-Sequenz dar. Die Nucleotidsequenz ist unter Seq ID No. 5 angegeben. Sequenzvergleiche zeigten, daß auch diese DNA-Sequenz in verschiedenen Bereichen starke Homologie zu der cDNA aus Reis aufwies, die lösliche Stärkesynthase codiert. Daher hybridisieren auch diese Sequenzen bei der Durchmusterung einer cDNA-Bibliothek mit der Probe aus Reis.
Die Insertion dieses Plasmids wurde später bei der Durchmusterung einer cDNA-Bibliothek aus Kartoffelknollen als Probe verwendet, um Sequenzen zu identifizieren, die GBSS II-Proteine codieren.
Neben dem Clon cGBSSII wurden bei der Durchmusterung der Expressionsbibliothek mit den polyclonalen Antikörpern, die gegen Stärkekorn-gebundene Proteine gerichtet sind, Clone isoliert, die cDNA-Insertionen aufwiesen, die für GBSS I aus Kartoffel codieren. Von einem dieser Clone, cGBSSI, wurde das Plasmid pGBSSI isoliert, und die Sequenz der cDNA-Insertion bestimmt. Diese stimmte weitgehend mit den bereits bekannten, GBSS I aus Kartoffel codierenden DNA-Sequenzen überein (Visser et al., Plant Sci. 64 (1989), 185-192; van der Leij et al., Mol. Gen. Genet. 228 (1990), 240-248). Diese cDNA-Insertion, enthalten in dem Plasmid pGBSS I, wurde daher später bei der Durchmusterung einer cDNA-Bibliothek aus Kartoffelknollen als Probe verwendet, um Sequenzen zu identifizieren, die GBSS I-Proteine codieren.

Die oben beschriebene cDNA-Bibliothek aus Kartoffel wurde zunächst nach Sequenzen durchgemustert, die GBSS I oder GBSS II aus Kartoffel codierten. Dazu wurden die Phagenplaques auf Nitrozellulose-Filter übertragen, die DNA durch NaOH-Behandlung denaturiert, die Filter neutralisiert und die DNA auf den Filtern durch Hitzebehandlung fixiert. Die Filter wurden in 0,25 M NaHPO₄, pH 7,2, 0,25 M NaCl, 7 % SDS, 1 mM EDTA, 25 % Formamid, 10 % PEG für 2 Stunden bei 42 °C vorhybridisiert. Anschließend wurden die Filter in 0,25 M NaHPO₄, pH 7,2, 0.25 M NaCl, 7 % SDS, 1 mM EDTA, 25 % Formamid, 10 % PEG nach Zugabe der entsprechenden radioaktiv markierten Probe über Nacht bei 42 °C hybridisiert. Als Probe wurde zum einen die cDNA-Insertion aus dem Plasmid pGBSSII verwendet, und zum anderen die cDNA-Insertion aus dem Plasmid pGBSSI.
Die Filter wurden anschließend 2 x 30 min in 0,1 x SSC, 0,5 % SDS bei 65 °C gewaschen und auf Röntgenfilmen exponiert.
Parallel wurden Filter derselben cDNA-Bibliothek mit der aus Reis stammenden radioaktiv markierten cDNA-Probe, die wie oben beschrieben hergestellt wurde, unter denselben Bedingungen hybridisiert wie für GBSS I und II beschrieben. Das Waschen der Filter erfolgte in diesem Fall für 2 x 30 min bei 40 °C mit 2 x SSC, 0,5 % SDS. Phagenclone, die nicht mit GBSS I oder GBSS II aus Kartoffel, aber mit der ReiscDNA hybridisierten, wurden unter Anwendung von Standardverfahren weiter gereinigt. Mit Hilfe der *in-vivo-excision*-Methode wurden von positiven Phagenclonen *E. coli*-Clone gewonnen, die ein doppelsträngiges pBluescript-Plasmid mit der jeweiligen cDNA-Insertion enthalten. Nach Überprüfung der Größe und des Restriktionsmusters der Insertionen wurden geeignete Clone einer Sequenzanalyse unterzogen.

### Beispiel 2

### Sequenzanalyse der cDNA-Insertion des Plasmids pSSSB

Aus einem entsprechend Beispiel 1 erhaltenen *E*. *coli*-Clon wurde das Plasmid pSSSB (Fig. 2) isoliert und seine cDNA-Insertion durch Standardverfahren mittels der Didesoxynucleotidmethode (Sanger et al., Proc. Natl. Acad. Sci. USA 74 (1977), 5463-5467) bestimmt. Die Insertion ist 1758 bp lang und stellt eine partielle cDNA dar. Die Nucleotidsequenz ist unter Seq ID No. 3 angegeben. Die korrespondierende Aminosäuresequenz ist unter Seq ID No. 4 dargestellt.

### Beispiel 3

### Isolierung der Vollängen-cDNA, die die Isoform GBSS II der Stärkekorn-gebundenen Stärkesynthase aus Solanum tuberosum codiert

Eine blattspezifische cDNA-Expressionsbank aus *Solanum tuberosum* L. cv. Désirée (Koßmann et al., Planta 186 (1992), 7-12) wurde nach Standardverfahren mittels Hybridisierung mit einem 5'-Fragment der cDNA-Insertion des Plasmids pGBSS II (1.9 kb) auf Vollänge-Clone hin durchgemustert. In Folge konnte das Plasmid pGBSS II-VK isoliert werden, das eine cDNA-Insertion mit einer Länge von ca. 2.8 kb enthält.

### Beispiel 4

### Sequenzanalyse der cDNA-Insertion des Plasmids pGBSS II-VK

Aus einem entsprechend Beispiel 3 erhaltenen *E. coli*-Clon wurde das Plasmid pGBSS II-VK isoliert und seine cDNA-Insertion durch Standardverfahren mittels der Didesoxynucleotidmethode (Sanger et al., Proc. Natl. Acad. Sci. USA 74 (1977), 5463-5467) bestimmt. Die Insertion ist ca. 2.8 kb lang. Die Nucleotidsequenz ist unter Seq ID No. 7 angegeben und umfaßt neben flankierenden Bereichen die gesamte das GBSSII-Protein aus Kartoffel codierende Region. Das aus der Aminosäuresequenz abgeleitete Molekulargewicht des Proteins beträgt ca. 85,1 kD.

### Beispiel 5

### Isolierung der Vollängen-cDNA, die die Isoform SSS B der löslichen Stärkesynthase aus Solanum tuberosum codiert

Eine blattspezifische cDNA-Expressionsbank aus *Solanum tuberosum* L. cv. Désirée (Koßmann et al., Plantä 186 (1992), 7-12) wurde nach Standardverfahren mittels Hybridisierung mit einem 5'-Fragment der cDNA-Insertion des Plasmids pSSS B (1.6 kb) auf Vollänge-Clone hin durchgemustert. In Folge konnte das Plasmid pSSS B-VK, isoliert werden, das eine cDNA-Insertion mit einer Länge von ca. 2.3 kb enthält.

### Beispiel 6

### Sequenzanalyse der cDNA-Insertion des Plasmids pSSS B-VK

Aus einem entsprechend Beispiel 5 erhaltenen *E. coli*-Clon wurde das Plasmid pSSS B-VK isoliert und seine cDNA-Insertion durch Standardverfahren mittels der Didesoxynucleotidmethode (Sanger et al., Proc. Natl. Acad. Sci. USA 74 (1977), 5463-5467) bestimmt. Die Insertion ist ca. 2.3 kb lang. Die Nucleotidsequenz ist unter Seq ID No. 9 angegeben und umfaßt neben flankierenden Sequenzen die gesamte codierende Region für die Isoform B der löslichen Stärkesynthase aus Kartoffel. Das aus der Aminosäuresequenz abgeleitete Molekulargewicht des Proteins beträgt ca. 78,6 kD.

### Beispiel 7

### Identifizierung, Isolierung und Charakterisierung einer cDNA, die die Isoform SSS A der löslichen Stärkesynthase aus Solanum tuberosum codiert

Aus einem Sequenzvergleich zwischen den bisher bekannten Sequenzen, die lösliche und Stärkekorn-gebundene Stärkesynthasen aus Pflanzen codieren (siehe Figur 7), war ersichtlich, daß es drei stark konservierte Bereiche zwischen den verschiedenen Proteinen gibt (Bereiche (I), (II) und (III) in Figur 7).

Um eine lösliche Stärkesynthase aus Kartoffel zu isolieren, wurden diese drei Bereiche ausgewählt, um polyclonale Peptidantikörper zu erzeugen. Dazu wurden drei synthetische Polypeptide mit den folgenden Aminosäuresequenzen hergestellt: Peptid 1:     NH2-PWSKTGGLGDVC-COOH     (Seq ID No. 16) Peptid 2: NH2-PSRFEPCGLNQLY-COOH (Seq ID No. 17) Peptid 3:    NH2-GTGGLRDTVENC-COOH    (Seq ID No. 13) Diese Peptide wurden an den KLH-Carrier ("keyhole limpet homocyanin") gekoppelt und anschließend zur Herstellung polyclonaler Antikörper in Kaninchen verwendet (Eurogentec, Seraing, Belgien).
Die resultierenden Antikörper wurden folgendermaßen bezeichnet: anti-SS1  polyclonaler Antikörper gegen das Peptid 1 anti-SS2  polyclonaler Antikörper gegen das Peptid 2 anti-SS3  polyclonaler Antikörper gegen das Peptid 3. Die Antikörper wurden mit angereinigten löslichen Stärkesynthasen aus Kartoffel auf ihre Spezifität hin untersucht.
Die Reinigung der löslichen Stärkesynthasen erfolgte dabei folgendermaßen:
2,5 kg Kartoffeln wurden in 2 Liter Puffer A aufgearbeitet. Nach Abtrennen der Stärke durch Zentrifugation bei 1000 g für 5 min wurde der Proteinextrakt an DEAE-FastFlow-Säulenmaterial (Pharmacia LKB) gebunden (äquilibriert mit Puffer B). Nach Waschen der Säule mit dem 5-fachen Säulenvolumen an Puffer B wurden gebundene Proteine mit 300 mM NaCl in Puffer B eluiert. Die eluierten Proteine wurden fraktionsweise aufgefangen, und Fraktionen mit einer hohen Stärkesynthase-Aktivität wurden vereinigt. Die vereinigten Fraktionen wurden durch Chromatographie über eine Gelfiltrationssäule (G25), die mit Puffer B äquilibriert wurde, entsalzt. Das Eluat wurde mit 1 Volumen 1 M Natrium-Citrat, 50 mM Tris-HCl pH 7,6, 2,5 mM DTT, 2 mM EDTA versetzt. Die Proteinlösung wurde auf eine mit Puffer C äquilibrierte Amylose-Resin-Säule (AR-Säule) aufgetragen. Die Säule wurde mit dem 20-fachen Säulenvolumen an Puffer C gewaschen. Gebundene Proteine wurden anschließend mit Puffer B eluiert.
Die Fraktionen, die eine hohe Stärkesynthase-Aktivität aufwiesen, wurden vereinigt und wiederum mit Hilfe von Gelfiltration über eine G25-Säule entsalzt.
Anschließend wurden die Fraktionen mit hoher Stärkesynthase-Aktivität auf eine mit Puffer B äquilibrierte MonoQ-Säule aufgetragen. Die Säule wurde mit dem 5-fachen Säulenvolumen an Puffer B gewaschen. Gebundene Proteine wurden mit Hilfe eines linearen NaCl-Gradienten von 0-300 mM eluiert und fraktionsweise gesammelt.

Die Analyse der Fraktionen hinsichtlich der Stärkesynthase-Aktivität und des Molekulargewichtes erfolgte mit Hilfe verschiedener Methoden:
a) Analyse der Fraktionen auf einem nativen Polyacrylamid-Gel
b) Analyse der Fraktionen auf einem denaturierenden SDS-Polyacrylamidgel und anschließende Silberfärbung
c) Bestimmung der Stärkesynthase-Aktivität durch Einbau radioaktiv-markierter ADP-Glucose (Amersham, UK) in neusynthetisierte Stärke.
d) Analyse der Fraktionen in einem Western Blot.

Für eine Western Blot-Analyse wurden 50 µg, 5 µg und 0,5 µg Protein eines Protein-Rohextraktes neben 15 µg Protein der Fraktionen, die von der DEAE-FastFlow-Säule eluiert wurden, 10 µg Protein der Fraktionen, die von der AR-Säule eluiert wurden und 3 µg Protein der Fraktionen, die von der MonoQ-Säule eluiert wurden, auf einem SDS-Polyacrylamid-Gel elektrophoretisch aufgetrennt. Die Proteine wurden mit Hilfe der Semidry-Elektroblot-Methode auf eine Nitrozellulosemembran übertragen.
Die Identifizierung von Proteinen, die von den Antikörpern anti-SS1, anti-SS2 oder anti-SS3 erkannt wurden, erfolgte mit Hilfe des "Blotting detection kit for rabbit anitbodies RPN 23" (Amersham UK) nach den Angaben des Herstellers.
Es wurden parallel drei Western Blot-Analysen durchgeführt mit den obenbeschriebenen polyxlonalen Antikörpern anti-SS1, anti-SS2 und anti-SS3. Dabei stellte sich heraus, daß der Antikörper anti-SS1 spezifisch GBSS I und GBSS II erkannte und der Antikörper anti-SS2 keine Spezifität aufwies. Lediglich der Antikörper anti-SS3 erkannte neben GBSS I und GBSS II im Western Blot spezifisch neue Proteine, insbesondere Proteine mit Molekulargewichten von 120-140 kd.

Der Antikörper anti-SS3 wurde anschließend verwendet, um eine cDNA-Bibliothek aus Kartoffelknollen nach Sequenzen durchzumustern, die lösliche Stärkesynthasen aus Kartoffel codieren. Hierfür wurde eine cDNA-Bibliothek, die wie in Beispiel 1 beschrieben hergestellt wurde, verwendet. Zur Analyse der Phagenplaques wurden diese auf Nitrozellulose-filter übertragen, die vorher für 30-60 min in einer 10 mM IPTG-Lösung inkubiert und anschließend auf Filterpapier getrocknet wurden. Der Transfer erfolgte für 3 h bei 37°C. Anschließend werden die Filter für 30 min bei Raumtemperatur in Blockreagenz inkubiert und zweimal für 5-10 min in TBST-Puffer gewaschen. Die Filter wurden mit dem polyclonalen Antikörper anti-SS3 in geeigneter Verdünnung für 1 h bei Raumtemperatur oder für 16 h bei 4°C geschüttelt. Die Identifizierung von Plaques, die ein Protein exprimierten, das von dem Antikörper anti-SS3 erkannt wurde, erfolgte mit Hilfe des "Blotting detection kit for rabbit antibodies RPN 23" (Amersham UK) nach den Angaben des Herstellers.
Phagenclone der cDNA-Bibliothek, die ein Protein exprimierten, das von dem Antikörper anti-SS3 erkannt wurde, wurden unter Anwendung von Standardverfahren weiter gereinigt. Mit Hilfe der *in vivo excision*-Methode (Stratagene) wurden von positiven Phagenclonen *E.coli*-clone gewonnen, die ein doppelsträngiges pBlueskript II SK-Plasmid mit der jeweiligen cDNA-Insertion zwischen der *EcoRI-* und der *Xho I*-Schnittstelle des Polylinkers enthalten. Nach Überprüfung der Größe und des Restriktionsmusters der Insertionen wurde ein geeigneter Clon einer Sequenzanalyse unterzogen.

### Beispiel 8

### Sequenzanalyse der cDNA-Insertion des Plasmids pSSSA

Aus einem entsprechend Beispiel 7 erhaltenen E. coli-Clon wurde das Plasmid pSSSA (Fig. 1) isoliert und seine cDNA-Insertion durch Standardverfahren mittels der Didesoxynucleotidmethode (Sanger et al., Proc. Natl. Acad. Sci. USA 74 (1977), 5463-5467) bestimmt. Die Insertion ist 2303 bp lang. Die Nucleotidsequenz ist unter Seq ID No. 1 angegeben. Die korrespondierende Aminosäuresequenz ist unter Seq ID No. 2 dargestellt.
Eine Sequenzanalyse und ein Sequenzvergleich mit bekannten DNA-Sequenzen zeigte, daß die unter Seq ID No. 1 dargestellte Sequenz neu ist und eine partielle codierende Region umfaßt, die ein Protein codiert, das Homologie zu Stärkesynthasen aus verschiedenen Organismen aufweist. Das durch diese cDNA-Insertion oder durch hybridisierende Sequenzen codierte Protein wird im Rahmen dieser Anmeldung als SSSA bezeichnet.
Diese DNA-Sequenz unterscheidet sich von der unter Seq ID No. 2 dargestellten DNA-Sequenz, die ebenfalls eine lösliche Stärkesynthase aus Kartoffel codiert, und ließ sich mit der unter Beispiel 1 beschriebenen Methode nicht aus einer cDNA-Bibliothek von Kartoffelknollen isolieren.

### Beispiel 9

### Isolierung der Vollängen-cDNA, die die Isoform SSS A der löslichen Stärkesynthase aus Solanum tuberosum codiert

Eine blattspezifische cDNA-Expressionsbank aus *Solanum tuberosum* L. cv. Désirée (Koßmann et al., Planta 186 (1992), 7-12) wurde nach Standardverfahren mittels Hybridisierung mit einem 5'-Fragment der cDNA-Insertion des Plasmids pSSSA (2.3 kb) auf Vollänge-Clone hin durchgemustert untersucht. In Folge konnte ein Clon isoliert werden, der eine im 5'-Bereich um ca. 1.86 kb längere cDNA-Insertion enthielt. Die cDNA-Insertion hat eine Gesamtlänge von ca. 4.16 kb isoliert werden.

### Beispiel 10

### Sequenzanalyse der cDNA-Insertion des Plasmids pSSSA-VK

Aus einem entsprechend Beispiel 9 erhaltenen *E. coli*-Clon wurde das Plasmid pSSSA-VK isoliert und seine cDNA-Insertion durch Standardverfahren mittels der Didesoxynucleotidmethode (Sanger et al., Proc. Natl. Acad. Sci. USA 74 (1977), 5463-5467) bestimmt. Die Insertion ist ca. 4.16 kb lang. Die Nucleotidsequenz ist unter Seq ID No. 11 angegeben. Die korrespondierende Aminosäuresequenz ist unter Seq ID No. 12 angegeben. Das aus der Aminosäuresequenz abgeleitete Molekulargewicht des SSSA-Proteins beträgt ca. 135 kD.

### Beispiel 11

### Konstruktion des Plasmids p35S-anti-SSSA und Einführung des Plasmids in das Genom von Kartoffelpflanzen

Aus dem Plasmid pSSSA wurde mit Hilfe der Restriktionsendonucleasen *Xba* I und *Asp* 718 ein ca. 2,1 kb großes DNA-Fragment isoliert, das die codierende Region für die Isoform A der löslichen Stärkesynthase aus Kartoffel umfaßt, und in den mit *Xba* I und *Asp* 718 geschnittenen Vektor pBinAR Hyg (DSM 9505) ligiert.
Durch die Insertion des cDNA-Fragmentes entsteht eine Expressionskassette, die folgendermaßen aus den Fragmenten A, B und C aufgebaut ist (Fig. 3):
Das Fragment A (529 bp) enthält den 35S-Promotor des Cauliflower-Mosaik-Virus (CaMV). Das Fragment umfaßt die Nucleotide 6909 bis 7437 des CaMV (Franck et al., Cell 21 (1980), 285-294).
Das Fragment B enthält neben flankierenden Bereichen die proteincodierende Region der Isoform A der löslichen Stärkesynthase aus *Solanum tuberosum.* Diese wurde wie oben beschrieben als *Xba I*/*Asp718*-Fragment aus pSSSA isoliert und in *antisense*-Orientierung an den 35S-Promotor in pBinAR Hyg fusioniert.
Fragment C (192 bp) enthält das Polyadenylierungssignal des Gens 3 der T-DNA des Ti-Plasmids pTiACH5 (Gielen et al., EMBO J. 3 (1984), 835-846).
Die Größe des Plasmids p35S-anti-SSSA beträgt ca. 13 kb.
Das Plasmid wurde mit Hilfe Agrobakterien-vermittelter Transformation in Kartoffelpflanzen transferiert wie oben beschrieben. Aus den transformierten Zellen wurden ganze Pflanzen regeneriert.
Als Ergebnis der Transformation zeigten transgene Kartoffelpflanzen eine Verringerung der Aktivität der Isoform A der löslichen Stärkesynthase (vergleiche Figur 8).
Die von diesen Pflanzen gebildete Stärke unterscheidet sich von in Wildtyp-Pflanzen synthetisierter Stärke in ihrem Phosphatgehalt, in der Viskosität wäßriger Lösungen, den Verkleisterungseigenschaften und der mittleren Stärkekorngröße. Die Ergebnisse sind in Tabelle I dargestellt.
Der Phosphatgehalt der in den transgenen Pflanzen gebildeten Stärke liegt um mindestens 30 %, vorzugsweise um 50 %, insbesondere um 70 % über den Werten der von in Wildtyp-Pflanzen synthetisierten Stärke.
Die Endviskosität der Stärke aus SSS A-"Antisense"-Pflanzen zeigt um mindestens 10 %, vorzugsweise um 20 %, insbesondere um 30 % niedrigere Werte im Vergleich zu Wildtyp-Pflanzen.
Die Verkleisterungstemperatur, die maximale Viskosität und die mittlere Stärkekorngröße der modifizierten Stärke liegen deutlich unter den Werten der in Wildtyp-Pflanzen gebildeten Stärke (siehe Tabelle I).

**Tabelle I**

| Charakteristika der Stärke aus Wildtyp- und SSS A-"Antisense"-Kartoffelpflanzen | | | |
|---|---|---|---|
| | Wildtyp | Linie 25 | Linie 39 |
| Phosphatgehalt [nmol mg⁻¹ Stärke⁻¹] | 8,50 ± 0,4 | 14,61 ± 0,3 | 14,54 ± 0,2 |
| Verkleisterungstemperatur [°C] | 69,5 | 67,4 | 66,2 |
| maximale Viskosität [cP] | 4044 | 3720 | 3756 |
| Endviskosität bei 50°C [cP] | 3312 | 2904 | 2400 |
| mittlere Stärkekorngröße [µm] | 29 | 24 | 27 |

### Beispiel 12

### Konstruktion des Plasmids p35S-anti-SSSB und Einführung des Plasmids in das Genom von Kartoffelpflanzen

Aus dem Plasmid pSSSB wurde mit Hilfe der Restriktionsendonucleasen *Xho I* und *Spe I* ein ca. 1,8 kb großes DNA-Fragment isoliert, das die codierende Region für die Isoform B der löslichen Stärkesynthase aus Kartoffel umfaßt, und in den mit *SmaI* geschnittenen Vektor pBinAR Hyg (DSM 9505) ligiert.

Durch die Insertion des cDNA-Fragmentes entsteht eine Expressionskassette, die folgendermaßen aus den Fragmenten A, B und C aufgebaut ist (Fig. 4) :
Das Fragment A (529 bp) enthält den 35S-Promotor des Cauliflower-Mosaik-Virus (CaMV). Das Fragment umfaßt die Nucleotide 6909 bis 7437 des CaMV (Franck et al., Cell 21 1980), 285-294).
Das Fragment B enthält neben flankierenden Bereichen einen Teil der proteincodierenden Region der Isoform B der löslichen Stärkesynthase aus *Solanum tuberosum.* Diese wurde wie oben beschrieben als *Xho I*/*Spe I*-Fragment aus pSSSB isoliert und in *antisense*-Orientierung an den 35S-Promotor in pBinAR Hyg fusioniert.
Fragment C (192 bp) enthält das Polyadenylierungssignal des Gens 3 der T-DNA des Ti-Plasmids pTiACH5 (Gielen et al., EMBO J. 3 (1984), 835-846).
Die Größe des Plasmids p35S-anti-SSSB beträgt ca. 13 kb.
Das Plasmid wurde mit Hilfe Agrobakterien-vermittelter Transformation in Kartoffelpflanzen transferiert wie oben beschrieben. Aus den transformierten Zellen wurden ganze Pflanzen regeneriert.
Als Ergebnis der Transformation zeigten transgene Kartoffelpflanzen eine Verringerung der Aktivität der Isoform B der löslichen Stärkesynthase (vergleiche Figur 8).

### Beispiel 13

### Konstruktion des Plasmids p35S-anti-GBSS II und Einführung des Plasmids in das Genom von Kartoffelpflanzen

Aus dem Plasmid pGBSS II wurde mit Hilfe der Restriktionsendonucleasen *Asp 718* und *Sma I* ein ca. 1,9 kb großes DNA-Fragment isoliert, das die codierende Region für die Isoform GBSS II der Stärkesynthase aus Kartoffel umfaßt. Die Fragmentenden wurden mit der T4 Polymerase geglättet und das Fragment in den mit *SmaI* geschnittenen Vektor pBinAR Hyg (DSM 9505) ligiert.
Durch die Insertion des cDNA-Fragmentes entsteht eine Expressionskassette, die folgendermaßen aus den Fragmenten A, B und C aufgebaut ist (Fig. 6):
Das Fragment A (529 bp) enthält den 35S-Promotor des Cauliflower-Mosaik-Virus (CaMV). Das Fragment umfaßt die Nucleotide 6909 bis 7437 des CaMV (Franck et al., Cell 21 (1980), 285-294).
Das Fragment B enthält neben flankierenden Bereichen einen Teil der proteincodierenden Region der Isoform GBSS II der Stärkesynthase aus *Solanum tuberosum.* Diese wurde wie oben beschrieben als *Asp 718*/*Sma I*-Fragment aus pGBSS II isoliert und nach Glättung der Fragmentenden in antisense-Orientierung an den 35S-Promotor in pBinAR Hyg fusioniert.
Fragment C (192 bp) enthält das Polyadenylierungssignal des Gens 3 der T-DNA des Ti-Plasmids pTiACH5 (Gielen et al., EMBO J. 3 (1984), 835-846).
Die Größe des Plasmids p35S-anti-GBSS II beträgt ca. 13 kb.
Das Plasmid wurde mit Hilfe Agrobakterien-vermittelter Transformation in Kartoffelpflanzen transferiert wie oben beschrieben. Aus den transformierten Zellen wurden ganze Pflanzen regeneriert.
Als Ergebnis der Transformation zeigten transgene Kartoffelpflanzen eine Verringerung der Aktivität der Isoform GBSS II der Stärkesynthase (vergleiche Figur 8).

Die von diesen Pflanzen gebildete Stärke unterscheidet sich von in Wildtyp-Pflanzen synthetisierter Stärke in ihrem Phosphatgehalt, in der Viskosität, den Verkleisterungseigenschaften und der mittleren Stärkekorngröße. Die Ergebnisse sind in Tabelle II dargestellt.

**Tabelle II**

| Charakteristika der Stärke aus Wildtyp- und GBSS II-"Antisense"-Pflanzen | | | | |
|---|---|---|---|---|
| | Wildtyp | Linie 14 | Linie 35 | Linie 44 |
| Phosphatgehalt [nmol mg⁻¹ Stärke⁻¹] | 6,99 ± 0,19 | 4,52 ± 0,2 | 4,13 ± 0,06 | 3,76 ± 0,12 |
| Verkleisterungstemperatur [°C] | 64,1 | 62,55 | 63,25 | 63,55 |
| maximale Viskosität [cP] | 4057 | 2831 | 2453 | 2587 |
| Endviskosität bei 50°C [cP] | 2849 | 2816 | 2597 | 2587 |
| mittlere Stärkekorngröße [µm] | 37 | 32 | 31 | 32 |

Der Phosphatgehalt der in den transgenen Pflanzen gebildeten Stärke liegt um mindestens 35 %, vorzugsweise um 40 %, insbesondere um 45 % unter den Werten der von in Wildtyp-Pflanzen synthetisierten Stärke.

Die maximale Viskosität der Stärke aus GBSS II-"antisense"-Pflanzen zeigt um mindestens 30 %, vorzugsweise um 35 %, insbesondere um 40 % niedrigere Werte im Vergleich zu Wildtyp-Pflanzen.

Die Verkleisterungstemperatur und die Endviskosität der modifizierten Stärke liegen unter den Werten der in Wildtyp-Pflanzen gebildeten Stärke. Die mittlere Stärkekorngröße der in den transgenen Pflanzen gebildeten Stärke ist deutlich geringer als die von Wildtyp-Stärke.

### Beispiel 14

### Überexpression der löslichen Stärkesynthasen SSS A und SSS B in E. coli

Für die Überexpression löslicher Stärkesynthasen in *E. coli* wurde der Stamm G6MD2 herangezogen. Hierbei handelt es sich um eine Mutante, die neben dem *glg-* auch im mal-Operon deletiert ist. Damit besitzt sie weder die Glycogen-Synthase (*glg*A), das Verzweigungsenzym (*glg*B) und die AGPase (*glg*C) noch die Amylomaltase (*mal*Q), die Maltodextrin-Phosphoylase (*mal*P) sowie weitere an der Metabolisierung von Maltose beteiligte Proteine. Aus diesem Grund ist die Mutante G6MD2 nicht fähig, über den ADP-Glucose-Weg Glycogen oder ausgehend von Maltose α-1,4-Glucane zu synthetisieren.
Zellen dieser Mutante wurden mit den cDNA-Clonen pSSSA-VK bzw. pSSSB-VK transformiert. Die Stärkesynthasen exprimierenden *E. coli*-Zellen wurden nach 2 h Induktion mit IPTG in 50 mM Tris-HCl pH 7,6, 10 % Glycerin, 2 mM EDTA, 2 mM DTT und 0,4 mM PMSF durch Ultraschall aufgeschlossen. Als Kontrolle dienten mit pBluescript transformierte Zellen. Die Abtrennung von intakten Zellen und Zellwandmaterial erfolgte durch eine Zentrifugation für 10 Minuten bei 13.000 g, und anschließend wurde die Proteinkonzentration des Überstandes bestimmt. 100 µg Proteinextrakt wurden dem Reaktionspuffer (Endkonzentration: 50 mM Tricine-NaOH pH 8,5, 25 mM Kaliumacetat, 2 mM EDTA und 2 mM DTT, 1 mM ADP-Gluose) zugegeben. Für die Untersuchung der Citrat-stimulierten Reaktion ("primer"-unabhängig) befand sich im Reaktionspuffer zusätzlich 0,5 M Natriumcitrat, während die "primer"-abhängige Reaktion in Anwesenheit von 0,02 % (Gew./Vol.) Maltooligosacchariden (Glucidex 19; 1-30 Glucose-Einheiten) getestet wurde. Die Reaktion wurde bei Raumtemperatur über Nacht durchgeführt. Die synthetisierten Glucane wurden dann mit Lugolscher Lösung nachgewiesen und zur weiteren Charakterisierung spektralphotometrisch untersucht.
Sowohl die Isoform SSS A als auch die Isoform SSS B synthetisierten in der "primer"-abhängigen Reaktion (Abwesenheit von Citrat) Glucane. Das Absorptionsmaximum des durch SSS B synthetisierten Glucans lag bei 614 nm, was einem Glucan von ca. 150 Glucose-Einheiten entspricht. Das von SSS A gebildete Glucan absorbierte bei 575 nm, was auf die Synthese von kurzkettigen Glucanen mit einem Polymerisationsgrad von ca. 50 Glucose-Einheiten hindeutet.
In der "primer"-unabhängigen, d.h. bei der Citrat-stimulierten, Reaktion lieferte allein die Isoform SSS B ein Glucan, welches nach Anfärbung mit Lugolscher Lösung bei 612 nm absorbierte. Die Isoform SSS A zeigte bei der "primer"-unabhängigen Reaktion keine Aktivität und synthetisierte folglich kein Glucan.
Die Proteinextrakte aus den mit pBluescript transformierten Zellen lieferten in keinem der Reaktionsansätze Produkte.

## Patentansprüche

1. DNA-Molekül codierend ein Protein mit der biologischen Aktivität einer Stärkesynthase der Isoform II (GBSSII) ausgewählt aus der Gruppe bestehend aus
(a) DNA-Molekülen, die ein Protein mit der unter SEQ ID NO: 8 dargestellten Aminosäuresequenz codieren;
(b) DNA-Molekülen, die die unter SEQ ID NO: 7 dargestellte Nucleotidsequenz umfassen;
(c) DNA-Molekülen, die mit den unter (a) oder (b) genannten DNA-Molekülen unter stringenten Bedingungen hybridisieren; und
(d) DNA-Molekülen, die eine Sequenzidentität von mindestens 80% zu einem DNA-Molekül nach (b) aufweisen.

2. DNA-Molekül nach Anspruch 1(d), das eine Sequenzidentität von über 90% zu einem DNA-Molekül nach Anspruch 1 (a) oder (b) aufweist.

3. Vektor enthaltend ein DNA-Molekül nach Anspruch 1 oder 2.

4. Vektor nach Anspruch 3, wobei das DNA-Molekül in sense-Orientierung mit DNA-Elementen verknüpft ist, die die Transkription und Synthese einer translatierbaren RNA in pro- oder eukaryontischen Zellen gewährleisten.

5. Wirtszellen enthaltend einen Vektor nach Anspruch 3 oder 4.

6. Protein oder biologisch aktives Fragment davon codiert durch ein DNA-Molekül nach Anspruch 1 oder 2.

7. Verfahren zur Herstellung eines Proteins nach Anspruch 6 oder eines biologisch aktiven Fragmentes davon, bei dem eine Wirtszelle nach Anspruch 5 unter Bedingungen kultiviert wird, die die Synthese des Proteins erlauben, und das Protein aus den kultivierten Zellen und/oder dem Kulturmedium isoliert wird.

8. Pflanzenzelle enthaltend ein DNA-Molekül nach Anspruch 1 oder 2 in Kombination mit einem heterologen Promotor.

9. Pflanze enthaltend Pflanzenzellen nach Anspruch 8.

10. Pflanze nach Anspruch 9, die eine Nutzpflanze ist.

11. Pflanze nach Anspruch 10, die eine stärkespeichernde Pflanze ist.

12. Pflanze nach Anspruch 11, die eine Kartoffelpflanze ist.

13. Vermehrungsmaterial einer Pflanze nach einem der Ansprüche 9 bis 12 enthaltend Pflanzenzellen nach Anspruch 8.

14. Verfahren zur Herstellung einer Stärke, wobei Stärke aus einer Pflanze nach einem der Ansprüche 9 bis 12 isoliert wird.

15. Stärke erhältlich aus einer Pflanze nach einem der Ansprüche 9 bis 12.

16. Transgene Pflanzenzelle, **dadurch gekennzeichnet, dass** in dieser Pflanzenzelle die Aktivität eines Proteins nach Anspruch 6 verringert ist.

17. Transgene Pflanzenzelle nach Anspruch 16, wobei in dieser Zelle eine anti-sense-RNA zu Transkripten eines DNA-Moleküls nach Anspruch 1 exprimiert wird.

18. Transgene Pflanzenzelle nach Anspruch 16, wobei in dieser Pflanzenzelle eine sense-RNA zur Erzielung eines Cosuppressions-Effekts exprimiert wird, wodurch die Verringerung der Aktivität des Proteins nach Anspruch 6 erreicht wird.

19. Pflanze enthaltend Pflanzenzellen nach einem der Ansprüche 16 bis 18.

20. Pflanze nach Anspruch 19, die eine Nutzpflanze ist.

21. Pflanze nach Anspruch 20, die eine stärkespeichernde Pflanze ist.

22. Pflanze nach Anspruch 21, die eine Kartoffelpflanze ist.

23. Vermehrungsmaterial einer Pflanze nach einem der Ansprüche 19 bis 22 enthaltend Pflanzenzellen nach einem der Ansprüche 16 bis 18.

24. Stärke erhältlich aus Pflanzen nach einem der Ansprüche 19 bis 22.


SEQUENZPROTOKOLL

(1) ALLGEMEINE ANGABEN:

(i) ANMELDER:
(A) NAME: Bayer CropScience GmbH
(B) STRASSE: Brüningstrasse 50
(C) ORT: Frankfurt/Main
(E) LAND: DE
(F) POSTLEITZAHL: 65929

(ii) BEZEICHNUNG DER ERFINDUNG: DNA-Molekuele codierend Enzyme, die an
der Staerkesynthese beteiligt sind, Vektoren, Bakterien,
transgene Pflanzenzellen und Pflanzen enthaltend diese
Molekuele

(iii) ANZAHL DER SEQUENZEN: 17

(iv) COMPUTER-LESBARE FASSUNG:
(A) DATENTRÄGER: Floppy disk
(B) COMPUTER: IBM PC compatible
(C) BETRIEBSSYSTEM: PC-DOS/MS-DOS
(D) SOFTWARE: PatentIn Release #1.0, Version #1.30 (EPA)

(2) ANGABEN ZU SEQ ID NO: 1:

(i) SEQUENZKENNZEICHEN:
(A) LÄNGE: 2303 Basenpaare
(B) ART: Nucleotid
(C) STRANGFORM: nicht bekannt
(D) TOPOLOGIE: linear

(ii) ART DES MOLEKÜLS: cDNA

(iii) HYPOTHETISCH: NEIN

(iv) ANTISENSE: NEIN

(vi) URSPRÜNLICHE HERKUNFT:
(A) ORGANISMUS: Solanum tuberosum
(B) STAMM: cv Berolina
(F) GEWEBETYP: Knollengewebe

(vii) UNMITTELBARE HERKUNFT:
(A) BIBLIOTHEK: cDNA-Bibliothek in pBluescriptSKII+

(ix) MERKMAL:
(A) NAME/SCHLÜSSEL: CDS
(B) LAGE:3..2033

(D) SONSTIGE ANGABEN:/function= "Polymerisierung von
Staerke"
/product= "Staerkesynthase"

(xi) SEQUENZBESCHREIBUNG: SEQ ID NO: 1:

GG CAC GAG GTC AAA AAG CTT GTT AAA TCT GAG AGA ATA GAT GGT GAT 47
His Glu Val Lys Lys Leu Val Lys Ser Glu Arg Ile Asp Gly Asp
1               5                   10                  15

TGG TGG TAT ACA GAG GTT GTT ATT CCT GAT CAG GCA CTT TTC TTG GAT 95
Trp Trp Tyr Thr Glu Val Val Ile Pro Asp Gln Ala Leu Phe Leu Asp
                20                  25                  30

TGG GTT TTT GCT GAT GGT CCA CCC AAG CAT GCC ATT GCT TAT GAT AAC 143
Trp Val Phe Ala Asp Gly Pro Pro Lys His Ala Ile Ala Tyr Asp Asn
            35                  40                  45

AAT CAC CGC CAA GAC TTC CAT GCC ATT GTC CCC AAC CAC ATT CCG GAG 191
Asn His Arg Gln Asp Phe His Ala Ile Val Pro Asn His Ile Pro Glu
        50                  55                  60

GAA TTA TAT TGG GTT GAG GAA GAA CAT CAG ATC TTT AAG ACA CTT CAG 239
Glu Leu Tyr Trp Val Glu Glu Glu His Gln Ile Phe Lys Thr Leu Gln
    65                  70                  75

GAG GAG AGA AGG CTT AGA GAA GCG GCT ATG CGT GCT AAG GTT GAA AAA 287
Glu Glu Arg Arg Leu Arg Glu Ala Ala Met Arg Ala Lys Val Glu Lys
80                  85                  90                  95

ACA GCA CTT CTG AAA ACT GAA ACA AAG GAA AGA ACT ATG AAA TCA TTT 335
Thr Ala Leu Leu Lys Thr Glu Thr Lys Glu Arg Thr Met Lys Ser Phe
                100                 105                 110

TTA CTG TCT CAG AAG CAT GTA GTA TAT ACT GAG CCT CTT GAT ATC CAA 383
Leu Leu Ser Gln Lys His Val Val Tyr Thr Glu Pro Leu Asp Ile Gln
            115                 120                 125

GCT GGA AGC AGC GTC ACA GTT TAC TAT AAT CCC GCC AAT ACA GTA CTT 431
Ala Gly Ser Ser Val Thr Val Tyr Tyr Asn Pro Ala Asn Thr Val Leu
        130                 135                 140

AAT GGT AAA CCT GAA ATT TGG TTC AGA TGT TCA TTT AAT CGC TGG ACT 479
Asn Gly Lys Pro Glu Ile Trp Phe Arg Cys Ser Phe Asn Arg Trp Thr
    145                 150                 155

CAC CGC CTG GGT CCA TTG CCA CCT CAG AAA ATG TCG CCT GCT GAA AAT 527
His Arg Leu Gly Pro Leu Pro Pro Gln Lys Met Ser Pro Ala Glu Asn
160                 165                 170                 175

GGC ACC CAT GTC AGA GCA ACT GTG AAG GTT CCA TTG GAT GCA TAT ATG 575
Gly Thr His Val Arg Ala Thr Val Lys Val Pro Leu Asp Ala Tyr Met
                180                 185                 190

ATG GAT TTT GTA TTT TCC GAG AGA GAA GAT GGT GGG ATT TTT GAC AAT 623
Met Asp Phe Val Phe Ser Glu Arg Glu Asp Gly Gly Ile Phe Asp Asn
            195                 200                 205

AAG AGC GGA ATG GAC TAT CAC ATA CCT GTG TTT GGA GGA GTC GCT AAA 671
Lys Ser Gly Met Asp Tyr His Ile Pro Val Phe Gly Gly Val Ala Lys
        210                 215                 220

GAA CCT CCA ATG CAT ATT GTC CAT ATT GCT GTC GAA ATG GCA CCA ATT 719
Glu Pro Pro Met His Ile Val His Ile Ala Val Glu Met Ala Pro Ile
    225                 230                 235

GCA AAG GTG GGA GGC CTT GGT GAT GTT GTT ACT AGT CTT TCC CGT GCT 767
Ala Lys Val Gly Gly Leu Gly Asp Val Val Thr Ser Leu Ser Arg Ala
240                 245                 250                 255

GTT CAA GAT TTA AAC CAT AAT GTG GAT ATT ATC TTA CCT AAG TAT GAC 815
Val Gln Asp Leu Asn His Asn Val Asp Ile Ile Leu Pro Lys Tyr Asp
                260                 265                 270

TGT TTG AAG ATG AAT AAT GTG AAG GAC TTT CGG TTT CAC AAA AAC TAC 863
Cys Leu Lys Met Asn Asn Val Lys Asp Phe Arg Phe His Lys Asn Tyr
            275                 280                 285

TTT TGG GGT GGG ACT GAA ATA AAA GTA TGG TTT GGA AAG GTG GAA GGT 911
Phe Trp Gly Gly Thr Glu Ile Lys Val Trp Phe Gly Lys Val Glu Gly
        290                 295                 300

CTC TCG GTC TAT TTT TTG GAG CCT CAA AAC GGG TTA TTT TCG AAA GGG 959
Leu Ser Val Tyr Phe Leu Glu Pro Gln Asn Gly Leu Phe Ser Lys Gly
    305                 310                 315

TGC GTC TAT GGT TGT AGC AAT GAT GGT GAA CGA TTT GGT TTC TTC TGT 1007
Cys Val Tyr Gly Cys Ser Asn Asp Gly Glu Arg Phe Gly Phe Phe Cys
320                 325                 330                 335

CAC GCG GCT TTG GAG TTT CTT CTG CAA GGT GGA TTT AGT CCG GAT ATC 1055
His Ala Ala Leu Glu Phe Leu Leu Gln Gly Gly Phe Ser Pro Asp Ile
                340                 345                 350

ATT CAT TGC CAT GAT TGG TCT AGT GCT CCT GTT GCT TGG CTC TTT AAG 1103
Ile His Cys His Asp Trp Ser Ser Ala Pro Val Ala Trp Leu Phe Lys
            355                 360                 365

GAA CAA TAT ACA CAC TAT GGT CTA AGC AAA TCT CGT ATA GTC TTC ACG 1151
Glu Gln Tyr Thr His Tyr Gly Leu Ser Lys Ser Arg Ile Val Phe Thr
        370                 375                 380

ATA CAT AAT CTT GAA TTT GGG GCA GAT CTC ATT GGG AGA GCA ATG ACT 1199
Ile His Asn Leu Glu Phe Gly Ala Asp Leu Ile Gly Arg Ala Met Thr
    385                 390                 395

AAC GCA GAC AAA GCT ACA ACA GTT TCA CCA ACT TAC TCA CAG GAG GTG 1247
Asn Ala Asp Lys Ala Thr Thr Val Ser Pro Thr Tyr Ser Gln Glu Val
400                 405                 410                 415

TCT GGA AAC CCT GTA ATT GCG CCT CAC CTT CAC AAG TTC CAT GGT ATA 1295
Ser Gly Asn Pro Val Ile Ala Pro His Leu His Lys Phe His Gly Ile
                420                 425                 430

GTG AAT GGG ATT GAC CCA GAT ATT TGG GAT CCT TTA AAC GAT AAG TTC 1343
Val Asn Gly Ile Asp Pro Asp Ile Trp Asp Pro Leu Asn Asp Lys Phe
            435                 440                 445

ATT CCG ATT CCG TAC ACC TCA GAA AAC GTT GTT GAA GGC AAA ACA GCA 1391
Ile Pro Ile Pro Tyr Thr Ser Glu Asn Val Val Glu Gly Lys Thr Ala
        450                 455                 460

GCC AAG GAA GCT TTG CAG CGA AAA CTT GGA CTG AAA CAG GCT GAC CTT 1439
Ala Lys Glu Ala Leu Gln Arg Lys Leu Gly Leu Lys Gln Ala Asp Leu
    465                 470                 475

CCT TTG GTA GGA ATT ATC ACC CGC TTA ACT CAC CAG AAA GGA ATC CAC 1487
Pro Leu Val Gly Ile Ile Thr Arg Leu Thr His Gln Lys Gly Ile His
480                 485                 490                 495

CTC ATT AAA CAT GCT ATT TGG CGC ACC TTG GAA CGG AAC GGA CAG GTA 1535
Leu Ile Lys His Ala Ile Trp Arg Thr Leu Glu Arg Asn Gly Gln Val
                500                 505                 510

GTC TTG CTT GGT TCT GCT CCT GAT CCT AGG GTA CAA AAC GAT TTT GTT 1583
Val Leu Leu Gly Ser Ala Pro Asp Pro Arg Val Gln Asn Asp Phe Val
            515                 520                 525

AAT TTG GCA AAT CAA TTG CAC TCC AAA TAT AAT GAC CGC GCA CGA CTC 1631
Asn Leu Ala Asn Gln Leu His Ser Lys Tyr Asn Asp Arg Ala Arg Leu
        530                 535                 540

TGT CTA ACA TAT GAC GAG CCA CTT TCT CAC CTG ATA TAT GCT GGT GCT 1679
Cys Leu Thr Tyr Asp Glu Pro Leu Ser His Leu Ile Tyr Ala Gly Ala
    545                 550                 555

GAT TTT ATT CTA GTT CCT TCA ATA TTT GAG CCA TGT GGA CTA ACA CAA 1727
Asp Phe Ile Leu Val Pro Ser Ile Phe Glu Pro Cys Gly Leu Thr Gln
560                 565                 570                 575

CTT ACC GCT ATG AGA TAT GGT TCA ATT CCA GTC GTG CGT AAA ACT GGA 1775
Leu Thr Ala Met Arg Tyr Gly Ser Ile Pro Val Val Arg Lys Thr Gly
                580                 585                 590

GGA CTT TAT GAT ACT GTA TTT GAT GTT GAC CAT GAC AAA GAG AGA GCA 1823
Gly Leu Tyr Asp Thr Val Phe Asp Val Asp His Asp Lys Glu Arg Ala
            595                 600                 605

CAA CAG TGT GGT CTT GAA CCA AAT GGA TTC AGC TTT GAT GGA GCA GAT 1871
Gln Gln Cys Gly Leu Glu Pro Asn Gly Phe Ser Phe Asp Gly Ala Asp
        610                 615                 620

GCT GGC GGA GTT GAT TAT GCT CTG AAT AGA GCT CTC TCT GCT TGG TAC 1919
Ala Gly Gly Val Asp Tyr Ala Leu Asn Arg Ala Leu Ser Ala Trp Tyr
    625                 630                 635

GAT GGT CGG GAT TGG TTC AAC TCT TTA TGC AAG CAG GTC ATG GAA CAA 1967
Asp Gly Arg Asp Trp Phe Asn Ser Leu Cys Lys Gln Val Met Glu Gln
640                 645                 650                 655

GAT TGG TCT TGG AAC CGA CCT GCT CTT GAT TAT TTG GAG CTT TAC CAT 2015
Asp Trp Ser Trp Asn Arg Pro Ala Leu Asp Tyr Leu Glu Leu Tyr His
                660                 665                 670

GCT GCT AGA AAG TTA GAA TAGTTAGTTT GTGAGATGCT AGCAGAAAAA 2063
Ala Ala Arg Lys Leu Glu
            675

TTCACGAGAT CTGCAATCTG TACAGGTTCA GTGTTTGCGT CTGGACAGCT TTTTATTTCC 2123

TATATCAAAG TATAAATCAA GTCTACACTG AGATCAATAG CAGACAGTCC TCAGTTCATT 2183

TCATTTTTTG TGCAACATAT GAAAGAGCTT AGCCTCTAAT AATGTAGTCA TTGATGATTA 2243

TTTGTTTTGG GAAGAAATGA GAAATCAAAG GATGCAAAAT ACTCTGAAAA AAAAAAAAAA 2303

(2) ANGABEN ZU SEQ ID NO: 2:

(i) SEQUENZKENNZEICHEN:
(A) LÄNGE: 677 Aminosäuren
(B) ART: Aminosäure
(D) TOPOLOGIE: linear

(ii) ART DES MOLEKÜLS: Protein
(xi) SEQUENZBESCHREIBUNG: SEQ ID NO: 2:

His Glu Val Lys Lys Leu Val Lys Ser Glu Arg Ile Asp Gly Asp Trp
1               5                   10                  15

Trp Tyr Thr Glu Val Val Ile Pro Asp Gln Ala Leu Phe Leu Asp Trp
            20                  25                  30

Val Phe Ala Asp Gly Pro Pro Lys His Ala Ile Ala Tyr Asp Asn Asn
        35                  40                  45

His Arg Gln Asp Phe His Ala Ile Val Pro Asn His Ile Pro Glu Glu
    50                  55                  60

Leu Tyr Trp Val Glu Glu Glu His Gln Ile Phe Lys Thr Leu Gln Glu
65                  70                  75                  80

Glu Arg Arg Leu Arg Glu Ala Ala Met Arg Ala Lys Val Glu Lys Thr
                85                  90                  95

Ala Leu Leu Lys Thr Glu Thr Lys Glu Arg Thr Met Lys Ser Phe Leu
            100                 105                 110

Leu Ser Gln Lys His Val Val Tyr Thr Glu Pro Leu Asp Ile Gln Ala
        115                 120                 125

Gly Ser Ser Val Thr Val Tyr Tyr Asn Pro Ala Asn Thr Val Leu Asn
    130                 135                 140

Gly Lys Pro Glu Ile Trp Phe Arg Cys Ser Phe Asn Arg Trp Thr His
145                 150                 155                 160

Arg Leu Gly Pro Leu Pro Pro Gln Lys Met Ser Pro Ala Glu Asn Gly
                165                 170                 175

Thr His Val Arg Ala Thr Val Lys Val Pro Leu Asp Ala Tyr Met Met
            180                 185                 190

Asp Phe Val Phe Ser Glu Arg Glu Asp Gly Gly Ile Phe Asp Asn Lys
        195                 200                 205

Ser Gly Met Asp Tyr His Ile Pro Val Phe Gly Gly Val Ala Lys Glu
    210                 215                 220

Pro Pro Met His Ile Val His Ile Ala Val Glu Met Ala Pro Ile Ala
225                 230                 235                 240

Lys Val Gly Gly Leu Gly Asp Val Val Thr Ser Leu Ser Arg Ala Val
                245                 250                 255

Gln Asp Leu Asn His Asn Val Asp Ile Ile Leu Pro Lys Tyr Asp Cys
            260                 265                 270

Leu Lys Met Asn Asn Val Lys Asp Phe Arg Phe His Lys Asn Tyr Phe
        275                 280                 285

Trp Gly Gly Thr Glu Ile Lys Val Trp Phe Gly Lys Val Glu Gly Leu
    290                 295                 300

Ser Val Tyr Phe Leu Glu Pro Gln Asn Gly Leu Phe Ser Lys Gly Cys
305                 310                 315                 320

Val Tyr Gly Cys Ser Asn Asp Gly Glu Arg Phe Gly Phe Phe Cys His
                325                 330                 335

Ala Ala Leu Glu Phe Leu Leu Gln Gly Gly Phe Ser Pro Asp Ile Ile
            340                 345                 350

His Cys His Asp Trp Ser Ser Ala Pro Val Ala Trp Leu Phe Lys Glu
        355                 360                 365

Gln Tyr Thr His Tyr Gly Leu Ser Lys Ser Arg Ile Val Phe Thr Ile
    370                 375                 380

His Asn Leu Glu Phe Gly Ala Asp Leu Ile Gly Arg Ala Met Thr Asn
385                 390                 395                 400

Ala Asp Lys Ala Thr Thr Val Ser Pro Thr Tyr Ser Gln Glu Val Ser
                405                 410                 415

Gly Asn Pro Val Ile Ala Pro His Leu His Lys Phe His Gly Ile Val
            420                 425                 430

Asn Gly Ile Asp Pro Asp Ile Trp Asp Pro Leu Asn Asp Lys Phe Ile
        435                 440                 445

Pro Ile Pro Tyr Thr Ser Glu Asn Val Val Glu Gly Lys Thr Ala Ala
    450                 455                 460

Lys Glu Ala Leu Gln Arg Lys Leu Gly Leu Lys Gln Ala Asp Leu Pro
465                 470                 475                 480

Leu Val Gly Ile Ile Thr Arg Leu Thr His Gln Lys Gly Ile His Leu
                485                 490                 495

Ile Lys His Ala Ile Trp Arg Thr Leu Glu Arg Asn Gly Gln Val Val
            500                 505                 510

Leu Leu Gly Ser Ala Pro Asp Pro Arg Val Gln Asn Asp Phe Val Asn
        515                 520                 525

Leu Ala Asn Gln Leu His Ser Lys Tyr Asn Asp Arg Ala Arg Leu Cys
    530                 535                 540

Leu Thr Tyr Asp Glu Pro Leu Ser His Leu Ile Tyr Ala Gly Ala Asp
545                 550                 555                 560

Phe Ile Leu Val Pro Ser Ile Phe Glu Pro Cys Gly Leu Thr Gln Leu
                565                 570                 575

Thr Ala Met Arg Tyr Gly Ser Ile Pro Val Val Arg Lys Thr Gly Gly
            580                 585                 590

Leu Tyr Asp Thr Val Phe Asp Val Asp His Asp Lys Glu Arg Ala Gln
        595                 600                 605

Gln Cys Gly Leu Glu Pro Asn Gly Phe Ser Phe Asp Gly Ala Asp Ala
    610                 615                 620

Gly Gly Val Asp Tyr Ala Leu Asn Arg Ala Leu Ser Ala Trp Tyr Asp
625                 630                 635                 640

Gly Arg Asp Trp Phe Asn Ser Leu Cys Lys Gln Val Met Glu Gln Asp
                645                 650                 655

Trp Ser Trp Asn Arg Pro Ala Leu Asp Tyr Leu Glu Leu Tyr His Ala
            660                 665                 670

Ala Arg Lys Leu Glu
        675

(2) ANGABEN ZU SEQ ID NO: 3:

(i) SEQUENZKENNZEICHEN:
(A) LÄNGE: 1758 Basenpaare
(B) ART: Nucleotid
(C) STRANGFORM: nicht bekannt
(D) TOPOLOGIE: linear

(ii) ART DES MOLEKÜLS: cDNA zu mRNA

(iii) HYPOTHETISCH: NEIN

(iv) ANTISENSE: NEIN

(vi) URSPRÜNLICHE HERKUNFT:
(A) ORGANISMUS: Solanum tuberosum
(B) STAMM: cv. Berolina
(F) GEWEBETYP: Knollengewebe

(vii) UNMITTELBARE HERKUNFT:
(A) BIBLIOTHEK: cDNA-Bibliothek in pBluescriptSKII+

(ix) MERKMAL:
(A) NAME/SCHLÜSSEL: CDS
(B) LAGE:1..1377
(D) SONSTIGE ANGABEN:/function= "Polymerisierung von
Staerke"
/product= "Staerkesynthase"

(xi) SEQUENZBESCHREIBUNG: SEQ ID NO: 3:

GGC ACG AGC AAT GCT GTT GAC CTT GAT GTG CGG GCC ACT GTC CAT TGC 48
Gly Thr Ser Asn Ala Val Asp Leu Asp Val Arg Ala Thr Val His Cys
1               5                   10                  15

TTT GGT GAT GCA CAG GAA GTA GCC TTC TAC CAT GAA TAC AGG GCA GGT 96
Phe Gly Asp Ala Gln Glu Val Ala Phe Tyr His Glu Tyr Arg Ala Gly
            20                  25                  30

GTT GAT TGG GTA TTT GTG GAC CAC TCT TCT TAC CGC AGA CCT GGA ACG 144
Val Asp Trp Val Phe Val Asp His Ser Ser Tyr Arg Arg Pro Gly Thr
        35                  40                  45

CCA TAT GGT GAT ATT TAT GGT GCA TTT GGT GAT AAT CAG TTT CGC TTC 192
Pro Tyr Gly Asp Ile Tyr Gly Ala Phe Gly Asp Asn Gln Phe Arg Phe
    50                  55                  60

ACT TTG CTT TCT CAC GCA GCA TGT GAA GCG CCA TTG GTT CTT CCA CTG 240
Thr Leu Leu Ser His Ala Ala Cys Glu Ala Pro Leu Val Leu Pro Leu
65                  70                  75                  80

GGA GGG TTC ACT TAT GGA GAG AAG TGC TTG TTT CTC GCT AAT GAT TGC 288
Gly Gly Phe Thr Tyr Gly Glu Lys Cys Leu Phe Leu Ala Asn Asp Cys
                85                  90                  95

AAC GCT GCC TTG GTT CCT TTA CTT TTA GCG GCC AAG TAT CGT CCT TAT 336
Asn Ala Ala Leu Val Pro Leu Leu Leu Ala Ala Lys Tyr Arg Pro Tyr
            100                 105                 110

GGT GTT TAC AAG GAT GCT CGT AGT ATT GTC GCA ATA CAC AAC ATT GCA 384
Gly Val Tyr Lys Asp Ala Arg Ser Ile Val Ala Ile His Asn Ile Ala
        115                 120                 125

CAT CAG GGA GTG GAG CCT GCA GTA ACC TAC AAT AAT TTG GGT TTG CCT 432
His Gln Gly Val Glu Pro Ala Val Thr Tyr Asn Asn Leu Gly Leu Pro
    130                 135                 140

CCA CAA TGG TAT GGA GCA GTT GAA TGG ATA TTT CCC ACA TGG GCA AGG 480
Pro Gln Trp Tyr Gly Ala Val Glu Trp Ile Phe Pro Thr Trp Ala Arg
145                 150                 155                 160

GCG CAT GCG CTT GAC ACT GGT GAA ACA GTG AAC GTT TTG AAA GGG GCA 528
Ala His Ala Leu Asp Thr Gly Glu Thr Val Asn Val Leu Lys Gly Ala
                165                 170                 175

ATA GCA GTT GCT GAT CGG ATA CTG ACA GTT AGC CAG GGA TAC TCA TGG 576
Ile Ala Val Ala Asp Arg Ile Leu Thr Val Ser Gln Gly Tyr Ser Trp
            180                 185                 190

GAA ATA ACA ACT CCT GAA GGG GGA TAT GGG CTA CAT GAG CTG TTG AGC 624
Glu Ile Thr Thr Pro Glu Gly Gly Tyr Gly Leu His Glu Leu Leu Ser
        195                 200                 205

AGT AGA CAG TCT GTT CTT AAT GGA ATT ACT AAT GGA ATA GAT GTT AAT 672
Ser Arg Gln Ser Val Leu Asn Gly Ile Thr Asn Gly Ile Asp Val Asn
    210                 215                 220

GAT TGG AAC CCG TCG ACA GAT GAG CAT ATC GCT TCG CAT TAC TCC ATC 720
Asp Trp Asn Pro Ser Thr Asp Glu His Ile Ala Ser His Tyr Ser Ile
225                 230                 235                 240

AAT GAC CTC TCC CCC CCT GGA AAG GTT CAG TGC AAG ACT GAT CTG CAA 768
Asn Asp Leu Ser Pro Pro Gly Lys Val Gln Cys Lys Thr Asp Leu Gln
                245                 250                 255

AAG GAA CTG GGC CTT CCA ATT CGA CCC GAT TGT CCA CTG ATT GGA TTT 816
Lys Glu Leu Gly Leu Pro Ile Arg Pro Asp Cys Pro Leu Ile Gly Phe
            260                 265                 270

ATT GGA AGG CTG GAC TAC CAG AAA GGT GTT GAC ATA ATC CTG TCA GCA 864
Ile Gly Arg Leu Asp Tyr Gln Lys Gly Val Asp Ile Ile Leu Ser Ala
        275                 280                 285

ATT CCA GAA CTT ATG CAG AAT GAT GTC CAA GTT GTA ATG CTT GGA TCT 912
Ile Pro Glu Leu Met Gln Asn Asp Val Gln Val Val Met Leu Gly Ser
    290                 295                 300

GGT GAG AAA CAA TAT GAA GAC TGG ATG AGA CAT ACA GAA AAT CTT TTT 960
Gly Glu Lys Gln Tyr Glu Asp Trp Met Arg His Thr Glu Asn Leu Phe
305                 310                 315                 320

AAA GAC AAA TTT CGT GCT TGG GTT GGA TTT AAT GTT CCA GTT TCT CAT 1008
Lys Asp Lys Phe Arg Ala Trp Val Gly Phe Asn Val Pro Val Ser His
                325                 330                 335

AGG ATA ACA GCA GGA TGC GAC ATA CTA TTG ATG CCC TCA AGA TTC GAA 1056
Arg Ile Thr Ala Gly Cys Asp Ile Leu Leu Met Pro Ser Arg Phe Glu
            340                 345                 350

CCG TGT GGC TTA AAC CAA TTG TAT GCA ATG AGA TAT GGC ACC ATA CCT 1104
Pro Cys Gly Leu Asn Gln Leu Tyr Ala Met Arg Tyr Gly Thr Ile Pro
        355                 360                 365

ATT GTT CAT AGC ACG GGG GGC CTA AGA GAC ACA GTG AAG GAT TTT AAT 1152
Ile Val His Ser Thr Gly Gly Leu Arg Asp Thr Val Lys Asp Phe Asn
    370                 375                 380

CCA TAT GCT CAA GAA GGA AAA GGT GAA GGT ACC GGG TGG ACA TTT TCT 1200
Pro Tyr Ala Gln Glu Gly Lys Gly Glu Gly Thr Gly Trp Thr Phe Ser
385                 390                 395                 400

CCT CTA ACG AGT GAA AAG TTG TTT GAT ACA CTG AAG CTG GCG ATC AGG 1248
Pro Leu Thr Ser Glu Lys Leu Phe Asp Thr Leu Lys Leu Ala Ile Arg
                405                 410                 415

ACT TAT ACA GAA CAT AAG TCA TCT TGG GAG GGA TTG ATG AAG AGA GGT 1296
Thr Tyr Thr Glu His Lys Ser Ser Trp Glu Gly Leu Met Lys Arg Gly
            420                 425                 430

ATG GGA AGG GAC TAT TCC TGG GAA AAT GCA GCC ATT CAA TAT GAG CAA 1344
Met Gly Arg Asp Tyr Ser Trp Glu Asn Ala Ala Ile Gln Tyr Glu Gln
        435                 440                 445

GTT TTC ACC TGG GCC TTT ATA GAT CCT CCA TAT GTCAGATGAT TTATCAAGAA 1397
Val Phe Thr Trp Ala Phe Ile Asp Pro Pro Tyr
    450                 455

AGATTGCAAA CGGGATACAT CATTAAACTA TACGCAGAGC TTTTGGTGCT ATTAGCTACT 1457

GTCATTGGGC GCGGAATGTT TGTGGTTCTT TCTGATTCAG AGAGATCAAG TTAGTTCCAA 1517

AGACATGTAG CCTGCCCCTG TCTGTGATGA AGTAAAACTA CAAAGGCAAT TAGAAACCCA 1577

CCAACAACTG CCTCCTTTGG GAGAAGAGTG GAAATATGTA AAAAAGAATT TTGAGTTTAA 1637

TGTCAATTGA ATTAATTATT CTCATTTTTA AAAAAAACAT CTCATCTCAT ACAATATATA 1697

AAATTGATCA TGATTGATGC CCCCTAAAAA AAAAAAAAAA AAAAAAAAAA AAAAAAAAAA 1757

A 1758

(2) ANGABEN ZU SEQ ID NO: 4:

(i) SEQUENZKENNZEICHEN:
(A) LÄNGE: 459 Aminosäuren
(B) ART: Aminosäure
(D) TOPOLOGIE: linear

(ii) ART DES MOLEKÜLS: Protein
(xi) SEQUENZBESCHREIBUNG: SEQ ID NO: 4:

Gly Thr Ser Asn Ala Val Asp Leu Asp Val Arg Ala Thr Val His Cys
1               5                   10                  15

Phe Gly Asp Ala Gln Glu Val Ala Phe Tyr His Glu Tyr Arg Ala Gly
            20                  25                  30

Val Asp Trp Val Phe Val Asp His Ser Ser Tyr Arg Arg Pro Gly Thr
        35                  40                  45

Pro Tyr Gly Asp Ile Tyr Gly Ala Phe Gly Asp Asn Gln Phe Arg Phe
    50                  55                  60

Thr Leu Leu Ser His Ala Ala Cys Glu Ala Pro Leu Val Leu Pro Leu
65                  70                  75                  80

Gly Gly Phe Thr Tyr Gly Glu Lys Cys Leu Phe Leu Ala Asn Asp Cys
                85                  90                  95

Asn Ala Ala Leu Val Pro Leu Leu Leu Ala Ala Lys Tyr Arg Pro Tyr
            100                 105                 110

Gly Val Tyr Lys Asp Ala Arg Ser Ile Val Ala Ile His Asn Ile Ala
        115                 120                 125

His Gln Gly Val Glu Pro Ala Val Thr Tyr Asn Asn Leu Gly Leu Pro
    130                 135                 140

Pro Gln Trp Tyr Gly Ala Val Glu Trp Ile Phe Pro Thr Trp Ala Arg
145                 150                 155                 160

Ala His Ala Leu Asp Thr Gly Glu Thr Val Asn Val Leu Lys Gly Ala
                165                 170                 175

Ile Ala Val Ala Asp Arg Ile Leu Thr Val Ser Gln Gly Tyr Ser Trp
            180                 185                 190

Glu Ile Thr Thr Pro Glu Gly Gly Tyr Gly Leu His Glu Leu Leu Ser
        195                 200                 205

Ser Arg Gln Ser Val Leu Asn Gly Ile Thr Asn Gly Ile Asp Val Asn
    210                 215                 220

Asp Trp Asn Pro Ser Thr Asp Glu His Ile Ala Ser His Tyr Ser Ile
225                 230                 235                 240

Asn Asp Leu Ser Pro Pro Gly Lys Val Gln Cys Lys Thr Asp Leu Gln
                245                 250                 255

Lys Glu Leu Gly Leu Pro Ile Arg Pro Asp Cys Pro Leu Ile Gly Phe
            260                 265                 270

Ile Gly Arg Leu Asp Tyr Gln Lys Gly Val Asp Ile Ile Leu Ser Ala
        275                 280                 285

Ile Pro Glu Leu Met Gln Asn Asp Val Gln Val Val Met Leu Gly Ser
    290                 295                 300

Gly Glu Lys Gln Tyr Glu Asp Trp Met Arg His Thr Glu Asn Leu Phe
305                 310                 315                 320

Lys Asp Lys Phe Arg Ala Trp Val Gly Phe Asn Val Pro Val Ser His
                325                 330                 335

Arg Ile Thr Ala Gly Cys Asp Ile Leu Leu Met Pro Ser Arg Phe Glu
            340                 345                 350

Pro Cys Gly Leu Asn Gln Leu Tyr Ala Met Arg Tyr Gly Thr Ile Pro
        355                 360                 365

Ile Val His Ser Thr Gly Gly Leu Arg Asp Thr Val Lys Asp Phe Asn
    370                 375                 380

Pro Tyr Ala Gln Glu Gly Lys Gly Glu Gly Thr Gly Trp Thr Phe Ser
385                 390                 395                 400

Pro Leu Thr Ser Glu Lys Leu Phe Asp Thr Leu Lys Leu Ala Ile Arg
                405                 410                 415

Thr Tyr Thr Glu His Lys Ser Ser Trp Glu Gly Leu Met Lys Arg Gly
            420                 425                 430

Met Gly Arg Asp Tyr Ser Trp Glu Asn Ala Ala Ile Gln Tyr Glu Gln
        435                 440                 445

Val Phe Thr Trp Ala Phe Ile Asp Pro Pro Tyr
    450                 455

(2) ANGABEN ZU SEQ ID NO: 5:

(i) SEQUENZKENNZEICHEN:
(A) LÄNGE: 1926 Basenpaare
(B) ART: Nucleotid
(C) STRANGFORM: nicht bekannt
(D) TOPOLOGIE: linear

(ii) ART DES MOLEKÜLS: cDNA zu mRNA

(iii) HYPOTHETISCH: NEIN

(iv) ANTISENSE: NEIN

(vi) URSPRÜNLICHE HERKUNFT:
(A) ORGANISMUS: Solanum tuberosum
(B) STAMM: cv. Berolina
(F) GEWEBETYP: Knollengewebe

(vii) UNMITTELBARE HERKUNFT:
(A) BIBLIOTHEK: cDNA-Bibliothek in pBluescriptSK+

(ix) MERKMAL:
(A) NAME/SCHLÜSSEL: CDS
(B) LAGE:2..1675
(D) SONSTIGE ANGABEN:/function= "Polymerisierung von
Staerke"
/product= "Staerkesynthase"

(xi) SEQUENZBESCHREIBUNG: SEQ ID NO: 5:

C GGC ACG AGC AAA AGT TTA GTA GAT GTT CCT GGA AAG AAG ATC CAG 46
Gly Thr Ser Lys Ser Leu Val Asp Val Pro Gly Lys Lys Ile Gln
1               5                   10                  15

TCT TAT ATG CCT TCA TTA CGT AAA GAA TCC TCA GCA TCC CAT GTG GAA 94
Ser Tyr Met Pro Ser Leu Arg Lys Glu Ser Ser Ala Ser His Val Glu
                20                  25                  30

CAG AGG AAT GAA AAT CTT GAA GGA TCA AGT GCT GAG GCA AAC GAA GAG 142
Gln Arg Asn Glu Asn Leu Glu Gly Ser Ser Ala Glu Ala Asn Glu Glu
            35                  40                  45

ACT GAA GAT CCT GTG AAT ATA GAT GAG AAA CCC CCT CCA TTG GCA GGA 190
Thr Glu Asp Pro Val Asn Ile Asp Glu Lys Pro Pro Pro Leu Ala Gly
        50                  55                  60

ACA AAT GTT ATG AAC ATT ATT TTG GTG GCT TCA GAA TGC GCT CCA TGG 238
Thr Asn Val Met Asn Ile Ile Leu Val Ala Ser Glu Cys Ala Pro Trp
    65                  70                  75

TCT AAA ACA GGT GGG CTT GGA GAT GTT GCT GGA GCA TTA CCC AAA GCT 286
Ser Lys Thr Gly Gly Leu Gly Asp Val Ala Gly Ala Leu Pro Lys Ala
80                  85                  90                  95

TTG GCT CGA CGT GGC CAC AGA GTT ATG GTT GTG GCA CCT CGT TAT GAC 334
Leu Ala Arg Arg Gly His Arg Val Met Val Val Ala Pro Arg Tyr Asp
                100                 105                 110

AAC TAT CCT GAA CCT CAA GAT TCT GGT GTA AGA AAA ATT TAT AAA GTT 382
Asn Tyr Pro Glu Pro Gln Asp Ser Gly Val Arg Lys Ile Tyr Lys Val
            115                 120                 125

GAT GGT CAG GAT GTG GAA GTG ACT TAC TTC CAA GCT TTT ATT GAT GGT 430
Asp Gly Gln Asp Val Glu Val Thr Tyr Phe Gln Ala Phe Ile Asp Gly
        130                 135                 140

GTG GAT TTT GTT TTC ATT GAC AGT CAT ATG TTT AGA CAC ATT GGG AAC 478
Val Asp Phe Val Phe Ile Asp Ser His Met Phe Arg His Ile Gly Asn
    145                 150                 155

AAC ATT TAC GGA GGG AAC CGT GTG GAT ATT TTA AAA CGC ATG GTT TTA 526
Asn Ile Tyr Gly Gly Asn Arg Val Asp Ile Leu Lys Arg Met Val Leu
160                 165                 170                 175

TTT TGC AAA GCA GCG ATT GAG GTT CCT TGG CAT GTT CCA TGT GGT GGG 574
Phe Cys Lys Ala Ala Ile Glu Val Pro Trp His Val Pro Cys Gly Gly
                180                 185                 190

GTC TGC TAT GGA GAT GGA AAT TTA GTG TTC ATT GCT AAT GAT TGG CAT 622
Val Cys Tyr Gly Asp Gly Asn Leu Val Phe Ile Ala Asn Asp Trp His
            195                 200                 205

ACT GCT TTA TTG CCA GTA TAT CTG AAA GCT TAT TAT CGT GAC AAT GGA 670
Thr Ala Leu Leu Pro Val Tyr Leu Lys Ala Tyr Tyr Arg Asp Asn Gly
        210                 215                 220

ATT ATG AAC TAT ACA AGA TCT GTC CTG GTG ATT CAT AAC ATC GCT CAT 718
Ile Met Asn Tyr Thr Arg Ser Val Leu Val Ile His Asn Ile Ala His
    225                 230                 235

CAG GGT CGT GGT CCT TTG GAG GAT TTT TCA TAT GTA GAT CTT CCA CCA 766
Gln Gly Arg Gly Pro Leu Glu Asp Phe Ser Tyr Val Asp Leu Pro Pro
240                 245                 250                 255

CAC TAT ATG GAC CCT TTC AAG TTG TAT GAC CCA GTA GGA GGT GAG CAT 814
His Tyr Met Asp Pro Phe Lys Leu Tyr Asp Pro Val Gly Gly Glu His
                260                 265                 270

TTC AAC ATT TTT GCG GCT GGT CTA AAG ACA GCA GAT CGT GTA GTT ACA 862
Phe Asn Ile Phe Ala Ala Gly Leu Lys Thr Ala Asp Arg Val Val Thr
            275                 280                 285

GTT AGT CAT GGA TAT TCA TGG GAA CTA AAG ACT TCC CAA GGT GGT TGG 910
Val Ser His Gly Tyr Ser Trp Glu Leu Lys Thr Ser Gln Gly Gly Trp
        290                 295                 300

GGA TTG CAT CAG ATA ATT AAT GAG AAC GAT TGG AAA TTA CAG GGT ATT 958
Gly Leu His Gln Ile Ile Asn Glu Asn Asp Trp Lys Leu Gln Gly Ile
    305                 310                 315

GTG AAT GGG ATT GAT ACA AAA GAG TGG AAC CCT GAG TTG GAC GTT CAC 1006
Val Asn Gly Ile Asp Thr Lys Glu Trp Asn Pro Glu Leu Asp Val His
320                 325                 330                 335

TTA CAG TCA GAT GGT TAC ATG AAC TAC TCC TTG GAC ACG CTA CAG ACT 1054
Leu Gln Ser Asp Gly Tyr Met Asn Tyr Ser Leu Asp Thr Leu Gln Thr
                340                 345                 350

GGC AAG CCT CAA TGT AAA GCT GCA TTG CAG AAG GAA CTT GGT TTA CCA     1102
Gly Lys Pro Gln Cys Lys Ala Ala Leu Gln Lys Glu Leu Gly Leu Pro
            355                 360                 365

GTT CGT GAT GAT GTC CCA CTG ATC GGT TTC ATT GGG AGG CTT GAC CCA     1150
Val Arg Asp Asp Val Pro Leu Ile Gly Phe Ile Gly Arg Leu Asp Pro
        370                 375                 380

CAA AAG GGT GTT GAT CTG ATT GCT GAG GCC AGT GCT TGG ATG ATG GGT     1198
Gln Lys Gly Val Asp Leu Ile Ala Glu Ala Ser Ala Trp Met Met Gly
    385                 390                 395

CAG GAT GTA CAA CTG GTC ATG TTG GGG ACG GGG AGG CGT GAC CTT GAA     1246
Gln Asp Val Gln Leu Val Met Leu Gly Thr Gly Arg Arg Asp Leu Glu
400                 405                 410                 415

CAG ATG CTA AGG CAA TTT GAG TGT CAA CAC AAT GAT AAA ATT AGA GGA     1294
Gln Met Leu Arg Gln Phe Glu Cys Gln His Asn Asp Lys Ile Arg Gly
                420                 425                 430

TGG GTT GGT TTC TCT GTG AAG ACT TCT CAT CGT ATA ACT GCT GGT GCA     1342
Trp Val Gly Phe Ser Val Lys Thr Ser His Arg Ile Thr Ala Gly Ala
            435                 440                 445

GAC ATT CTG CTC ATG CCT TCT AGA TTT GAG GCC TTG CGA CTG AAC CAG     1390
Asp Ile Leu Leu Met Pro Ser Arg Phe Glu Ala Leu Arg Leu Asn Gln
        450                 455                 460

CTT TAT GCA ATG AAA TAT GGG ACT ATT CCT GTT GTT CAT GCA GTA GGA     1438
Leu Tyr Ala Met Lys Tyr Gly Thr Ile Pro Val Val His Ala Val Gly
    465                 470                 475

GGA CTC AGA GAT ACT GTG CAG CCC TTT GAT CCT TTT AAT GAG TCA GGA     1486
Gly Leu Arg Asp Thr Val Gln Pro Phe Asp Pro Phe Asn Glu Ser Gly
480                 485                 490                 495

CTG GGG TGG ACC TTC AGT AGG GCT GAA GCT AGC CAG CTG ATC CAC GCA     1534
Leu Gly Trp Thr Phe Ser Arg Ala Glu Ala Ser Gln Leu Ile His Ala
                500                 505                 510

TTA GGA AAT TGC TTA CTG ACT TAT CGT GAG TAC AAA AAG AGT TGG GAG     1582
Leu Gly Asn Cys Leu Leu Thr Tyr Arg Glu Tyr Lys Lys Ser Trp Glu
            515                 520                 525

GGG ATT CAG ACA CGT TGT ATG ACA CAA GAC TTA AGT TGG GAT AAT GCT     1630
Gly Ile Gln Thr Arg Cys Met Thr Gln Asp Leu Ser Trp Asp Asn Ala
        530                 535                 540

GCT CAG AAC TAT GAA GAA GTT CTC ATC GCT GCT AAG TAT CAG TGG         1675
Ala Gln Asn Tyr Glu Glu Val Leu Ile Ala Ala Lys Tyr Gln Trp
    545                 550                 555

TGAGGTTCAT TACTTGTAGA TATTTGGGGA TTTTGGCCAT TGTATCAAGT TCTAATGATG     1735

GGATTTCAGA GACATGTTTC TGGTATCGAC ACGAGAGGAT GCATGCAACA AGTTGGCTAA     1795

CTATCATACT ACTACCACGT CAGGAATGAT TGCCGCACTT GATCATGTAA TCATGTATAT 1855

ACTCTATTTT GTTTGCAAAA TGTAGTTACA TGTTGCAATT TCTAAAAAAA AAAAAAAAAA 1915

AAAAAAAAAA A 1926

(2) ANGABEN ZU SEQ ID NO: 6:

(i) SEQUENZKENNZEICHEN:
(A) LÄNGE: 558 Aminosäuren
(B) ART: Aminosäure
(D) TOPOLOGIE: linear

(ii) ART DES MOLEKÜLS: Protein
(xi) SEQUENZBESCHREIBUNG: SEQ ID NO: 6:

Gly Thr Ser Lys Ser Leu Val Asp Val Pro Gly Lys Lys Ile Gln Ser
1               5                   10                  15

Tyr Met Pro Ser Leu Arg Lys Glu Ser Ser Ala Ser His Val Glu Gln
            20                  25                  30

Arg Asn Glu Asn Leu Glu Gly Ser Ser Ala Glu Ala Asn Glu Glu Thr
        35                  40                  45

Glu Asp Pro Val Asn Ile Asp Glu Lys Pro Pro Pro Leu Ala Gly Thr
    50                  55                  60

Asn Val Met Asn Ile Ile Leu Val Ala Ser Glu Cys Ala Pro Trp Ser
65                  70                  75                  80

Lys Thr Gly Gly Leu Gly Asp Val Ala Gly Ala Leu Pro Lys Ala Leu
                85                  90                  95

Ala Arg Arg Gly His Arg Val Met Val Val Ala Pro Arg Tyr Asp Asn
            100                 105                 110

Tyr Pro Glu Pro Gln Asp Ser Gly Val Arg Lys Ile Tyr Lys Val Asp
        115                 120                 125

Gly Gln Asp Val Glu Val Thr Tyr Phe Gln Ala Phe Ile Asp Gly Val
    130                 135                 140

Asp Phe Val Phe Ile Asp Ser His Met Phe Arg His Ile Gly Asn Asn
145                 150                 155                 160

Ile Tyr Gly Gly Asn Arg Val Asp Ile Leu Lys Arg Met Val Leu Phe
                165                 170                 175

Cys Lys Ala Ala Ile Glu Val Pro Trp His Val Pro Cys Gly Gly Val
            180                 185                 190

Cys Tyr Gly Asp Gly Asn Leu Val Phe Ile Ala Asn Asp Trp His Thr
        195                 200                 205

Ala Leu Leu Pro Val Tyr Leu Lys Ala Tyr Tyr Arg Asp Asn Gly Ile
    210                 215                 220

Met Asn Tyr Thr Arg Ser Val Leu Val Ile His Asn Ile Ala His Gln
225                 230                 235                 240

Gly Arg Gly Pro Leu Glu Asp Phe Ser Tyr Val Asp Leu Pro Pro His
                245                 250                 255

Tyr Met Asp Pro Phe Lys Leu Tyr Asp Pro Val Gly Gly Glu His Phe
            260                 265                 270

Asn Ile Phe Ala Ala Gly Leu Lys Thr Ala Asp Arg Val Val Thr Val
        275                 280                 285

Ser His Gly Tyr Ser Trp Glu Leu Lys Thr Ser Gln Gly Gly Trp Gly
    290                 295                 300

Leu His Gln Ile Ile Asn Glu Asn Asp Trp Lys Leu Gln Gly Ile Val
305                 310                 315                 320

Asn Gly Ile Asp Thr Lys Glu Trp Asn Pro Glu Leu Asp Val His Leu
                325                 330                 335

Gln Ser Asp Gly Tyr Met Asn Tyr Ser Leu Asp Thr Leu Gln Thr Gly
            340                 345                 350

Lys Pro Gln Cys Lys Ala Ala Leu Gln Lys Glu Leu Gly Leu Pro Val
        355                 360                 365

Arg Asp Asp Val Pro Leu Ile Gly Phe Ile Gly Arg Leu Asp Pro Gln
    370                 375                 380

Lys Gly Val Asp Leu Ile Ala Glu Ala Ser Ala Trp Met Met Gly Gln
385                 390                 395                 400

Asp Val Gln Leu Val Met Leu Gly Thr Gly Arg Arg Asp Leu Glu Gln
                405                 410                 415

Met Leu Arg Gln Phe Glu Cys Gln His Asn Asp Lys Ile Arg Gly Trp
            420                 425                 430

Val Gly Phe Ser Val Lys Thr Ser His Arg Ile Thr Ala Gly Ala Asp
        435                 440                 445

Ile Leu Leu Met Pro Ser Arg Phe Glu Ala Leu Arg Leu Asn Gln Leu
    450                 455                 460

Tyr Ala Met Lys Tyr Gly Thr Ile Pro Val Val His Ala Val Gly Gly
465                 470                 475                 480

Leu Arg Asp Thr Val Gln Pro Phe Asp Pro Phe Asn Glu Ser Gly Leu
                485                 490                 495

Gly Trp Thr Phe Ser Arg Ala Glu Ala Ser Gln Leu Ile His Ala Leu
            500                 505                 510

Gly Asn Cys Leu Leu Thr Tyr Arg Glu Tyr Lys Lys Ser Trp Glu Gly
        515                 520                 525

Ile Gln Thr Arg Cys Met Thr Gln Asp Leu Ser Trp Asp Asn Ala Ala
    530                 535                 540

Gln Asn Tyr Glu Glu Val Leu Ile Ala Ala Lys Tyr Gln Trp
545                 550                 555

(2) ANGABEN ZU SEQ ID NO: 7:

     (i) SEQUENZKENNZEICHEN:
          (A) LÄNGE: 2793 Basenpaare
          (B) ART: Nucleotid
          (C) STRANGFORM: Einzelstrang
          (D) TOPOLOGIE: linear

    (ii) ART DES MOLEKÜLS: cDNA zu mRNA

    (vi) URSPRÜNLICHE HERKUNFT:
          (A) ORGANISMUS: Solanum tuberosum
          (B) STAMM: cv Désirée
          (F) GEWEBETYP: Blattgewebe

   (vii) UNMITTELBARE HERKUNFT:
          (A) BIBLIOTHEK: cDNA-Bibliothek in Lambda ZAPII

    (ix) MERKMAL:
          (A) NAME/SCHLÜSSEL: CDS
          (B) LAGE:242..2542

    (xi) SEQUENZBESCHREIBUNG: SEQ ID NO: 7:

CCGCCCATTT TTCACCAAAC GTTTTTGACA TTGACCTCCA TTGTCGTTAC TTCTTGGTTT      60

CTCTTTCAAT ATTGCTTCAC AATCCCTAAT TCTCTGTACT AGTCTCTATC TCAATTGGGT     120

TTTCTTTACT TGTCAATTAT CTCTACTGGG TCGGCTTCTA TTTCCACTAG GTCACTCTGG     180

TTCTTGAAAT CTTGGATTCC TATTATCCCT GTGAACTTCA TCTTTTGTGA TTTCTACTGT     240

A ATG GAG AAT TCC ATT CTT CTT CAT AGT GGA AAT CAG TTC CAC CCC         286
  Met Glu Asn Ser Ile Leu Leu His Ser Gly Asn Gln Phe His Pro
    1               5                  10                  15

AAC TTA CCC CTT TTA GCA CTT AGG CCC AAA AAA TTA TCT CTA ATT CAT       334
Asn Leu Pro Leu Leu Ala Leu Arg Pro Lys Lys Leu Ser Leu Ile His
                 20                  25                  30

GGC TCC AGT AGA GAG CAA ATG TGG AGG ATC AAG CGC GTT AAA GCA ACA 382
Gly Ser Ser Arg Glu Gln Met Trp Arg Ile Lys Arg Val Lys Ala Thr
            35                  40                  45

GGT GAA AAT TCT GGG GAA GCT GCA AGT GCT GAT GAA TCG AAT GAT GCC 430
Gly Glu Asn Ser Gly Glu Ala Ala Ser Ala Asp Glu Ser Asn Asp Ala
        50                  55                  60

TTA CAG GTT ACA ATT GAA AAG AGC AAA AAG GTT TTA GCC ATG CAA CAG 478
Leu Gln Val Thr Ile Glu Lys Ser Lys Lys Val Leu Ala Met Gln Gln
    65                  70                  75

GAC CTA CTT CAA CAG ATT GCA GAA AGA AGA AAA GTA GTC TCT TCA ATA 526
Asp Leu Leu Gln Gln Ile Ala Glu Arg Arg Lys Val Val Ser Ser Ile
80                  85                  90                  95

AAA AGC AGT CTT GCC AAT GCC AAA GGT ACT TAT GAT GGT GGG AGT GGT 574
Lys Ser Ser Leu Ala Asn Ala Lys Gly Thr Tyr Asp Gly Gly Ser Gly
                100                 105                 110

AGC TTA TCA GAT GTT GAT ATC CCT GAC GTG GAT AAA GAT TAT AAT GTT 622
Ser Leu Ser Asp Val Asp Ile Pro Asp Val Asp Lys Asp Tyr Asn Val
            115                 120                 125

ACT GTA CCT AGT ACT GCT GCT ACT CCA ATT ACT GAT GTC GAT AAA AAT 670
Thr Val Pro Ser Thr Ala Ala Thr Pro Ile Thr Asp Val Asp Lys Asn
        130                 135                 140

ACA CCG CCT GCT ATA AGC CAA GAT TTT GTT GAA AGT AAA AGA GAA ATC 718
Thr Pro Pro Ala Ile Ser Gln Asp Phe Val Glu Ser Lys Arg Glu Ile
    145                 150                 155

AAA AGG GAC CTG GCC GAT GAA AGG GCA CCC CCA CTG TCG AGA TCA TCT 766
Lys Arg Asp Leu Ala Asp Glu Arg Ala Pro Pro Leu Ser Arg Ser Ser
160                 165                 170                 175

ATC ACA GCC AGT AGC CAG ATT TCC TCT ACT GTA AGT TCC AAA AGA ACG 814
Ile Thr Ala Ser Ser Gln Ile Ser Ser Thr Val Ser Ser Lys Arg Thr
                180                 185                 190

TTG AAT GTC CCT CCA GAA ACT CCG AAG TCC AGT CAA GAG ACA CTT TTG 862
Leu Asn Val Pro Pro Glu Thr Pro Lys Ser Ser Gln Glu Thr Leu Leu
            195                 200                 205

GAT GTG AAT TCA CGC AAA AGT TTA GTA GAT GTT CCT GGA AAG AAG ATC 910
Asp Val Asn Ser Arg Lys Ser Leu Val Asp Val Pro Gly Lys Lys Ile
        210                 215                 220

CAG TCT TAT ATG CCT TCA TTA CGT AAA GAA TCC TCA GCA TCC CAT GTG 958
Gln Ser Tyr Met Pro Ser Leu Arg Lys Glu Ser Ser Ala Ser His Val
    225                 230                 235

GAA CAG AGG AAT GAA AAT CTT GAA GGA TCA AGT GCT GAG GCA AAC GAA 1006
Glu Gln Arg Asn Glu Asn Leu Glu Gly Ser Ser Ala Glu Ala Asn Glu
240                 245                 250                 255

GAG ACT GAA GAT CCT GTG AAT ATA GAT GAG AAA CCC CCT CCA TTG GCA 1054
Glu Thr Glu Asp Pro Val Asn Ile Asp Glu Lys Pro Pro Pro Leu Ala
                260                 265                 270

GGA ACA AAT GTT ATG AAC ATT ATT TTG GTG GCT TCA GAA TGC GCT CCA 1102
Gly Thr Asn Val Met Asn Ile Ile Leu Val Ala Ser Glu Cys Ala Pro
            275                 280                 285

TGG TCT AAA ACA GGT GGG CTT GGA GAT GTT GCT GGA GCA TTA CCC AAA 1150
Trp Ser Lys Thr Gly Gly Leu Gly Asp Val Ala Gly Ala Leu Pro Lys
        290                 295                 300

GCT TTG GCT CGA CGT GGC CAC AGA GTT ATG GTT GTG GCA CCT CGT TAT 1198
Ala Leu Ala Arg Arg Gly His Arg Val Met Val Val Ala Pro Arg Tyr
    305                 310                 315

GAC AAC TAT CCT GAA CCT CAA GAT TCT GGT GTA AGA AAA ATT TAT AAA 1246
Asp Asn Tyr Pro Glu Pro Gln Asp Ser Gly Val Arg Lys Ile Tyr Lys
320                 325                 330                 335

GTT GAT GGT CAG GAT GTG GAA GTG ACT TAC TTC CAA GCT TTT ATT GAT 1294
Val Asp Gly Gln Asp Val Glu Val Thr Tyr Phe Gln Ala Phe Ile Asp
                340                 345                 350

GGT GTG GAT TTT GTT TTC ATT GAC AGT CAT ATG TTT AGA CAC ATT GGG 1342
Gly Val Asp Phe Val Phe Ile Asp Ser His Met Phe Arg His Ile Gly
            355                 360                 365

AAC AAC ATT TAC GGA GGG AAC CGT GTG GAT ATT TTA AAA CGC ATG GTT 1390
Asn Asn Ile Tyr Gly Gly Asn Arg Val Asp Ile Leu Lys Arg Met Val
        370                 375                 380

TTA TTT TGC AAA GCA GCG ATT GAG GTT CCT TGG CAT GTT CCA TGT GGT 1438
Leu Phe Cys Lys Ala Ala Ile Glu Val Pro Trp His Val Pro Cys Gly
    385                 390                 395

GGG GTC TGC TAT GGA GAT GGA AAT TTA GTG TTC ATT GCT AAT GAT TGG 1486
Gly Val Cys Tyr Gly Asp Gly Asn Leu Val Phe Ile Ala Asn Asp Trp
400                 405                 410                 415

CAT ACT GCT TTA TTG CCA GTA TAT CTG AAA GCT TAT TAT CGT GAC AAT 1534
His Thr Ala Leu Leu Pro Val Tyr Leu Lys Ala Tyr Tyr Arg Asp Asn
                420                 425                 430

GGA ATT ATG AAC TAT ACA AGA TCT GTC CTG GTG ATT CAT AAC ATC GCT 1582
Gly Ile Met Asn Tyr Thr Arg Ser Val Leu Val Ile His Asn Ile Ala
            435                 440                 445

CAT CAG GGT CGT GGT CCT TTG GAG GAT TTT TCA TAT GTA GAT CTT CCA 1630
His Gln Gly Arg Gly Pro Leu Glu Asp Phe Ser Tyr Val Asp Leu Pro
        450                 455                 460

CCA CAC TAT ATG GAC CCT TTC AAG TTG TAT GAC CCA GTA GGA GGT GAG 1678
Pro His Tyr Met Asp Pro Phe Lys Leu Tyr Asp Pro Val Gly Gly Glu
    465                 470                 475

CAT TTC AAC ATT TTT GCG GCT GGT CTA AAG ACA GCA GAT CGT GTA GTT 1726
His Phe Asn Ile Phe Ala Ala Gly Leu Lys Thr Ala Asp Arg Val Val
480                 485                 490                 495

ACA GTT AGT CAT GGA TAT TCA TGG GAA CTA AAG ACT TCC CAA GGT GGT 1774
Thr Val Ser His Gly Tyr Ser Trp Glu Leu Lys Thr Ser Gln Gly Gly
                500                 505                 510

TGG GGA TTG CAT CAG ATA ATT AAT GAG AAC GAT TGG AAA TTA CAG GGT 1822
Trp Gly Leu His Gln Ile Ile Asn Glu Asn Asp Trp Lys Leu Gln Gly
            515                 520                 525

ATT GTG AAT GGG ATT GAT ACA AAA GAG TGG AAC CCT GAG TTG GAC GTT 1870
Ile Val Asn Gly Ile Asp Thr Lys Glu Trp Asn Pro Glu Leu Asp Val
        530                 535                 540

CAC TTA CAG TCA GAT GGT TAC ATG AAC TAC TCC TTG GAC ACG CTA CAG 1918
His Leu Gln Ser Asp Gly Tyr Met Asn Tyr Ser Leu Asp Thr Leu Gln
    545                 550                 555

ACT GGC AAG CCT CAA TGT AAA GCT GCA TTG CAG AAG GAA CTT GGT TTA 1966
Thr Gly Lys Pro Gln Cys Lys Ala Ala Leu Gln Lys Glu Leu Gly Leu
560                 565                 570                 575

CCA GTT CGT GAT GAT GTC CCA CTG ATC GGT TTC ATT GGG AGG CTT GAC 2014
Pro Val Arg Asp Asp Val Pro Leu Ile Gly Phe Ile Gly Arg Leu Asp
                580                 585                 590

CCA CAA AAG GGT GTT GAT CTG ATT GCT GAG GCC AGT GCT TGG ATG ATG 2062
Pro Gln Lys Gly Val Asp Leu Ile Ala Glu Ala Ser Ala Trp Met Met
            595                 600                 605

GGT CAG GAT GTA CAA CTG GTC ATG TTG GGG ACG GGG AGG CGT GAC CTT 2110
Gly Gln Asp Val Gln Leu Val Met Leu Gly Thr Gly Arg Arg Asp Leu
        610                 615                 620

GAA CAG ATG CTA AGG CAA TTT GAG TGT CAA CAC AAT GAT AAA ATT AGA 2158
Glu Gln Met Leu Arg Gln Phe Glu Cys Gln His Asn Asp Lys Ile Arg
    625                 630                 635

GGA TGG GTT GGT TTC TCT GTG AAG ACT TCT CAT CGT ATA ACT GCT GGT 2206
Gly Trp Val Gly Phe Ser Val Lys Thr Ser His Arg Ile Thr Ala Gly
640                 645                 650                 655

GCA GAC ATT CTG CTC ATG CCT TCT AGA TTT GAG CCT TGC GGA CTG AAC 2254
Ala Asp Ile Leu Leu Met Pro Ser Arg Phe Glu Pro Cys Gly Leu Asn
                660                 665                 670

CAG CTT TAT GCA ATG AAA TAT GGG ACT ATT CCT GTT GTT CAT GCA GTA 2302
Gln Leu Tyr Ala Met Lys Tyr Gly Thr Ile Pro Val Val His Ala Val
            675                 680                 685

GGA GGA CTC AGA GAT ACT GTG CAG CCC TTT GAT CCT TTT AAT GAG TCA 2350
Gly Gly Leu Arg Asp Thr Val Gln Pro Phe Asp Pro Phe Asn Glu Ser
        690                 695                 700

GGA CTG GGG TGG ACC TTC AGT AGG GCT GAA GCT AGC CAG CTG ATC CAC 2398
Gly Leu Gly Trp Thr Phe Ser Arg Ala Glu Ala Ser Gln Leu Ile His
    705                 710                 715

GCA TTA GGA AAT TGC TTA CTG ACT TAT CGT GAG TAC AAA AAG AGT TGG 2446
Ala Leu Gly Asn Cys Leu Leu Thr Tyr Arg Glu Tyr Lys Lys Ser Trp
720                 725                 730                 735

GAG GGG ATT CAG ACA CGT TGT ATG ACA CAA GAC TTA AGT TGG GAT AAT 2494
Glu Gly Ile Gln Thr Arg Cys Met Thr Gln Asp Leu Ser Trp Asp Asn
                740                 745                 750

GCT GCT CAG AAC TAT GAA GAA GTT CTC ATC GCT GCT AAG TAT CAG TGG 2542
Ala Ala Gln Asn Tyr Glu Glu Val Leu Ile Ala Ala Lys Tyr Gln Trp
            755                 760                 765

TGAGGTTCAT TACTTGTAGA TATTTGGGGA TTTTGGCCAT TGTATCAAGT TCTAATGATG 2602

GGATTTCAGA GACATGTTTC TGGTATCGAC ACGAGAGGAT GCATGCAACA AGTTGGCTAA 2662

CTATCATACT ACTACCACGT CAGGAATGAT TGCCGCACTT GATCATGTAA TCATGTATAT 2722

ACTCTATTTT GTTTGCAAAA TGTAGTTACA TGTTGCAATT TCTAAAAAAA AAAAAAAAAA 2782

AAAAAAAAAA A 2793

(2) ANGABEN ZU SEQ ID NO: 8:

(i) SEQUENZKENNZEICHEN:
(A) LÄNGE: 767 Aminosäuren
(B) ART: Aminosäure
(D) TOPOLOGIE: linear

(ii) ART DES MOLEKÜLS: Protein
(xi) SEQUENZBESCHREIBUNG: SEQ ID NO: 8:

Met Glu Asn Ser Ile Leu Leu His Ser Gly Asn Gln Phe His Pro Asn
1               5                   10                  15

Leu Pro Leu Leu Ala Leu Arg Pro Lys Lys Leu Ser Leu Ile His Gly
            20                  25                  30

Ser Ser Arg Glu Gln Met Trp Arg Ile Lys Arg Val Lys Ala Thr Gly
        35                  40                  45

Glu Asn Ser Gly Glu Ala Ala Ser Ala Asp Glu Ser Asn Asp Ala Leu
50                  55                  60

Gln Val Thr Ile Glu Lys Ser Lys Lys Val Leu Ala Met Gln Gln Asp
65                  70                  75                  80

Leu Leu Gln Gln Ile Ala Glu Arg Arg Lys Val Val Ser Ser Ile Lys
                85                  90                  95

Ser Ser Leu Ala Asn Ala Lys Gly Thr Tyr Asp Gly Gly Ser Gly Ser
            100                 105                 110

Leu Ser Asp Val Asp Ile Pro Asp Val Asp Lys Asp Tyr Asn Val Thr
        115                 120                 125

Val Pro Ser Thr Ala Ala Thr Pro Ile Thr Asp Val Asp Lys Asn Thr
    130                 135                 140

Pro Pro Ala Ile Ser Gln Asp Phe Val Glu Ser Lys Arg Glu Ile Lys
145                 150                 155                 160

Arg Asp Leu Ala Asp Glu Arg Ala Pro Pro Leu Ser Arg Ser Ser Ile
                165                 170                 175

Thr Ala Ser Ser Gln Ile Ser Ser Thr Val Ser Ser Lys Arg Thr Leu
            180                 185                 190

Asn Val Pro Pro Glu Thr Pro Lys Ser Ser Gln Glu Thr Leu Leu Asp
        195                 200                 205

Val Asn Ser Arg Lys Ser Leu Val Asp Val Pro Gly Lys Lys Ile Gln
    210                 215                 220

Ser Tyr Met Pro Ser Leu Arg Lys Glu Ser Ser Ala Ser His Val Glu
225                 230                 235                 240

Gln Arg Asn Glu Asn Leu Glu Gly Ser Ser Ala Glu Ala Asn Glu Glu
                245                 250                 255

Thr Glu Asp Pro Val Asn Ile Asp Glu Lys Pro Pro Pro Leu Ala Gly
            260                 265                 270

Thr Asn Val Met Asn Ile Ile Leu Val Ala Ser Glu Cys Ala Pro Trp
        275                 280                 285

Ser Lys Thr Gly Gly Leu Gly Asp Val Ala Gly Ala Leu Pro Lys Ala
    290                 295                 300

Leu Ala Arg Arg Gly His Arg Val Met Val Val Ala Pro Arg Tyr Asp
305                 310                 315                 320

Asn Tyr Pro Glu Pro Gln Asp Ser Gly Val Arg Lys Ile Tyr Lys Val
                325                 330                 335

Asp Gly Gln Asp Val Glu Val Thr Tyr Phe Gln Ala Phe Ile Asp Gly
            340                 345                 350

Val Asp Phe Val Phe Ile Asp Ser His Met Phe Arg His Ile Gly Asn
        355                 360                 365

Asn Ile Tyr Gly Gly Asn Arg Val Asp Ile Leu Lys Arg Met Val Leu
    370                 375                 380

Phe Cys Lys Ala Ala Ile Glu Val Pro Trp His Val Pro Cys Gly Gly
385                 390                 395                 400

Val Cys Tyr Gly Asp Gly Asn Leu Val Phe Ile Ala Asn Asp Trp His
                405                 410                 415

Thr Ala Leu Leu Pro Val Tyr Leu Lys Ala Tyr Tyr Arg Asp Asn Gly
            420                 425                 430

Ile Met Asn Tyr Thr Arg Ser Val Leu Val Ile His Asn Ile Ala His
        435                 440                 445

Gln Gly Arg Gly Pro Leu Glu Asp Phe Ser Tyr Val Asp Leu Pro Pro
    450                 455                 460

His Tyr Met Asp Pro Phe Lys Leu Tyr Asp Pro Val Gly Gly Glu His
465                 470                 475                 480

Phe Asn Ile Phe Ala Ala Gly Leu Lys Thr Ala Asp Arg Val Val Thr
                485                 490                 495

Val Ser His Gly Tyr Ser Trp Glu Leu Lys Thr Ser Gln Gly Gly Trp
            500                 505                 510

Gly Leu His Gln Ile Ile Asn Glu Asn Asp Trp Lys Leu Gln Gly Ile
        515                 520                 525

Val Asn Gly Ile Asp Thr Lys Glu Trp Asn Pro Glu Leu Asp Val His
    530                 535                 540

Leu Gln Ser Asp Gly Tyr Met Asn Tyr Ser Leu Asp Thr Leu Gln Thr
545                 550                 555                 560

Gly Lys Pro Gln Cys Lys Ala Ala Leu Gln Lys Glu Leu Gly Leu Pro
                565                 570                 575

Val Arg Asp Asp Val Pro Leu Ile Gly Phe Ile Gly Arg Leu Asp Pro
            580                 585                 590

Gln Lys Gly Val Asp Leu Ile Ala Glu Ala Ser Ala Trp Met Met Gly
        595                 600                 605

Gln Asp Val Gln Leu Val Met Leu Gly Thr Gly Arg Arg Asp Leu Glu
    610                 615                 620

Gln Met Leu Arg Gln Phe Glu Cys Gln His Asn Asp Lys Ile Arg Gly
625                 630                 635                 640

Trp Val Gly Phe Ser Val Lys Thr Ser His Arg Ile Thr Ala Gly Ala
                645                 650                 655

Asp Ile Leu Leu Met Pro Ser Arg Phe Glu Pro Cys Gly Leu Asn Gln
            660                 665                 670

Leu Tyr Ala Met Lys Tyr Gly Thr Ile Pro Val Val His Ala Val Gly
        675                 680                 685

Gly Leu Arg Asp Thr Val Gln Pro Phe Asp Pro Phe Asn Glu Ser Gly
    690                 695                 700

Leu Gly Trp Thr Phe Ser Arg Ala Glu Ala Ser Gln Leu Ile His Ala
705                 710                 715                 720

Leu Gly Asn Cys Leu Leu Thr Tyr Arg Glu Tyr Lys Lys Ser Trp Glu
                725                 730                 735

Gly Ile Gln Thr Arg Cys Met Thr Gln Asp Leu Ser Trp Asp Asn Ala
            740                 745                 750

Ala Gln Asn Tyr Glu Glu Val Leu Ile Ala Ala Lys Tyr Gln Trp
        755                 760                 765

(2) ANGABEN ZU SEQ ID NO: 9:

(i) SEQUENZKENNZEICHEN:
(A) LÄNGE: 2360 Basenpaare
(B) ART: Nucleotid
(C) STRANGFORM: Einzelstrang
(D) TOPOLOGIE: linear

(ii) ART DES MOLEKÜLS: cDNA

(vi) URSPRÜNLICHE HERKUNFT:
(A) ORGANISMUS: Solanum tuberosum
(B) STAMM: cv. Désirée
(F) GEWEBETYP: Blattgewebe

(vii) UNMITTELBARE HERKUNFT:
(A) BIBLIOTHEK: cDNA-Bibliothek in Lambda ZAPII

(ix) MERKMAL:
(A) NAME/SCHLÜSSEL: CDS
(B) LAGE:68..1990

(xi) SEQUENZBESCHREIBUNG: SEQ ID NO: 9:

AGATTTTCTA TATTGAAAGA TTTTGTCTTT ACATGATTCT TGATTTTACA GCAGGTGTCA        60

ATACCAA ATG GGG TCT CTG CAA ACA CCC ACA AAT CTT AGC AAT AAG TCA 109
Met Gly Ser Leu Gln Thr Pro Thr Asn Leu Ser Asn Lys Ser
1               5                   10

TGT TTA TGT GTG TCA GGG AGA GTT GTG AAG GGT TTG AGG GTA GAA AGA 157
Cys Leu Cys Val Ser Gly Arg Val Val Lys Gly Leu Arg Val Glu Arg
15                  20                  25                  30

CAA GTG GGG TTG GGG TTT TCT TGG TTG TTG AAG GGA CGA AGA AAC AGA 205
Gln Val Gly Leu Gly Phe Ser Trp Leu Leu Lys Gly Arg Arg Asn Arg
                35                  40                  45

AAA GTT CAA TCT TTG TGT GTT ACA AGT AGT GTT TCA GAT GGT TCA TCA 253
Lys Val Gln Ser Leu Cys Val Thr Ser Ser Val Ser Asp Gly Ser Ser
            50                  55                  60

ATT GCT GAA AAT AAG AAT GTG TCA GAA GGG CTT CTT TTG GGT GCT GAG 301
Ile Ala Glu Asn Lys Asn Val Ser Glu Gly Leu Leu Leu Gly Ala Glu
        65                  70                  75

AGA GAT GGT TCT GGC TCT GTT GTT GGT TTT CAA TTG ATT CCA CAT TCT 349
Arg Asp Gly Ser Gly Ser Val Val Gly Phe Gln Leu Ile Pro His Ser
    80                  85                  90

GTT GCA GGA GAT GCA ACA ATG GTA GAA TCT CAT GAT ATT GTA GCC AAT 397
Val Ala Gly Asp Ala Thr Met Val Glu Ser His Asp Ile Val Ala Asn
95                  100                 105                 110

GAT AGA GAT GAC TTG AGT GAG GAT ACT GAG GAG ATG GAG GAA ACC CCA 445
Asp Arg Asp Asp Leu Ser Glu Asp Thr Glu Glu Met Glu Glu Thr Pro
                115                 120                 125

ATC AAA TTA ACT TTC AAT ATC ATT TTT GTT ACT GCT GAA GCA GCT CCA 493
Ile Lys Leu Thr Phe Asn Ile Ile Phe Val Thr Ala Glu Ala Ala Pro
            130                 135                 140

TAT TCT AAG ACT GGT GGA TTA GGA GAT GTT TGT GGT TCT TTG CCA ATG 541
Tyr Ser Lys Thr Gly Gly Leu Gly Asp Val Cys Gly Ser Leu Pro Met
        145                 150                 155

GCA CTA GCT GCT CGG GGT CAT CGT GTA ATG GTC GTT TCA CCT AGG TAT 589
Ala Leu Ala Ala Arg Gly His Arg Val Met Val Val Ser Pro Arg Tyr
    160                 165                 170

TTG AAT GGA GGT CCT TCA GAT GAA AAG TAC GCC AAT GCT GTT GAC CTT 637
Leu Asn Gly Gly Pro Ser Asp Glu Lys Tyr Ala Asn Ala Val Asp Leu
175                 180                 185                 190

GAT GTG CGG GCC ACT GTC CAT TGC TTT GGT GAT GCA CAG GAA GTA GCC 685
Asp Val Arg Ala Thr Val His Cys Phe Gly Asp Ala Gln Glu Val Ala
                195                 200                 205

TTC TAC CAT GAA TAC AGG GCA GGT GTT GAT TGG GTA TTT GTG GAC CAC 733
Phe Tyr His Glu Tyr Arg Ala Gly Val Asp Trp Val Phe Val Asp His
            210                 215                 220

TCT TCT TAC TGC AGA CCT GGA ACG CCA TAT GGT GAT ATT TAT GGT GCA 781
Ser Ser Tyr Cys Arg Pro Gly Thr Pro Tyr Gly Asp Ile Tyr Gly Ala
        225                 230                 235

TTT GGT GAT AAT CAG TTT CGC TTC ACT TTG CTT TCT CAC GCA GCA TGT 829
Phe Gly Asp Asn Gln Phe Arg Phe Thr Leu Leu Ser His Ala Ala Cys
    240                 245                 250

GAA GCG CCA TTG GTT CTT CCA CTG GGA GGG TTC ACT TAT GGA GAG AAG 877
Glu Ala Pro Leu Val Leu Pro Leu Gly Gly Phe Thr Tyr Gly Glu Lys
255                 260                 265                 270

TGC TTG TTT CTC GCT AAT GAT TGG CAT GCT GCC CTG GTT CCT TTA CTT 925
Cys Leu Phe Leu Ala Asn Asp Trp His Ala Ala Leu Val Pro Leu Leu
                275                 280                 285

TTA GCG GCC AAG TAT CGT CCT TAT GGT GTT TAC AAG GAT GCT CGT AGT 973
Leu Ala Ala Lys Tyr Arg Pro Tyr Gly Val Tyr Lys Asp Ala Arg Ser
            290                 295                 300

ATT GTC GCA ATA CAC AAC ATT GCA CAT CAG GGA GTG GAG CCT GCA GTA 1021
Ile Val Ala Ile His Asn Ile Ala His Gln Gly Val Glu Pro Ala Val
        305                 310                 315

ACC TAC AAT AAT TTG GGT TTG CCT CCA CAA TGG TAT GGA GCA GTT GAA 1069
Thr Tyr Asn Asn Leu Gly Leu Pro Pro Gln Trp Tyr Gly Ala Val Glu
    320                 325                 330

TGG ATA TTT CCC ACA TGG GCA AGG GCG CAT GCG CTT GAC ACT GGT GAA 1117
Trp Ile Phe Pro Thr Trp Ala Arg Ala His Ala Leu Asp Thr Gly Glu
335                 340                 345                 350

ACA GTG AAC GTT TTG AAA GGG GCA ATA GCA GTT GCT GAT CGG ATA CTG 1165
Thr Val Asn Val Leu Lys Gly Ala Ile Ala Val Ala Asp Arg Ile Leu
                355                 360                 365

ACA GTT AGC CAG GGA TAC TCA TGG GAA ATA ACA ACT CCT GAA GGG GGA 1213
Thr Val Ser Gln Gly Tyr Ser Trp Glu Ile Thr Thr Pro Glu Gly Gly
            370                 375                 380

TAT GGG CTA CAT GAG CTG TTG AGC AGT AGA CAG TCT GTT CTT AAT GGA 1261
Tyr Gly Leu His Glu Leu Leu Ser Ser Arg Gln Ser Val Leu Asn Gly
        385                 390                 395

ATT ACT AAT GGA ATA GAT GTT AAT GAT TGG AAC CCG TCG ACA GAT GAG 1309
Ile Thr Asn Gly Ile Asp Val Asn Asp Trp Asn Pro Ser Thr Asp Glu
    400                 405                 410

CAT ATT GCT TCG CAT TAC TCC ATC AAT GAC CTC TCC GGA AAG GTT CAG 1357
His Ile Ala Ser His Tyr Ser Ile Asn Asp Leu Ser Gly Lys Val Gln
415                 420                 425                 430

TGC AAG ACT GAT CTG CAA AAG GAA CTG GGC CTT CCA ATT CGA CCT GAT 1405
Cys Lys Thr Asp Leu Gln Lys Glu Leu Gly Leu Pro Ile Arg Pro Asp
                435                 440                 445

TGT CCT CTG ATT GGA TTT ATT GGA AGG CTG GAC TAC CAG AAA GGT GTT 1453
Cys Pro Leu Ile Gly Phe Ile Gly Arg Leu Asp Tyr Gln Lys Gly Val
            450                 455                 460

GAC ATA ATC CTG TCA GCA ATT CCA GAA CTT ATG CAG AAT GAT GTC CAA 1501
Asp Ile Ile Leu Ser Ala Ile Pro Glu Leu Met Gln Asn Asp Val Gln
        465                 470                 475

GTT GTA ATG CTT GGA TCT GGT GAG AAA CAA TAT GAA GAC TGG ATG AGA 1549
Val Val Met Leu Gly Ser Gly Glu Lys Gln Tyr Glu Asp Trp Met Arg
    480                 485                 490

CAT ACA GAA AAT CTT TTT AAA GAC AAA TTT CGT GCT TGG GTT GGA TTT 1597
His Thr Glu Asn Leu Phe Lys Asp Lys Phe Arg Ala Trp Val Gly Phe
495                 500                 505                 510

AAT GTT CCA GTT TCT CAT AGG ATA ACA GCA GGA TGC GAC ATA CTA TTG 1645
Asn Val Pro Val Ser His Arg Ile Thr Ala Gly Cys Asp Ile Leu Leu
                515                 520                 525

ATG CCC TCA AGA TTC GAA CCG TGT GGC TTA AAC CAA TTG TAT GCA ATG 1693
Met Pro Ser Arg Phe Glu Pro Cys Gly Leu Asn Gln Leu Tyr Ala Met
            530                 535                 540

AGA TAT GGC ACC ATA CCT ATT GTT CAT AGC ACG GGG GGC CTA AGA GAC 1741
Arg Tyr Gly Thr Ile Pro Ile Val His Ser Thr Gly Gly Leu Arg Asp
        545                 550                 555

ACA GTG AAG GAT TTT AAT CCA TAT GCT CAA GAA GGA ATA GGT GAA GGT 1789
Thr Val Lys Asp Phe Asn Pro Tyr Ala Gln Glu Gly Ile Gly Glu Gly
    560                 565                 570

ACC GGG TGG ACA TTT TCT CCT CTA ACG AGT GAA AAG TTG CTT GAT ACA 1837
Thr Gly Trp Thr Phe Ser Pro Leu Thr Ser Glu Lys Leu Leu Asp Thr
575                 580                 585                 590

CTG AAG CTG GCA ATC GGG ACT TAT ACA GAA CAT AAG TCA TCT TGG GAG 1885
Leu Lys Leu Ala Ile Gly Thr Tyr Thr Glu His Lys Ser Ser Trp Glu
                595                 600                 605

GGA TTG ATG AGG AGA GGT ATG GGA AGG GAC TAT TCC TGG GAA AAT GCA 1933
Gly Leu Met Arg Arg Gly Met Gly Arg Asp Tyr Ser Trp Glu Asn Ala
            610                 615                 620

GCC ATT CAA TAT GAA CAA GTT TTC ACC TGG GCC TTT ATA GAT CCT CCA 1981
Ala Ile Gln Tyr Glu Gln Val Phe Thr Trp Ala Phe Ile Asp Pro Pro
        625                 630                 635

TAT GTC AGA TGATTTATCA AGAAAGATTG CAAACGGGAT ACATCATTAA 2030
Tyr Val Arg
    640

ACTATACGCG GAGCTTTTGG TGCTATTAGC TACTGTCATT GGGCGCGGAA TGTTTGTGGT 2090

TCTTTCTGAT TCAGAGAGAT CAAGTTAGTT CCAAAGACAT ACGTAGCCTG TCCCTGTCTG 2150

TGAGGGAGTA AAACTACAAA AGGCAATTAG AAACCACCAA GAACTGGCTC CTTTGGGAGA 2210

AGAGTGGAAA TATGTAAAAA AGAATTTTGA GTTTAATGTC AATTGATTAA TTGTTCTCAT 2270

TTTTAAAAAA AACATCTCAT CTCATACAAT ATATAAAATT GATCATGATT GATGAAAAAA 2330

AAAAAAAAAA AAAAAAAAAA AAAAAAAAAA 2360

(2) ANGABEN ZU SEQ ID NO: 10:

(i) SEQUENZKENNZEICHEN:
(A) LÄNGE: 641 Aminosäuren
(B) ART: Aminosäure
(D) TOPOLOGIE: linear

(ii) ART DES MOLEKÜLS: Protein
(xi) SEQUENZBESCHREIBUNG: SEQ ID NO: 10:

Met Gly Ser Leu Gln Thr Pro Thr Asn Leu Ser Asn Lys Ser Cys Leu
1               5                   10                  15

Cys Val Ser Gly Arg Val Val Lys Gly Leu Arg Val Glu Arg Gln Val
            20                  25                  30

Gly Leu Gly Phe Ser Trp Leu Leu Lys Gly Arg Arg Asn Arg Lys Val
        35                  40                  45

Gln Ser Leu Cys Val Thr Ser Ser Val Ser Asp Gly Ser Ser Ile Ala
    50                  55                  60

Glu Asn Lys Asn Val Ser Glu Gly Leu Leu Leu Gly Ala Glu Arg Asp
65                  70                  75                  80

Gly Ser Gly Ser Val Val Gly Phe Gln Leu Ile Pro His Ser Val Ala
                85                  90                  95

Gly Asp Ala Thr Met Val Glu Ser His Asp Ile Val Ala Asn Asp Arg
            100                 105                 110

Asp Asp Leu Ser Glu Asp Thr Glu Glu Met Glu Glu Thr Pro Ile Lys
        115                 120                 125

Leu Thr Phe Asn Ile Ile Phe Val Thr Ala Glu Ala Ala Pro Tyr Ser
    130                 135                 140

Lys Thr Gly Gly Leu Gly Asp Val Cys Gly Ser Leu Pro Met Ala Leu
145                 150                 155                 160

Ala Ala Arg Gly His Arg Val Met Val Val Ser Pro Arg Tyr Leu Asn
                165                 170                 175

Gly Gly Pro Ser Asp Glu Lys Tyr Ala Asn Ala Val Asp Leu Asp Val
            180                 185                 190

Arg Ala Thr Val His Cys Phe Gly Asp Ala Gln Glu Val Ala Phe Tyr
        195                 200                 205

His Glu Tyr Arg Ala Gly Val Asp Trp Val Phe Val Asp His Ser Ser
    210                 215                 220

Tyr Cys Arg Pro Gly Thr Pro Tyr Gly Asp Ile Tyr Gly Ala Phe Gly
225                 230                 235                 240

Asp Asn Gln Phe Arg Phe Thr Leu Leu Ser His Ala Ala Cys Glu Ala
                245                 250                 255

Pro Leu Val Leu Pro Leu Gly Gly Phe Thr Tyr Gly Glu Lys Cys Leu
            260                 265                 270

Phe Leu Ala Asn Asp Trp His Ala Ala Leu Val Pro Leu Leu Leu Ala
        275                 280                 285

Ala Lys Tyr Arg Pro Tyr Gly Val Tyr Lys Asp Ala Arg Ser Ile Val
    290                 295                 300

Ala Ile His Asn Ile Ala His Gln Gly Val Glu Pro Ala Val Thr Tyr
305                 310                 315                 320

Asn Asn Leu Gly Leu Pro Pro Gln Trp Tyr Gly Ala Val Glu Trp Ile
                325                 330                 335

Phe Pro Thr Trp Ala Arg Ala His Ala Leu Asp Thr Gly Glu Thr Val
            340                 345                 350

Asn Val Leu Lys Gly Ala Ile Ala Val Ala Asp Arg Ile Leu Thr Val
        355                 360                 365

Ser Gln Gly Tyr Ser Trp Glu Ile Thr Thr Pro Glu Gly Gly Tyr Gly
    370                 375                 380

Leu His Glu Leu Leu Ser Ser Arg Gln Ser Val Leu Asn Gly Ile Thr
385                 390                 395                 400

Asn Gly Ile Asp Val Asn Asp Trp Asn Pro Ser Thr Asp Glu His Ile
                405                 410                 415

Ala Ser His Tyr Ser Ile Asn Asp Leu Ser Gly Lys Val Gln Cys Lys
            420                 425                 430

Thr Asp Leu Gln Lys Glu Leu Gly Leu Pro Ile Arg Pro Asp Cys Pro
        435                 440                 445

Leu Ile Gly Phe Ile Gly Arg Leu Asp Tyr Gln Lys Gly Val Asp Ile
    450                 455                 460

Ile Leu Ser Ala Ile Pro Glu Leu Met Gln Asn Asp Val Gln Val Val
465                 470                 475                 480

Met Leu Gly Ser Gly Glu Lys Gln Tyr Glu Asp Trp Met Arg His Thr
                485                 490                 495

Glu Asn Leu Phe Lys Asp Lys Phe Arg Ala Trp Val Gly Phe Asn Val
            500                 505                 510

Pro Val Ser His Arg Ile Thr Ala Gly Cys Asp Ile Leu Leu Met Pro
        515                 520                 525

Ser Arg Phe Glu Pro Cys Gly Leu Asn Gln Leu Tyr Ala Met Arg Tyr
    530                 535                 540

Gly Thr Ile Pro Ile Val His Ser Thr Gly Gly Leu Arg Asp Thr Val
545                 550                 555                 560

Lys Asp Phe Asn Pro Tyr Ala Gln Glu Gly Ile Gly Glu Gly Thr Gly
                565                 570                 575

Trp Thr Phe Ser Pro Leu Thr Ser Glu Lys Leu Leu Asp Thr Leu Lys
            580                 585                 590

Leu Ala Ile Gly Thr Tyr Thr Glu His Lys Ser Ser Trp Glu Gly Leu
        595                 600                 605

Met Arg Arg Gly Met Gly Arg Asp Tyr Ser Trp Glu Asn Ala Ala Ile
    610                 615                 620

Gln Tyr Glu Gln Val Phe Thr Trp Ala Phe Ile Asp Pro Pro Tyr Val
625                 630                 635                 640

Arg

(2) ANGABEN ZU SEQ ID NO: 11:

    (i) SEQUENZKENNZEICHEN:
        (A) LÄNGE: 4168 Basenpaare
        (B) ART: Nucleotid
        (C) STRANGFORM: Einzelstrang
        (D) TOPOLOGIE: linear

    (ii) ART DES MOLEKÜLS: cDNA zum RNA

    (vi) URSPRÜNLICHE HERKUNFT:
        (A) ORGANISMUS: Solanum tuberosum
        (B) STAMM: cv. Désirée
        (F) GEWEBETYP: Blattgewebe

(vii) UNMITTELBARE HERKUNFT:
(A) BIBLIOTHEK: cDNA-Bibliothek in Lambda ZAPII

(ix) MERKMAL:
(A) NAME/SCHLÜSSEL: CDS
(B) LAGE:307..3897

(xi) SEQUENZBESCHREIBUNG: SEQ ID NO: 11:

TTTTTTAATA GATTTTTAAA ACCCCATTAA AGCAAATACG TATATAATTG CAGCACAGAT 60

ACAGAGAGGG AGAGAGAAAG ATAGTGTGTT GATGAAGGAG AAGAGAGATA TTTCACATGG 120

GATGTTCTAT TTGATTCTGT GGTGAACAAG AGTTTTACAA AGAACATTCC TTTTTCTTTT 180

TTTCTTGGTT CTTGTGTGGG TCAGCCATGG ATGTTCCATT TCCACTGCAT AGACCATTGA 240

GTTGCACAAG TGTCTCCAAT GCAATAACCC ACCTCAAGAT CAAACCTTTT CTTGGGTTTG 300

TCTCTC ATG GAA CCA CAA GTC TAT CAG TAC AAT CTT CTT CAT GGA GGA 348
Met Glu Pro Gln Val Tyr Gln Tyr Asn Leu Leu His Gly Gly
1               5                   10

AGG ATG GAA ATG GTT ACT GGG GTT TCA TTT CCA TTT TGT GCA AAT CTC 396
Arg Met Glu Met Val Thr Gly Val Ser Phe Pro Phe Cys Ala Asn Leu
15                  20                  25                  30

TCG GGA AGA AGA CGG AGA AAA GTT TCA ACT ACT AGG AGT CAA GGA TCT 444
Ser Gly Arg Arg Arg Arg Lys Val Ser Thr Thr Arg Ser Gln Gly Ser
                35                  40                  45

TCA CCT AAG GGG TTT GTG CCA AGG AAG CCC TCA GGG ATG AGC ACG CAA 492
Ser Pro Lys Gly Phe Val Pro Arg Lys Pro Ser Gly Met Ser Thr Gln
            50                  55                  60

AGA AAG GTT CAG AAG AGC AAT GGT GAT AAA GAA AGT CAA AGT ACT TCA 540
Arg Lys Val Gln Lys Ser Asn Gly Asp Lys Glu Ser Gln Ser Thr Ser
        65                  70                  75

ACA TCT AAA GAA TCT GAA ATT TCC AAC CAG AAG ACG GTT GAA GCA AGA 588
Thr Ser Lys Glu Ser Glu Ile Ser Asn Gln Lys Thr Val Glu Ala Arg
    80                  85                  90

GTT GAA ACT AGT GAC GAT GAC ACT AAA GTA GTG GTG AGG GAC CAC AAG 636
Val Glu Thr Ser Asp Asp Asp Thr Lys Val Val Val Arg Asp His Lys
95                  100                 105                 110

TTT CTG GAG GAT GAG GAT GAA ATC AAT GGT TCT ACT AAA TCA ATA AGT 684
Phe Leu Glu Asp Glu Asp Glu Ile Asn Gly Ser Thr Lys Ser Ile Ser
                115                 120                 125

ATG TCA CCT GTT CGT GTA TCA TCT CAA TTT GTT GAA AGT GAA GAA ACT 732
Met Ser Pro Val Arg Val Ser Ser Gln Phe Val Glu Ser Glu Glu Thr
            130                 135                 140

GGT GGT GAT GAC AAG GAT GCT GTA AAG TTA AAC AAA TCA AAG AGA TCG 780
Gly Gly Asp Asp Lys Asp Ala Val Lys Leu Asn Lys Ser Lys Arg Ser
        145                 150                 155

GAA GAG AGT GAT TTT CTA ATT GAT TCT GTA ATA AGA GAA CAA AGT GGA 828
Glu Glu Ser Asp Phe Leu Ile Asp Ser Val Ile Arg Glu Gln Ser Gly
    160                 165                 170

TCT CAG GGG GAA ACT AAT GCC AGT AGC AAG GGA AGC CAT GCT GTG GGT 876
Ser Gln Gly Glu Thr Asn Ala Ser Ser Lys Gly Ser His Ala Val Gly
175                 180                 185                 190

ACA AAA CTT TAT GAG ATA TTG CAG GTG GAT GTT GAG CCA CAA CAA TTG 924
Thr Lys Leu Tyr Glu Ile Leu Gln Val Asp Val Glu Pro Gln Gln Leu
                195                 200                 205

AAA GAA AAT AAT GCT GGG AAT GTT GAA TAC AAA GGA CCT GTA GCA AGT 972
Lys Glu Asn Asn Ala Gly Asn Val Glu Tyr Lys Gly Pro Val Ala Ser
            210                 215                 220

AAG CTA TTG GAA ATT ACT AAG GCT AGT GAT GTG GAA CAC ACT GAA AGC 1020
Lys Leu Leu Glu Ile Thr Lys Ala Ser Asp Val Glu His Thr Glu Ser
        225                 230                 235

AAT GAG ATT GAT GAC TTA GAC ACT AAT AGT TTC TTT AAA TCA GAT TTA 1068
Asn Glu Ile Asp Asp Leu Asp Thr Asn Ser Phe Phe Lys Ser Asp Leu
    240                 245                 250

ATT GAA GAG GAT GAG CCA TTA GCT GCA GGA ACA GTG GAG ACT GGA GAT 1116
Ile Glu Glu Asp Glu Pro Leu Ala Ala Gly Thr Val Glu Thr Gly Asp
255                 260                 265                 270

TCT TCT CTA AAC TTA AGA TTG GAG ATG GAA GCA AAT CTA CGT AGG CAG 1164
Ser Ser Leu Asn Leu Arg Leu Glu Met Glu Ala Asn Leu Arg Arg Gln
                275                 280                 285

GCT ATA GAA AGG CTT GCC GAG GAA AAT TTA TTG CAA GGG ATC AGA TTA 1212
Ala Ile Glu Arg Leu Ala Glu Glu Asn Leu Leu Gln Gly Ile Arg Leu
            290                 295                 300

TTT TGT TTT CCA GAG GTT GTA AAA CCT GAT GAA GAT GTC GAG ATA TTT 1260
Phe Cys Phe Pro Glu Val Val Lys Pro Asp Glu Asp Val Glu Ile Phe
        305                 310                 315

CTT AAC AGA GGT CTT TCC ACT TTG AAG AAT GAG TCT GAT GTC TTG ATT 1308
Leu Asn Arg Gly Leu Ser Thr Leu Lys Asn Glu Ser Asp Val Leu Ile
    320                 325                 330

ATG GGA GCT TTT AAT GAG TGG CGC TAT AGG TCT TTT ACT ACA AGG CTA 1356
Met Gly Ala Phe Asn Glu Trp Arg Tyr Arg Ser Phe Thr Thr Arg Leu
335                 340                 345                 350

ACT GAG ACT CAT CTC AAT GGA GAT TGG TGG TCT TGC AAG ATC CAT GTT 1404
Thr Glu Thr His Leu Asn Gly Asp Trp Trp Ser Cys Lys Ile His Val
                355                 360                 365

CCC AAG GAA GCA TAC AGG GCT GAT TTT GTG TTT TTT AAT GGA CAA GAT 1452
Pro Lys Glu Ala Tyr Arg Ala Asp Phe Val Phe Phe Asn Gly Gln Asp
            370                 375                 380

GTC TAT GAC AAC AAT GAT GGA AAT GAC TTC AGT ATA ACT GTG AAA GGT 1500
Val Tyr Asp Asn Asn Asp Gly Asn Asp Phe Ser Ile Thr Val Lys Gly
        385                 390                 395

GGT ATG CAA ATC ATT GAC TTT GAA AAT TTC TTG CTT GAG GAG AAA TGG 1548
Gly Met Gln Ile Ile Asp Phe Glu Asn Phe Leu Leu Glu Glu Lys Trp
    400                 405                 410

AGA GAA CAG GAG AAA CTT GCT AAA GAA CAA GCT GAA AGA GAA AGA CTA 1596
Arg Glu Gln Glu Lys Leu Ala Lys Glu Gln Ala Glu Arg Glu Arg Leu
415                 420                 425                 430

GCG GAA GAA CAA AGA CGA ATA GAA GCA GAG AAA GCT GAA ATT GAA GCT 1644
Ala Glu Glu Gln Arg Arg Ile Glu Ala Glu Lys Ala Glu Ile Glu Ala
                435                 440                 445

GAC AGA GCA CAA GCA AAG GAA GAG GCT GCA AAG AAA AAG AAA GTA TTG 1692
Asp Arg Ala Gln Ala Lys Glu Glu Ala Ala Lys Lys Lys Lys Val Leu
            450                 455                 460

CGA GAA TTG ATG GTA AAA GCC ACG AAG ACT CGT GAT ATC ACG TGG TAC 1740
Arg Glu Leu Met Val Lys Ala Thr Lys Thr Arg Asp Ile Thr Trp Tyr
        465                 470                 475

ATA GAG CCA AGT GAA TTT AAA TGC GAG GAC AAG GTC AGG TTA TAC TAT 1788
Ile Glu Pro Ser Glu Phe Lys Cys Glu Asp Lys Val Arg Leu Tyr Tyr
    480                 485                 490

AAC AAA AGT TCA GGT CCT CTC TCC CAT GCT AAG GAC TTG TGG ATC CAC 1836
Asn Lys Ser Ser Gly Pro Leu Ser His Ala Lys Asp Leu Trp Ile His
495                 500                 505                 510

GGA GGA TAT AAT AAT TGG AAG GAT GGT TTG TCT ATT GTC AAA AAG CTT 1884
Gly Gly Tyr Asn Asn Trp Lys Asp Gly Leu Ser Ile Val Lys Lys Leu
                515                 520                 525

GTT AAA TCT GAG AGA ATA GAT GGT GAT TGG TGG TAT ACA GAG GTT GTT 1932
Val Lys Ser Glu Arg Ile Asp Gly Asp Trp Trp Tyr Thr Glu Val Val
            530                 535                 540

ATT CCT GAT CAG GCA CTT TTC TTG GAT TGG GTT TTT GCT GAT GGT CCA 1980
Ile Pro Asp Gln Ala Leu Phe Leu Asp Trp Val Phe Ala Asp Gly Pro
        545                 550                 555

CCC AAG CAT GCC ATT GCT TAT GAT AAC AAT CAC CGC CAA GAC TTC CAT 2028
Pro Lys His Ala Ile Ala Tyr Asp Asn Asn His Arg Gln Asp Phe His
    560                 565                 570

GCC ATT GTC CCC AAC CAC ATT CCG GAG GAA TTA TAT TGG GTT GAG GAA 2076
Ala Ile Val Pro Asn His Ile Pro Glu Glu Leu Tyr Trp Val Glu Glu
575                 580                 585                 590

GAA CAT CAG ATC TTT AAG ACA CTT CAG GAG GAG AGA AGG CTT AGA GAA 2124
Glu His Gln Ile Phe Lys Thr Leu Gln Glu Glu Arg Arg Leu Arg Glu
                595                 600                 605

GCG GCT ATG CGT GCT AAG GTT GAA AAA ACA GCA CTT CTG AAA ACT GAA 2172
Ala Ala Met Arg Ala Lys Val Glu Lys Thr Ala Leu Leu Lys Thr Glu
            610                 615                 620

ACA AAG GAA AGA ACT ATG AAA TCA TTT TTA CTG TCT CAG AAG CAT GTA 2220
Thr Lys Glu Arg Thr Met Lys Ser Phe Leu Leu Ser Gln Lys His Val
        625                 630                 635

GTA TAT ACT GAG CCT CTT GAT ATC CAA GCT GGA AGC AGC GTC ACA GTT 2268
Val Tyr Thr Glu Pro Leu Asp Ile Gln Ala Gly Ser Ser Val Thr Val
    640                 645                 650

TAC TAT AAT CCC GCC AAT ACA GTA CTT AAT GGT AAA CCT GAA ATT TGG 2316
Tyr Tyr Asn Pro Ala Asn Thr Val Leu Asn Gly Lys Pro Glu Ile Trp
655                 660                 665                 670

TTC AGA TGT TCA TTT AAT CGC TGG ACT CAC CGC CTG GGT CCA TTG CCA 2364
Phe Arg Cys Ser Phe Asn Arg Trp Thr His Arg Leu Gly Pro Leu Pro
                675                 680                 685

CCT CAG AAA ATG TCG CCT GCT GAA AAT GGC ACC CAT GTC AGA GCA ACT 2412
Pro Gln Lys Met Ser Pro Ala Glu Asn Gly Thr His Val Arg Ala Thr
            690                 695                 700

GTG AAG GTT CCA TTG GAT GCA TAT ATG ATG GAT TTT GTA TTT TCC GAG 2460
Val Lys Val Pro Leu Asp Ala Tyr Met Met Asp Phe Val Phe Ser Glu
        705                 710                 715

AGA GAA GAT GGT GGG ATT TTT GAC AAT AAG AGC GGA ATG GAC TAT CAC 2508
Arg Glu Asp Gly Gly Ile Phe Asp Asn Lys Ser Gly Met Asp Tyr His
    720                 725                 730

ATA CCT GTG TTT GGA GGA GTC GCT AAA GAA CCT CCA ATG CAT ATT GTC 2556
Ile Pro Val Phe Gly Gly Val Ala Lys Glu Pro Pro Met His Ile Val
735                 740                 745                 750

CAT ATT GCT GTC GAA ATG GCA CCA ATT GCA AAG GTG GGA GGC CTT GGT 2604
His Ile Ala Val Glu Met Ala Pro Ile Ala Lys Val Gly Gly Leu Gly
                755                 760                 765

GAT GTT GTT ACT AGT CTT TCC CGT GCT GTT CAA GAT TTA AAC CAT AAT 2652
Asp Val Val Thr Ser Leu Ser Arg Ala Val Gln Asp Leu Asn His Asn
            770                 775                 780

GTG GAT ATT ATC TTA CCT AAG TAT GAC TGT TTG AAG ATG AAT AAT GTG 2700
Val Asp Ile Ile Leu Pro Lys Tyr Asp Cys Leu Lys Met Asn Asn Val
        785                 790                 795

AAG GAC TTT CGG TTT CAC AAA AAC TAC TTT TGG GGT GGG ACT GAA ATA 2748
Lys Asp Phe Arg Phe His Lys Asn Tyr Phe Trp Gly Gly Thr Glu Ile
    800                 805                 810

AAA GTA TGG TTT GGA AAG GTG GAA GGT CTC TCG GTC TAT TTT TTG GAG 2796
Lys Val Trp Phe Gly Lys Val Glu Gly Leu Ser Val Tyr Phe Leu Glu
815                 820                 825                 830

CCT CAA AAC GGG TTA TTT TCG AAA GGG TGC GTC TAT GGT TGT AGC AAT 2844
Pro Gln Asn Gly Leu Phe Ser Lys Gly Cys Val Tyr Gly Cys Ser Asn
                835                 840                 845

GAT GGT GAA CGA TTT GGT TTC TTC TGT CAC GCG GCT TTG GAG TTT CTT 2892
Asp Gly Glu Arg Phe Gly Phe Phe Cys His Ala Ala Leu Glu Phe Leu
            850                 855                 860

CTG CAA GGT GGA TTT AGT CCG GAT ATC ATT CAT TGC CAT GAT TGG TCT 2940
Leu Gln Gly Gly Phe Ser Pro Asp Ile Ile His Cys His Asp Trp Ser
        865                 870                 875

AGT GCT CCT GTT GCT TGG CTC TTT AAG GAA CAA TAT ACA CAC TAT GGT 2988
Ser Ala Pro Val Ala Trp Leu Phe Lys Glu Gln Tyr Thr His Tyr Gly
    880                 885                 890

CTA AGC AAA TCT CGT ATA GTC TTC ACG ATA CAT AAT CTT GAA TTT GGG 3036
Leu Ser Lys Ser Arg Ile Val Phe Thr Ile His Asn Leu Glu Phe Gly
895                 900                 905                 910

GCA GAT CTC ATT GGG AGA GCA ATG ACT AAC GCA GAC AAA GCT ACA ACA 3084
Ala Asp Leu Ile Gly Arg Ala Met Thr Asn Ala Asp Lys Ala Thr Thr
                915                 920                 925

GTT TCA CCA ACT TAC TCA CAG GAG GTG TCT GGA AAC CCT GTA ATT GCG 3132
Val Ser Pro Thr Tyr Ser Gln Glu Val Ser Gly Asn Pro Val Ile Ala
            930                 935                 940

CCT CAC CTT CAC AAG TTC CAT GGT ATA GTG AAT GGG ATT GAC CCA GAT 3180
Pro His Leu His Lys Phe His Gly Ile Val Asn Gly Ile Asp Pro Asp
        945                 950                 955

ATT TGG GAT CCT TTA AAC GAT AAG TTC ATT CCG ATT CCG TAC ACC TCA 3228
Ile Trp Asp Pro Leu Asn Asp Lys Phe Ile Pro Ile Pro Tyr Thr Ser
    960                 965                 970

GAA AAC GTT GTT GAA GGC AAA ACA GCA GCC AAG GAA GCT TTG CAG CGA 3276
Glu Asn Val Val Glu Gly Lys Thr Ala Ala Lys Glu Ala Leu Gln Arg
975                 980                 985                 990

AAA CTT GGA CTG AAA CAG GCT GAC CTT CCT TTG GTA GGA ATT ATC ACC 3324
Lys Leu Gly Leu Lys Gln Ala Asp Leu Pro Leu Val Gly Ile Ile Thr
                995                 1000                1005

CGC TTA ACT CAC CAG AAA GGA ATC CAC CTC ATT AAA CAT GCT ATT TGG 3372
Arg Leu Thr His Gln Lys Gly Ile His Leu Ile Lys His Ala Ile Trp
            1010                1015                1020

CGC ACC TTG GAA CGG AAC GGA CAG GTA GTC TTG CTT GGT TCT GCT CCT 3420
Arg Thr Leu Glu Arg Asn Gly Gln Val Val Leu Leu Gly Ser Ala Pro
        1025                1030                1035

GAT CCT AGG GTA CAA AAC GAT TTT GTT AAT TTG GCA AAT CAA TTG CAC 3468
Asp Pro Arg Val Gln Asn Asp Phe Val Asn Leu Ala Asn Gln Leu His
    1040                1045                1050

TCC AAA TAT AAT GAC CGC GCA CGA CTC TGT CTA ACA TAT GAC GAG CCA 3516
Ser Lys Tyr Asn Asp Arg Ala Arg Leu Cys Leu Thr Tyr Asp Glu Pro
1055                1060                1065                1070

CTT TCT CAC CTG ATA TAT GCT GGT GCT GAT TTT ATT CTA GTT CCT TCA 3564
Leu Ser His Leu Ile Tyr Ala Gly Ala Asp Phe Ile Leu Val Pro Ser
                1075                1080                1085

ATA TTT GAG CCA TGT GGA CTA ACA CAA CTT ACC GCT ATG AGA TAT GGT 3612
Ile Phe Glu Pro Cys Gly Leu Thr Gln Leu Thr Ala Met Arg Tyr Gly
            1090                1095                1100

TCA ATT CCA GTC GTG CGT AAA ACT GGA GGA CTT TAT GAT ACT GTA TTT 3660
Ser Ile Pro Val Val Arg Lys Thr Gly Gly Leu Tyr Asp Thr Val Phe
        1105                1110                1115

GAT GTT GAC CAT GAC AAA GAG AGA GCA CAA CAG TGT GGT CTT GAA CCA 3708
Asp Val Asp His Asp Lys Glu Arg Ala Gln Gln Cys Gly Leu Glu Pro
    1120                1125                1130

AAT GGA TTC AGC TTT GAT GGA GCA GAT GCT GGC GGA GTT GAT TAT GCT 3756
Asn Gly Phe Ser Phe Asp Gly Ala Asp Ala Gly Gly Val Asp Tyr Ala
1135                1140                1145                1150

CTG AAT AGA GCT CTC TCT GCT TGG TAC GAT GGT CGG GAT TGG TTC AAC 3804
Leu Asn Arg Ala Leu Ser Ala Trp Tyr Asp Gly Arg Asp Trp Phe Asn
                1155                1160                1165

TCT TTA TGC AAG CAG GTC ATG GAA CAA GAT TGG TCT TGG AAC CGA CCT 3852
Ser Leu Cys Lys Gln Val Met Glu Gln Asp Trp Ser Trp Asn Arg Pro
            1170                1175                1180

GCT CTT GAT TAT TTG GAG CTT TAC CAT GCT GCT AGA AAG TTA GAA 3897
Ala Leu Asp Tyr Leu Glu Leu Tyr His Ala Ala Arg Lys Leu Glu
        1185                1190                1195

TAGTTAGTTT GTGAGATGCT AGCAGAAAAA TTCACGAGAT CTGCAATCTG TACAGGTTCA 3957

GTGTTTGCGT CTGGACAGCT TTTTTATTTC CTATATCAAA GTATAAATCA AGTCTACACT 4017

GAGATCAATA GCAGACAGTC CTCAGTTCAT TTCATTTTTT GTGCAACATA TGAAAGAGCT 4077

TAGCCTCTAA TAATGTAGTC ATTGATGATT ATTTGTTTTG GGAAGAAATG AGAAATCAAA 4137

GGATGCAAAA TACTCTGAAA AAAAAAAAAA A 4168

(2) ANGABEN ZU SEQ ID NO: 12:

(i) SEQUENZKENNZEICHEN:
(A) LÄNGE: 1197 Aminosäuren
(B) ART: Aminosäure
(D) TOPOLOGIE: linear

(ii) ART DES MOLEKÜLS: Protein
(xi) SEQUENZBESCHREIBUNG: SEQ ID NO: 12:

Met Glu Pro Gln Val Tyr Gln Tyr Asn Leu Leu His Gly Gly Arg Met
1               5                   10                  15

Glu Met Val Thr Gly Val Ser Phe Pro Phe Cys Ala Asn Leu Ser Gly
            20                  25                  30

Arg Arg Arg Arg Lys Val Ser Thr Thr Arg Ser Gln Gly Ser Ser Pro
        35                  40                  45

Lys Gly Phe Val Pro Arg Lys Pro Ser Gly Met Ser Thr Gln Arg Lys
    50                  55                  60

Val Gln Lys Ser Asn Gly Asp Lys Glu Ser Gln Ser Thr Ser Thr Ser
65                  70                  75                  80

Lys Glu Ser Glu Ile Ser Asn Gln Lys Thr Val Glu Ala Arg Val Glu
                85                  90                  95

Thr Ser Asp Asp Asp Thr Lys Val Val Val Arg Asp His Lys Phe Leu
            100                 105                 110

Glu Asp Glu Asp Glu Ile Asn Gly Ser Thr Lys Ser Ile Ser Met Ser
        115                 120                 125

Pro Val Arg Val Ser Ser Gln Phe Val Glu Ser Glu Glu Thr Gly Gly
    130                 135                 140

Asp Asp Lys Asp Ala Val Lys Leu Asn Lys Ser Lys Arg Ser Glu Glu
145                 150                 155                 160

Ser Asp Phe Leu Ile Asp Ser Val Ile Arg Glu Gln Ser Gly Ser Gln
                165                 170                 175

Gly Glu Thr Asn Ala Ser Ser Lys Gly Ser His Ala Val Gly Thr Lys
            180                 185                 190

Leu Tyr Glu Ile Leu Gln Val Asp Val Glu Pro Gln Gln Leu Lys Glu
        195                 200                 205

Asn Asn Ala Gly Asn Val Glu Tyr Lys Gly Pro Val Ala Ser Lys Leu
    210                 215                 220

Leu Glu Ile Thr Lys Ala Ser Asp Val Glu His Thr Glu Ser Asn Glu
225                 230                 235                 240

Ile Asp Asp Leu Asp Thr Asn Ser Phe Phe Lys Ser Asp Leu Ile Glu
                245                 250                 255

Glu Asp Glu Pro Leu Ala Ala Gly Thr Val Glu Thr Gly Asp Ser Ser
            260                 265                 270

Leu Asn Leu Arg Leu Glu Met Glu Ala Asn Leu Arg Arg Gln Ala Ile
        275                 280                 285

Glu Arg Leu Ala Glu Glu Asn Leu Leu Gln Gly Ile Arg Leu Phe Cys
    290                 295                 300

Phe Pro Glu Val Val Lys Pro Asp Glu Asp Val Glu Ile Phe Leu Asn
305                 310                 315                 320

Arg Gly Leu Ser Thr Leu Lys Asn Glu Ser Asp Val Leu Ile Met Gly
                325                 330                 335

Ala Phe Asn Glu Trp Arg Tyr Arg Ser Phe Thr Thr Arg Leu Thr Glu
            340                 345                 350

Thr His Leu Asn Gly Asp Trp Trp Ser Cys Lys Ile His Val Pro Lys
        355                 360                 365

Glu Ala Tyr Arg Ala Asp Phe Val Phe Phe Asn Gly Gln Asp Val Tyr
    370                 375                 380

Asp Asn Asn Asp Gly Asn Asp Phe Ser Ile Thr Val Lys Gly Gly Met
385                 390                 395                 400

Gln Ile Ile Asp Phe Glu Asn Phe Leu Leu Glu Glu Lys Trp Arg Glu
                405                 410                 415

Gln Glu Lys Leu Ala Lys Glu Gln Ala Glu Arg Glu Arg Leu Ala Glu
            420                 425                 430

Glu Gln Arg Arg Ile Glu Ala Glu Lys Ala Glu Ile Glu Ala Asp Arg
        435                 440                 445

Ala Gln Ala Lys Glu Glu Ala Ala Lys Lys Lys Lys Val Leu Arg Glu
    450                 455                 460

Leu Met Val Lys Ala Thr Lys Thr Arg Asp Ile Thr Trp Tyr Ile Glu
465                 470                 475                 480

Pro Ser Glu Phe Lys Cys Glu Asp Lys Val Arg Leu Tyr Tyr Asn Lys
                485                 490                 495

Ser Ser Gly Pro Leu Ser His Ala Lys Asp Leu Trp Ile His Gly Gly
            500                 505                 510

Tyr Asn Asn Trp Lys Asp Gly Leu Ser Ile Val Lys Lys Leu Val Lys
        515                 520                 525

Ser Glu Arg Ile Asp Gly Asp Trp Trp Tyr Thr Glu Val Val Ile Pro
    530                 535                 540

Asp Gln Ala Leu Phe Leu Asp Trp Val Phe Ala Asp Gly Pro Pro Lys
545                 550                 555                 560

His Ala Ile Ala Tyr Asp Asn Asn His Arg Gln Asp Phe His Ala Ile
                565                 570                 575

Val Pro Asn His Ile Pro Glu Glu Leu Tyr Trp Val Glu Glu Glu His
            580                 585                 590

Gln Ile Phe Lys Thr Leu Gln Glu Glu Arg Arg Leu Arg Glu Ala Ala
        595                 600                 605

Met Arg Ala Lys Val Glu Lys Thr Ala Leu Leu Lys Thr Glu Thr Lys
    610                 615                 620

Glu Arg Thr Met Lys Ser Phe Leu Leu Ser Gln Lys His Val Val Tyr
625                 630                 635                 640

Thr Glu Pro Leu Asp Ile Gln Ala Gly Ser Ser Val Thr Val Tyr Tyr
                645                 650                 655

Asn Pro Ala Asn Thr Val Leu Asn Gly Lys Pro Glu Ile Trp Phe Arg
            660                 665                 670

Cys Ser Phe Asn Arg Trp Thr His Arg Leu Gly Pro Leu Pro Pro Gln
        675                 680                 685

Lys Met Ser Pro Ala Glu Asn Gly Thr His Val Arg Ala Thr Val Lys
    690                 695                 700

Val Pro Leu Asp Ala Tyr Met Met Asp Phe Val Phe Ser Glu Arg Glu
705                 710                 715                 720

Asp Gly Gly Ile Phe Asp Asn Lys Ser Gly Met Asp Tyr His Ile Pro
                725                 730                 735

Val Phe Gly Gly Val Ala Lys Glu Pro Pro Met His Ile Val His Ile
            740                 745                 750

Ala Val Glu Met Ala Pro Ile Ala Lys Val Gly Gly Leu Gly Asp Val
        755                 760                 765

Val Thr Ser Leu Ser Arg Ala Val Gln Asp Leu Asn His Asn Val Asp
    770                 775                 780

Ile Ile Leu Pro Lys Tyr Asp Cys Leu Lys Met Asn Asn Val Lys Asp
785                 790                 795                 800

Phe Arg Phe His Lys Asn Tyr Phe Trp Gly Gly Thr Glu Ile Lys Val
                805                 810                 815

Trp Phe Gly Lys Val Glu Gly Leu Ser Val Tyr Phe Leu Glu Pro Gln
            820                 825                 830

Asn Gly Leu Phe Ser Lys Gly Cys Val Tyr Gly Cys Ser Asn Asp Gly
        835                 840                 845

Glu Arg Phe Gly Phe Phe Cys His Ala Ala Leu Glu Phe Leu Leu Gln
    850                 855                 860

Gly Gly Phe Ser Pro Asp Ile Ile His Cys His Asp Trp Ser Ser Ala
865                 870                 875                 880

Pro Val Ala Trp Leu Phe Lys Glu Gln Tyr Thr His Tyr Gly Leu Ser
                885                 890                 895

Lys Ser Arg Ile Val Phe Thr Ile His Asn Leu Glu Phe Gly Ala Asp
            900                 905                 910

Leu Ile Gly Arg Ala Met Thr Asn Ala Asp Lys Ala Thr Thr Val Ser
        915                 920                 925

Pro Thr Tyr Ser Gln Glu Val Ser Gly Asn Pro Val Ile Ala Pro His
    930                 935                 940

Leu His Lys Phe His Gly Ile Val Asn Gly Ile Asp Pro Asp Ile Trp
945                 950                 955                 960

Asp Pro Leu Asn Asp Lys Phe Ile Pro Ile Pro Tyr Thr Ser Glu Asn
                965                 970                 975

Val Val Glu Gly Lys Thr Ala Ala Lys Glu Ala Leu Gln Arg Lys Leu
            980                 985                 990

Gly Leu Lys Gln Ala Asp Leu Pro Leu Val Gly Ile Ile Thr Arg Leu
        995                 1000                1005

Thr His Gln Lys Gly Ile His Leu Ile Lys His Ala Ile Trp Arg Thr
    1010                1015                1020

Leu Glu Arg Asn Gly Gln Val Val Leu Leu Gly Ser Ala Pro Asp Pro
1025                1030                1035                1040

Arg Val Gln Asn Asp Phe Val Asn Leu Ala Asn Gln Leu His Ser Lys
                1045                1050                1055

Tyr Asn Asp Arg Ala Arg Leu Cys Leu Thr Tyr Asp Glu Pro Leu Ser
            1060                1065                1070

His Leu Ile Tyr Ala Gly Ala Asp Phe Ile Leu Val Pro Ser Ile Phe
        1075                1080                1085

Glu Pro Cys Gly Leu Thr Gln Leu Thr Ala Met Arg Tyr Gly Ser Ile
    1090                1095                1100

Pro Val Val Arg Lys Thr Gly Gly Leu Tyr Asp Thr Val Phe Asp Val
1105                1110                1115                1120

Asp His Asp Lys Glu Arg Ala Gln Gln Cys Gly Leu Glu Pro Asn Gly
                1125                1130                1135

Phe Ser Phe Asp Gly Ala Asp Ala Gly Gly Val Asp Tyr Ala Leu Asn
            1140                1145                1150

Arg Ala Leu Ser Ala Trp Tyr Asp Gly Arg Asp Trp Phe Asn Ser Leu
        1155                1160                1165

Cys Lys Gln Val Met Glu Gln Asp Trp Ser Trp Asn Arg Pro Ala Leu
    1170                1175                1180

Asp Tyr Leu Glu Leu Tyr His Ala Ala Arg Lys Leu Glu
1185                1190                1195

(2) ANGABEN ZU SEQ ID NO: 13:

(i) SEQUENZKENNZEICHEN:
(A) LÄNGE: 12 Aminosäuren
(B) ART: Aminosäure
(C) STRANGFORM: Einzelstrang
(D) TOPOLOGIE: linear

(ii) ART DES MOLEKÜLS: Peptid

(xi) SEQUENZBESCHREIBUNG: SEQ ID NO: 13:

Gly Thr Gly Gly Leu Arg Asp Thr Val Glu Asn Cys
1               5                   10

(2) ANGABEN ZU SEQ ID NO: 14:

(i) SEQUENZKENNZEICHEN:
(A) LÄNGE: 30 Basenpaare
(B) ART: Nucleotid
(C) STRANGFORM: Einzelstrang
(D) TOPOLOGIE: linear

(ii) ART DES MOLEKÜLS: Sonstige Nucleinsäure
(A) BESCHREIBUNG: /desc = "Oligonucleotid"

(xi) SEQUENZBESCHREIBUNG: SEQ ID NO: 14:

ACAGGATCCT GTGCTATGCG GCGTGTGAAG 30

(2) ANGABEN ZU SEQ ID NO: 15:

(i) SEQUENZKENNZEICHEN:
(A) LÄNGE: 32 Basenpaare
(B) ART: Nucleotid
(C) STRANGFORM: Einzelstrang
(D) TOPOLOGIE: linear

(ii) ART DES MOLEKÜLS: Sonstige Nucleinsäure
(A) BESCHREIBUNG: /desc = "Oligonucleotid"

(xi) SEQUENZBESCHREIBUNG: SEQ ID NO: 15:

TTGGGATCCG CAATGCCCAC AGCATTTTTT TC 32

(2) ANGABEN ZU SEQ ID NO: 16:

(i) SEQUENZKENNZEICHEN:
(A) LÄNGE: 12 Aminosäuren
(B) ART: Aminosäure
(C) STRANGFORM: Einzelstrang
(D) TOPOLOGIE: linear

(ii) ART DES MOLEKÜLS: Peptid

(xi) SEQUENZBESCHREIBUNG: SEQ ID NO: 16:

Pro Trp Ser Lys Thr Gly Gly Leu Gly Asp Val Cys
1               5                   10

(2) ANGABEN ZU SEQ ID NO: 17:

(i) SEQUENZKENNZEICHEN:
(A) LÄNGE: 13 Aminosäuren
(B) ART: Aminosäure
(C) STRANGFORM: Einzelstrang
(D) TOPOLOGIE: linear

(ii) ART DES MOLEKÜLS: Peptid

(xi) SEQUENZBESCHREIBUNG: SEQ ID NO: 17:

Pro Ser Arg Phe Glu Pro Cys Gly Leu Asn Gln Leu Tyr
1               5                   10